# EUROPEAN PATENT APPLICATION

(11) **EP 1 535 906 A1**
(43) Date of publication of application: **01.06.2005**
(21) Application number: 03788133.1
(22) Date of filing: 18.08.2003
(51) Int. Cl.: C07D 207/16, A61K 31/4025, A61K 31/403, A61K 31/404, A61K 31/4245, A61K 31/427, A61K 31/428, A61K 31/429, A61K 31/437, A61K 31/438, A61K 31/4439, A61K 31/454, A61K 31/5377, A61K 31/541, A61K 45/00, A61P 3/04, A61P 3/10, A61P 13/08, A61P 17/00

(54) **NITROGEN-CONTAINING COMPOUNDS**

(30) Priority: 19.08.2002 JP 2002238673
(71) Applicant: ONO PHARMACEUTICAL CO., LTD., Osaka-shi, Osaka 541-8526 (JP)
(72) Inventor: NAKAI, Hisao, c/o Ono Pharmaceutical Co. Ltd., Mishima-gun, Osaka 618-8585 (JP); KONDO, Takashi, c/o Ono Pharmaceutical Co. Ltd., Mishima-gun, Osaka 618-8585 (JP); YAMAMOTO, Susumu, c/o Ono Pharmaceutical Co. Ltd., Mishima-gun, Osaka 618-8585 (JP)
(74) Representative: Henkel, Feiler & Hänzel
(86) International application number: PCT/JP2003/010401
(87) International publication number: WO 2004/016587

(57) **Abstract**

A compound represented by formula (I); 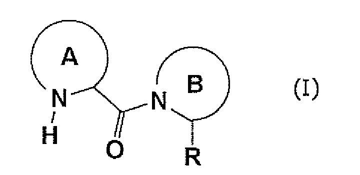 wherein ring A represents a nitrogen containing heterocyclic ring, ring B represents 5-membered heterocyclic ring which may have substituents, R represents a hydrogen atom or cyano and the other symbols represent as described in the specification; or a salt thereof. The compound represented by formula (I) has an inhibitory activity of DPP-IV, and therefore is useful as a preventive and/or treatment agent for type 2 diabetes mellitus, obesity, autoimmune disease, cancer metastasis, AIDS virus infection, dermatosis, prostatic hypertrophy and the like

## Description

### FIELD OF THE INVENTION

The present invention relates to a nitrogen-containing compound.

More in detail, this invention relates to a compound represented by formula (I) 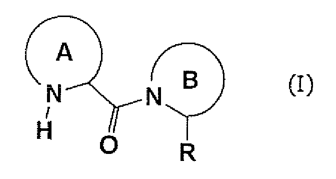 wherein all symbols are same meaning as defined hereinafter; and a salt thereof, a process for producing thereof and a pharmaceutical composition thereof as an active ingredient.

### BACKGROUND

Dipeptidyl peptidase IV (DPP-IV) is a member of the serine protease family, which cleaves a dipeptide, Xaa-Pro or Xaa-Ala, from a peptide having Pro or Ala as a second amino acid from N-terminal. It is widely expressed during a mammal organization, with the highest levels being in the kidney, liver, an intestinal tract epithelium, a placenta, and plasma. It is involved in metabolism of various bioactive peptides, especially strong in secretion of insulin. DPP-IV is remarkable for its activity to deactivate glucagon-like peptide-1 (GLP-1) which regulates postprandial blood glucose level.

Though a continuous subcutaneous injection of GLP-1 is tried as a therapy for patients with non-insulin-dependent (type 2) diabetes mellitus, cleaved GLP-1 may weaken its effect. Therefore the best way to prolong GLP-1 activity is thought to prevent the degradation of GLP-1, namely inhibit DPP-IV, which is the most important enzyme to inactivate it. To inhibit DPP-IV is expected useful in type 2 diabetes mellitus because it enhance GLP-1 activity, stimulate insulin release and improve glucose metabolism. It is also useful to anti-obesity agent of its appetite-reducing effect.

It is also known that DPP-IV has a relation to metabolism of neuropeptide Y, activation of immunocompetent cell, T-cell, adhesion of cancer cells to endothelia and penetration of a HIV to the lymphocyte. Therefore, it is thought that an inhibition of DPP-IV is useful for the medical treatment of an autoimmune disease, cancer transition, HIV infection, and the like

Besides, it is found that DPP-IV highly expresses in dermal fibroblasts of patients with psoriasis, rheumatoid arthritis and lichen planus and its activity is high in patients with benign prostatic hyperplasia. It is also expected that an inhibition of DPP-IV is also useful for the medical treatment of dermatoses and benign prostatic hyperplasia.

On the other hand, in the specification of WO02/14271, a compound represented by formula (W) 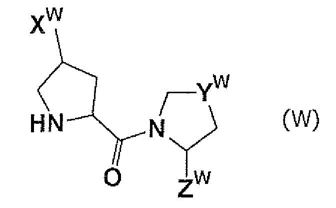 wherein all symbols are same meaning as defined within the specification; is described that it has an inhibitory activity of DPP-IV.

In the specification of WO95/15309, a compound represented by formula (Y) 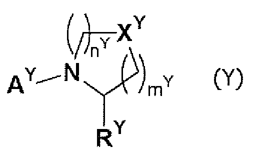 wherein all symbols are same meaning as defined within the specification; is described that it has an inhibitory activity of DPP-IV.

In the specification of WO01/55105, a compound represented by formula (Z) 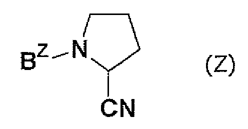 wherein all symbols are same meaning as defined within the specification;
is described that it has an inhibitory activity of DPP-IV.

### DISCLOSURE OF THE INVENTION

The present inventors have made extensive studies to find out a compound with an inhibitory activity of DPP-IV and finally have found that the object is achieved by the compound represented by formula (I).

The compound of the formula (I) of the present invention is unknown as an inhibitor of DPP-IV.

The present invention relates to
1. A compound represented by formula (I) 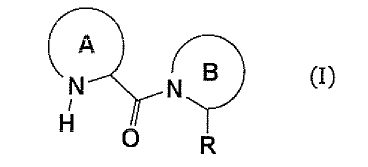 wherein ring A represents nitrogen-containing heterocyclic ring which may have substituents,
   ring B represents 5-membered heterocyclic ring which may have substituents,
   and R represents hydrogen atom or cyano;
   or a salt thereof,
2. A compound represented by formula (IA) 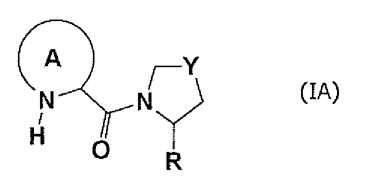 wherein Y represents -CH₂-, oxygen atom, nitrogen atom, or sulfur atom which may be oxidized; the ring represented 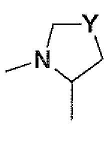 may be substituted;
   the other symbols represent same meaning as the above described 1;
   according to above described 1, or a salt thereof,
3. A compound represented by formula (IA-1) 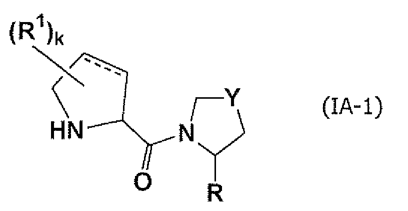 wherein k of R¹ each, independently, is
   1) C1-8 carbon chain which may be substituted by 1-5 of R²,
   2) carbocyclic ring which may be substituted by 1-5 of R ³,
   3) heterocyclic ring which may be substituted by 1-5 of R³, and carbon atom of this heterocyclic ring binds to ring A,
      or
   4) two R¹ taken together with abutting carbon atoms or a same carbon of ring A form carbocyclic ring or heterocyclic ring, and these rings may be substituted by 1-5 of R³;
      R² represents halogen, nitro, cyano, oxo, OR¹⁰, NR¹¹R¹², SR¹⁰, SO₂R¹³, COOR¹⁰, CONR¹¹R¹², COR¹³, =N-OR¹⁰, SO₂NR¹¹R¹², OCOR¹³, OSO₂R¹³, NR¹⁴CONR¹¹R¹², NR¹⁴COOR¹⁰, OCOOR¹⁰, OCONR¹¹R¹², SO₂OR¹⁰, OSO₂OR¹⁰, SOR¹³, carbocyclic ring which may be substituted by 1-5 of R³, or heterocyclic ring which may be substituted by 1-5 of R³;
      R³ represents C1-8 carbon chain which may be substituted by 1-5 of the group selected form halogen, nitro, cyano, oxo, OR¹⁰, NR¹¹R¹², SR¹⁰, SO₂R¹³, COOR¹⁰, CONR¹¹R¹², COR¹³, =N-OR¹⁰, SO₂NR¹¹R¹², OCOR¹³, OSO₂R¹³, NR¹⁴CONR¹¹R¹², NR¹⁴COOR¹⁰, OCOOR¹⁰, OCONR¹¹R¹², SO₂OR¹⁰, OSO₂OR¹⁰, SOR¹³; or halogen, nitro, cyano, oxo, OR¹⁰, NR¹¹R¹², SR¹⁰, SO₂R¹³, COOR¹⁰, CONR¹¹R¹², COR¹³, =N-OR¹⁰, SO₂NR¹¹R¹², OCOR¹³, OSO₂R¹³, NR¹⁴CONR¹¹R¹², NR¹⁴COOR¹⁰, OCOOR¹⁰, OCONR¹¹R¹², SO₂OR ¹⁰, OSO₂OR¹⁰, SOR¹³, carbocyclic ring which may be substituted or heterocyclic ring which may be substituted;
      R¹⁰ represents

   1) hydrogen atom,
   2) C1-8 carbon chain which may have substituents,
   3) carbocyclic ring which may have substituents, or
   4) heterocyclic ring which may have substituents;
      R¹¹, R¹², and R¹⁴ each independently represents

   1) hydrogen atom
   2) C1-8 carbon chain which may have substituents,
   3) carbocyclic ring which may have substituents,
   4) heterocyclic ring which may have substituents,
   5) COR¹³, or
   6) SO₂R¹³;
   R¹³ represents
   1) C1-8 carbon chain which may have substituents,
   2) carbocyclic ring which may have substituents,
   3) heterocyclic ring which may have substituents,
      k represents 0 or an integer of 1 to 5; 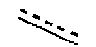 represents a single bond or a double bond;
      with the proviso that, when 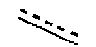 means a single bond, then k represents an integer of 1 to 5;
      the other symbols represent same meaning as the above described 1 and 2; according to above described 1 or a salt thereof,
4. A compound represented by formula (IA-2) 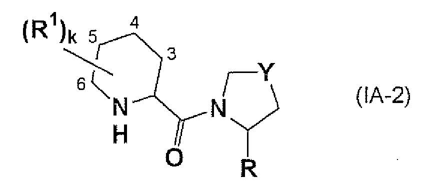 wherein all symbols represent same meaning as that of above described 1 and 2, with the proviso that, a ring represented by 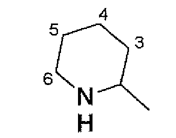 taken together with two R¹ doesn't form 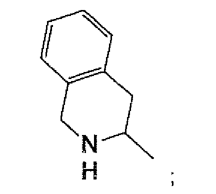 according to above described 1 or a salt thereof,
5. A compound represented by formula (IA-3) 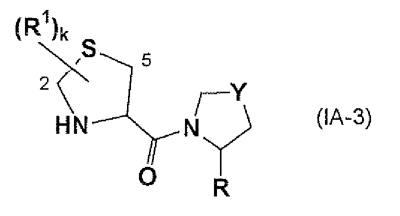 wherein all symbols represent same meaning as that of above described 2; according to above described 1, or a salt thereof,
6. A compound represented by formula (IA-4) 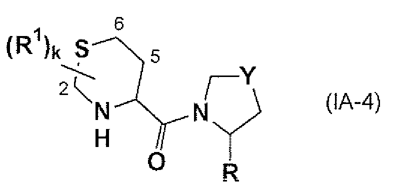 wherein all symbols represent same meaning as that of above described 2; according to above described 1, or a salt thereof,
7. A Dipeptidyl peptidase IV inhibitor which comprises the compound represented by formula (I) described in any one of above 1 to 5, or a salt thereof,
8. An agent according to the above 7, which is preventive and/or therapeutic agent for Dipeptidyl peptidase IV mediated diseases,
9. The agent according to the above 8, wherein the DPP-IV -mediated diseases are diabetes mellitus, obesity, autoimmune disease, cancer metastasis, AIDS virus infection, dermatosis or prostatic hypertrophy,
10. The DPP-IV inhibitor which comprises the compound represented by formula (I) described in the above 1 or a salt thereof, and one or at least two medicaments selected from PPAR agonist, sulfonyl urea system hypoglycaemic agent, insulin sensitizer, α-glucosidase inhibitor and acute effect type insulin secretagogue,
11. A method of inhibiting DPP-IV which comprises administering to a mammal an effective amount of the compound represented by formula (I) or a salt thereof,
12. Use of a compound represented by formula (I) or a salt thereof, for the manufacture of DPP-IV inhibitor,
13. A pharmaceutical composition, which comprise a compound described in the above 1 or a salt thereof,
   and
14. A prodrug of the compound described in the above 1.

In the present specification, a nitrogen-containing heterocyclic ring represented by ring A represents as following

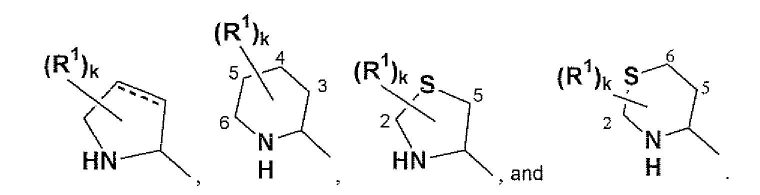

In the present specification, C1-8 carbon chain means C1-8 alkyl, C2-8 alkenyl, C2-8 alkynyl, C1-8 alkylidene, C3-8 alkenylidene and C3-8 alkynylidene.

C1-8 alkyl means methyl, ethyl, propyl, butyl, pentyl, hexyl, heptyl, octyl and isomeric groups thereof.

C2-8 alkenyl means ethenyl, propenyl, butenyl, pentenyl, hexenyl, heptenyl, octenyl and isomeric groups thereof.

C2-8 alkynyl means ethynyl, propynyl, butynyl, pentynyl, hexynyl, heptynyl, octynyl and isomeric groups thereof.

C1-8 alkylidene means methylidene, ethylidene, propylidene, butylidene, pentylidene, hexylidene, heptylidene, octylidene and isomeric groups thereof.

C3-8 alkenylidene means propenylidene, butenylidene, pentenylidene, hexenyllidene, heptenylidene, octenylidene and isomeric groups thereof.

C3-8 alkynylidene means propynylidene, butynylidene, pentynylidene, hexynyllidene, heptynylidene, octynylidene and isomeric groups thereof.

In the present specification, halogen atom means fluorine, chlorine, bromine and iodine.

In the present specification, the "carbocyclic ring" represents "C3-15 monocyclic, bicyclic or tricyclic carbocyclic ring".

The "C3-15 monocyclic, bicyclic or tricyclic carbocyclic ring" represents a C3-15 monocyclic bicyclic or tricyclic carbocyclic aryl, or partially or completely saturated one thereof, a spiro-bound bicyclic carbocyclic ring and a crosslinked bicyclic carbocyclic ring. For example, cyclopropane, cyclobutane, cyclopentane, cyclohexane, cycloheptane, cyclooctane, cyclononane, cydodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indan, naphthalene, dihydronaphthalene, teterahydronaphthalene, perhydronaphthalene, heptalene, perhydroheptalene, biphenylene, as-indacene, s-indacene, acenaphthylene, acenaphthene, fluorene, phenalene, phenanthrene, anthracene, spiro[4.4]nonane, spiro[4.5]decane, spiro[5.5]undecane, bicyclo[2.2.1]heptane, bicyclo[2.2.1]hept-2-ene, bicyclo[3.1.1]heptane, bicyclo[3.1.1]hept-2-ene, bicyclo[2.2.2]octane, bicyclo[2.2.2]oct-2-ene, adamantane, noradamantane, norbornane ring and the like.

In the present specification, the "heterocyclic ring" represents a "3-15 membered monocyclic, bicyclic or tricyclic heterocyclic ring containing 1 to 5 hetero atom(s) selected from oxygen atom(s), nitrogen atom(s), and sulfur atom(s)".

The "3-15 membered monocyclic, bicyclic or tricyclic heterocyclic ring containing 1 to 5 hetero atom(s) selected from oxygen atom(s), nitrogen atom(s), and sulfur atom(s)" represents 3-15 membered monocyclic, bicyclic or tricyclic heterocyclic aryl containing 1 to 5 hetero atom(s) selected from oxygen atom(s), nitrogen atom(s), and sulfur atom(s), or partially or completely saturated one thereof,

The "3-15 membered monocyclic, bicyclic or tricyclic heterocyclic aryl containing 1 to 5 hetero atom(s) selected from oxygen atom(s), nitrogen atom(s), and sulfur atom(s)" represents, for example, pyrrole, imidazole, triazole, tetrazole, pyrazole, pyridine, pyrazine, pyrimidine, pyridazine, azepin, diazepin, furan, pyran, oxepine, thiophene, thiopyran, thiepin, oxazole, isoxazole, thiazole, isothiazole, furazan, oxadiazole, oxazine, oxadiazin, oxazepine, oxadiazepine, thiadiazole, thiazine, thiadiazine, thiazepine, thiadiazepin, indole, isoindole, indolizine, benzodioxole, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepin, benzoxazepine, benzoxadiazepine, benzothiepine, benzothiazepin, benzothiadiazepine, benzoazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, perimidine and the like.

The "3-15 membered monocyclic, bicyclic or tricyclic partially or completely saturated heterocyclic aryl containing 1 to 5 hetero atom(s) selected from oxygen atom(s), nitrogen atom(s), and sulfur atom(s)" represents, for example, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindan, benzodioxane, chroman, benzodithiolane
benzodithiane, and the like.

In the present specification, a ring which is formed by two R¹ taken together with abutting carbon atoms, that is a ring fused with ring A represents 3-10 membered monocyclic, bicyclic or tricyclic saturated or unsaturated carbocyclic ring, for example, cyclopropane, cyclobutane, cyclopentane, cyclohexane, cycloheptane, cyclooctane, cyclononane, cyclodecane, cyclopentene, cyclohexene, cycloheptene, cyclooctene, cyclononene, cyclodecene, cyclopentadiene, cyclohexadiene, cycloheptadiene, cyclooctadiene, cyclononadiene, cyclodecadiene, benzene, naphthalene, indan, and the like.

In the present specification, a ring which is formed by two R¹ taken together with same carbons, that is a spiro-carbocyclic ring (ring C) represents 3-15 membered monocyclic, bicyclic or tricyclic saturated or unsaturated carbocyclic ring, for example, cyclopropane, cyclobutane, cyclopentane, cyclohexane, cycloheptane, cyclooctane, cyclononane, cyclodecane, cyclopentene, cyclohexene, cycloheptene, cyclooctene, cyclononene, cyclodecene, cyclopentadiene, cyclohexadiene, cycloheptadiene, cyclooctadiene, cyclononadiene, cyclodecadiene, dihydronaphthalene, teterahydronaphthalene, indene, indan, fluorine, and the like.

In the present specification, a ring which is formed by two R¹ taken together with abutting carbon atoms, that is a ring fused with ring A, represents 3-10 membered partially or completely saturated monocyclic or bicyclic heterocyclic ring containing 1 to 5 hetero atom(s) selected from oxygen atom(s), nitrogen atom(s), and sulfur atom(s), for example, imidazole, thiazole, oxazole, pyrazole, imidazoline thiazolidine, oxazolidine, pyrolidine, dihydropyrrole, piperidine, dihydropyridine tetrahydropyridine, tetrahydrofuran, tetrahydropyran, dihydropyran, dihydrofuran, tetrahydrothiophene, dihydrothiophene, dihydrothiaine, tetrahydrothiaine, benzofuran, benzothiophene, indole, indoline, indazole, benzodioxole, quinoline, isoquinoline, quinolizine, quinazoline, naphthyridine, phthalazine, quinoxaline, cinnoline, 1,4-dioxaspiro[4.5]decane and the like.

In the present specification, a ring which is formed by two R¹ taken together with same carbons, that is a spiro-carbocyclic ring (ring C) represents 3-10 membered saturated or partially unsaturated monocyclic or bicyclic heterocyclic ring containing 1 to 5 hetero atom(s) selected from oxygen atom(s), nitrogen atom(s), and sulfur atom(s), for example, oxetane, azetidine, pyrrolidine, dihydropyrrole, piperidine, dihydropyridine, tetrahydropyridine, tetrahydrofuran, tetrahydropyran, dihydropyran, dihydrofuran, tetrahydro thiophene, dihydrothiophene, dihydrothiain, tetrahydrothiain, tetrahydroquinoline, perhydroquinoline, dihydroindole, perhydroindole and the like.

In the present specification, "C1-8 carbon chain" in "C1-8 carbon chain which may have substituents" represents same meaning as that of above described "C1-8 carbon chain".

In the present specification, "substituents" in "C1-8 carbon bond which may have substituents" represents, for example, 1-5 substituent(s) selected from hydroxyl, C1-8 alkoxy, mono(C1-8 alkyl)amino, di(C1-8 alkyl)amino, C2-8 acyl, C1-8 alkoxycarbonyl, benzyloxycarbonyl, carboxy, halogen, (C1-8 alkyl)sulfonylamino, C2-8 acylamino, phenyl, C3-8 cycloalkyl, and pyridyl.

In the present specification, "carbocyclic ring" and "heterocyclic ring" in "carbocyclic ring or heterocyclic ring which may have substituents" represents same meaning as that of above described "carbocyclic ring" and "heterocyclic ring".

In the present specification, "substituent" in "carbocyclic ring or heterocyclic ring which may have substituents" represents, for example, 1-5 substituent(s) selected from C1-8 carbon chain, hydroxyl, C1-8 alkoxy, mono(C1-8 alkyl) amino, di(C1-8 alkyl)amino, C2-8 acyl, C1-8 alkoxycarbonyl, benzyloxycarbonyl, carboxy, halogen, (C1-8 alkyl)sulfonylamino, C2-8 acylamino, phenyl, C3-8 cycloalkyl, and pyridyl.

In the present specification, 5-membered ring containing nitrogen represented by ring B includes, for example, pyrrolidine, oxazolidine, or thiazolidine whose sulfur atom may be oxidized.

In the present specification, "substituent" in "5-membered hetrocyclic ring which may have substituents" represented by ring B includes same meaning as that of above described "substituent" in "carbocyclic ring or heterocyclic ring which may have substituents".

In the present invention, all following groups; 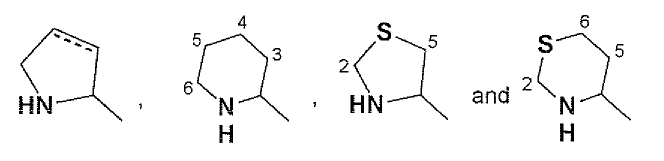 are preferable for nitrogen-containing heterocyclic ring represented by ring A.

In the other words, all compounds represented by formula (IA-1), (IA-2), (IA-3), and (IA-4) in compounds represented by formula (I) are preferable.

All groups represented by R¹ are preferable, more preferable R¹ is C1-8 alkyl which may have substituents, C2-8 alkenyl which may have substituents, C2-8 alkynyl which may have substituents, C1-8 alkylidene which may have substituents, carbocyclic ring which may have substituents, and heterocyclic ring which may have substituents.

More preferable R¹ is C1-8 alkyl which may have substituents, carbocyclic ring which may have substituents, and heterocyclic ring which may have substituents. Preferable carbocyclic ring is C3-8 cycloalkane, benzene, adamantane. Preferable heterocyclic ring is pyridine, furan, thiophene, thiazole, benzodioxole. When R¹ is benzene, the substituent of benzene includes alkyl which may have substituents, hydroxyl, C1-8 alkoxy, acyl, phenoxy, carboxy, CONR¹¹R¹², OSO²R¹³, NR¹¹SO²R¹³, halogen, cyano, carbocyclic ring which may have substituents, and heterocyclic ring which may have substituents,

When R¹ is benzene, it is especially suitable that benzene has hydroxyl as a substituent, arbitrarily with C1-8 alkyl or C1-8 alkoxy, acyl, cyano, phenoxy even more.

Moreover, two R¹ as substituents of ring A, taken together may form spiro-cyclic ring C or fused ring D.

When ring A is 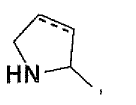 preferable k is an integer of 1 to 3, and more preferable k is an integer of 1 to 2.

When ring A is 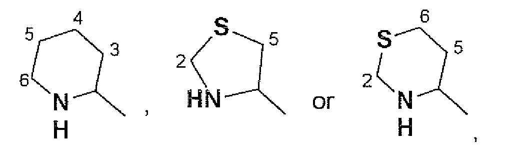 preferable k is 0 or an integer of 1 to 3 and more preferable k is an integer of 1 to 2.

Preferable Y is each of -CH₂- and sulfur atom.

Preferable R is each of hydrogen atom and cyano. When Y is -CH₂-, preferable R is cyano.

In the compound of the present invention represented by formula (I), preferably compound is the compound of formula (I-a) 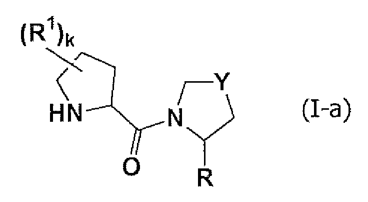 wherein all symbols represent same meanings as described above,
formula (I-b) 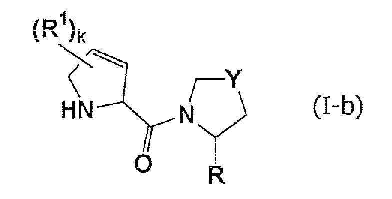 wherein all symbols represent same meanings as described above,
formula (I-d) 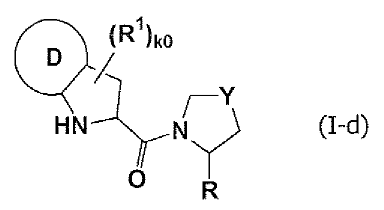 wherein k₀ represents 0 or an integer of 1 to 4; the other symbols represent same meaning as described above,
formula (I-e) 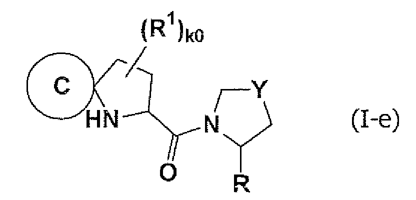 wherein all symbols represent same meanings as described above,
formula (I-f) 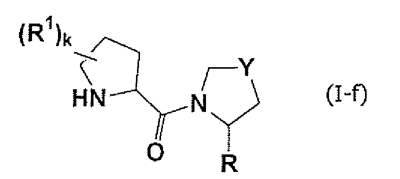 wherein all symbols represent same meanings as described above,
formula (I-g) 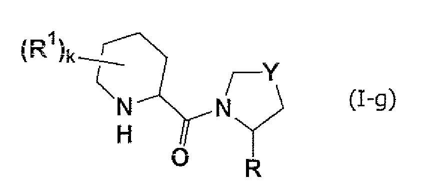 wherein all symbols represent same meanings as described above, formula (I-h) 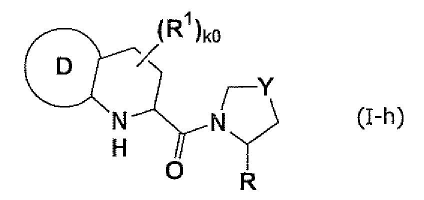 wherein all symbols represent same meanings as described above, formula (I-j) 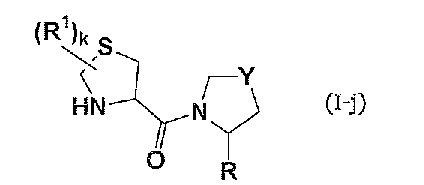 wherein all symbols represent same meanings as described above, formula (l-k) 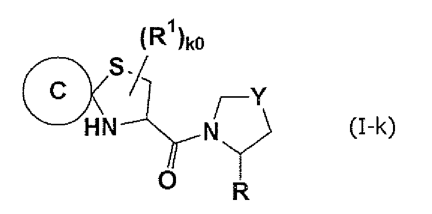 wherein all symbols represent same meanings as described above, formula (I-m) 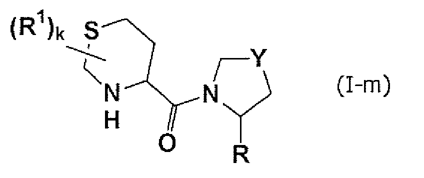 wherein all symbols represent same meanings as described above, formula (I-n) 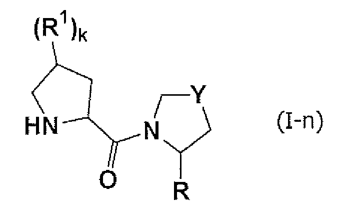 wherein all symbols represent same meanings as described above,
and formula (I-p) 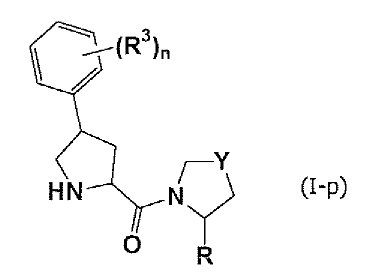 wherein n represents 0 or an integer of 1 to 5, and the other symbols represent same meaning as described above.

In addition, the compounds described in Examples and shown in following Table 1 to 10 are preferable. In the following tables, Ph represents phenyl.

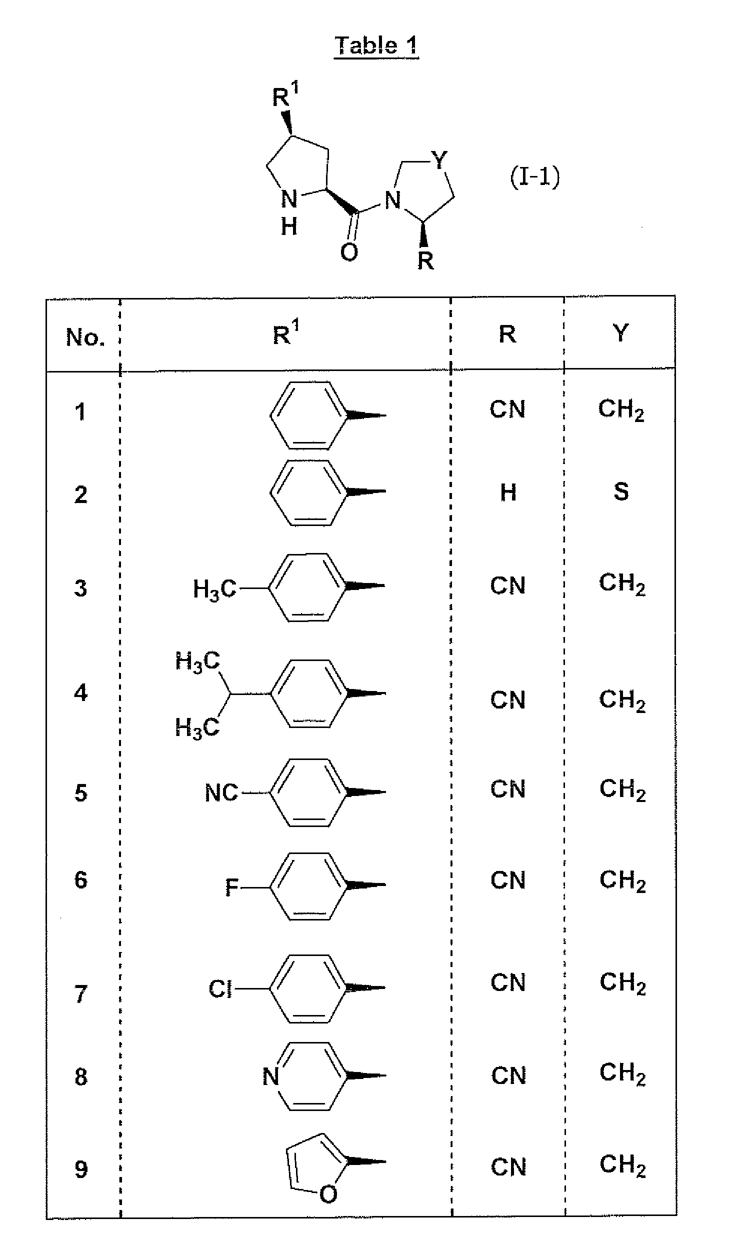

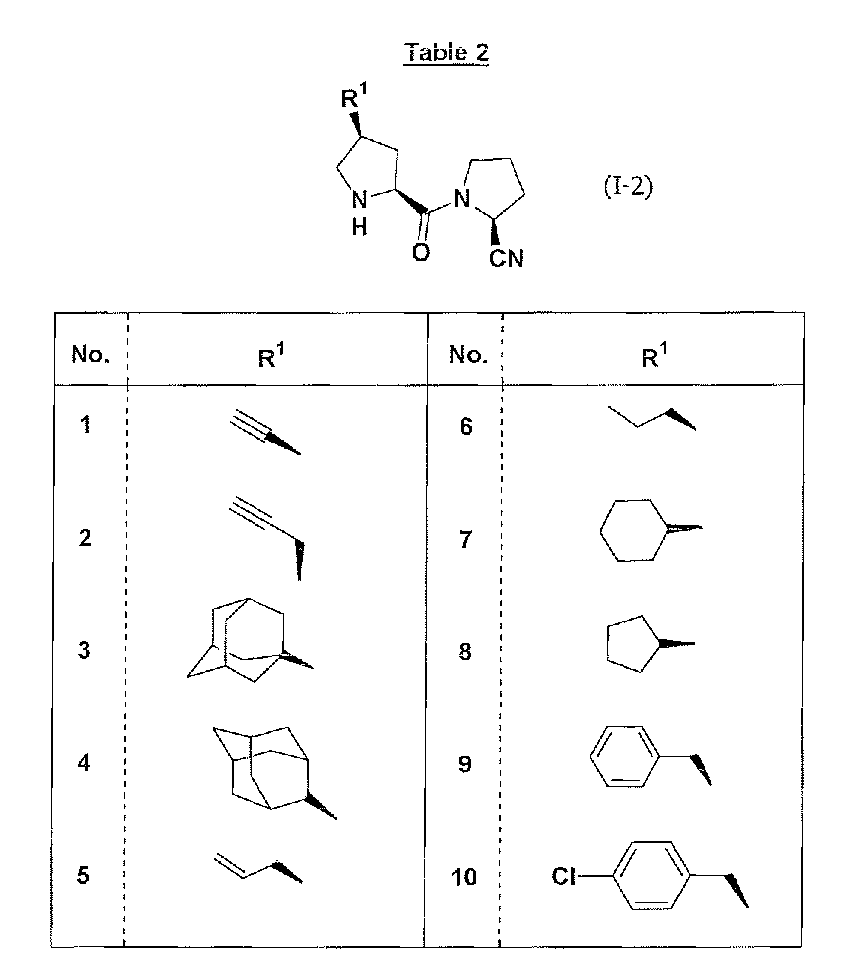

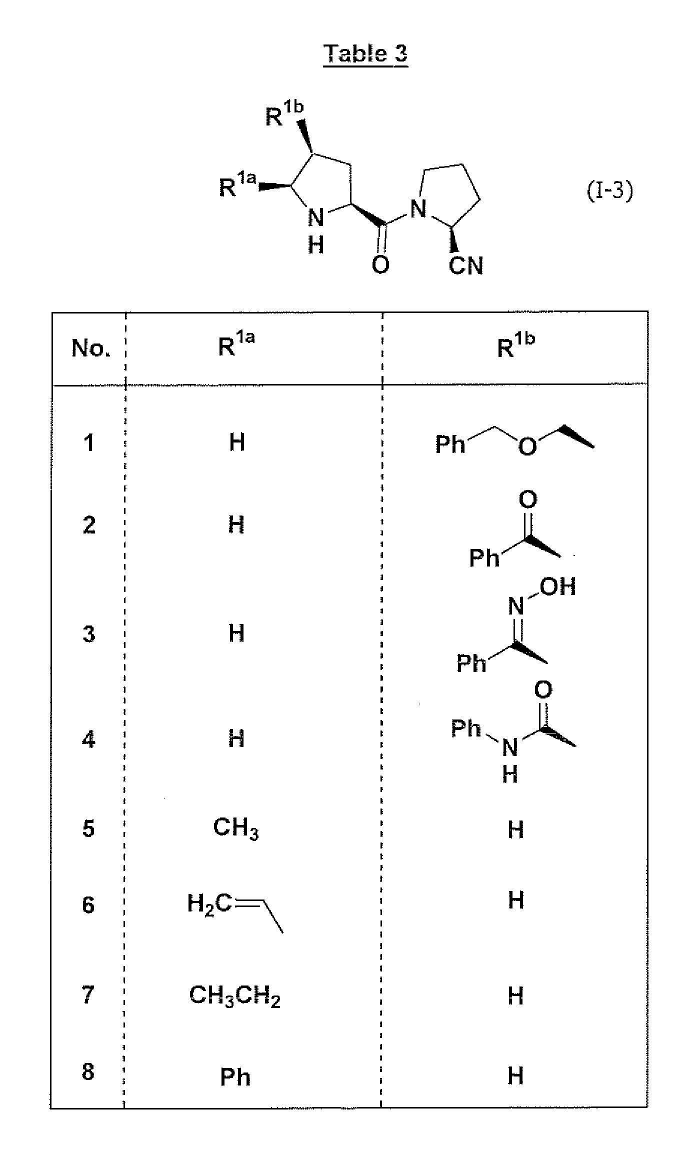

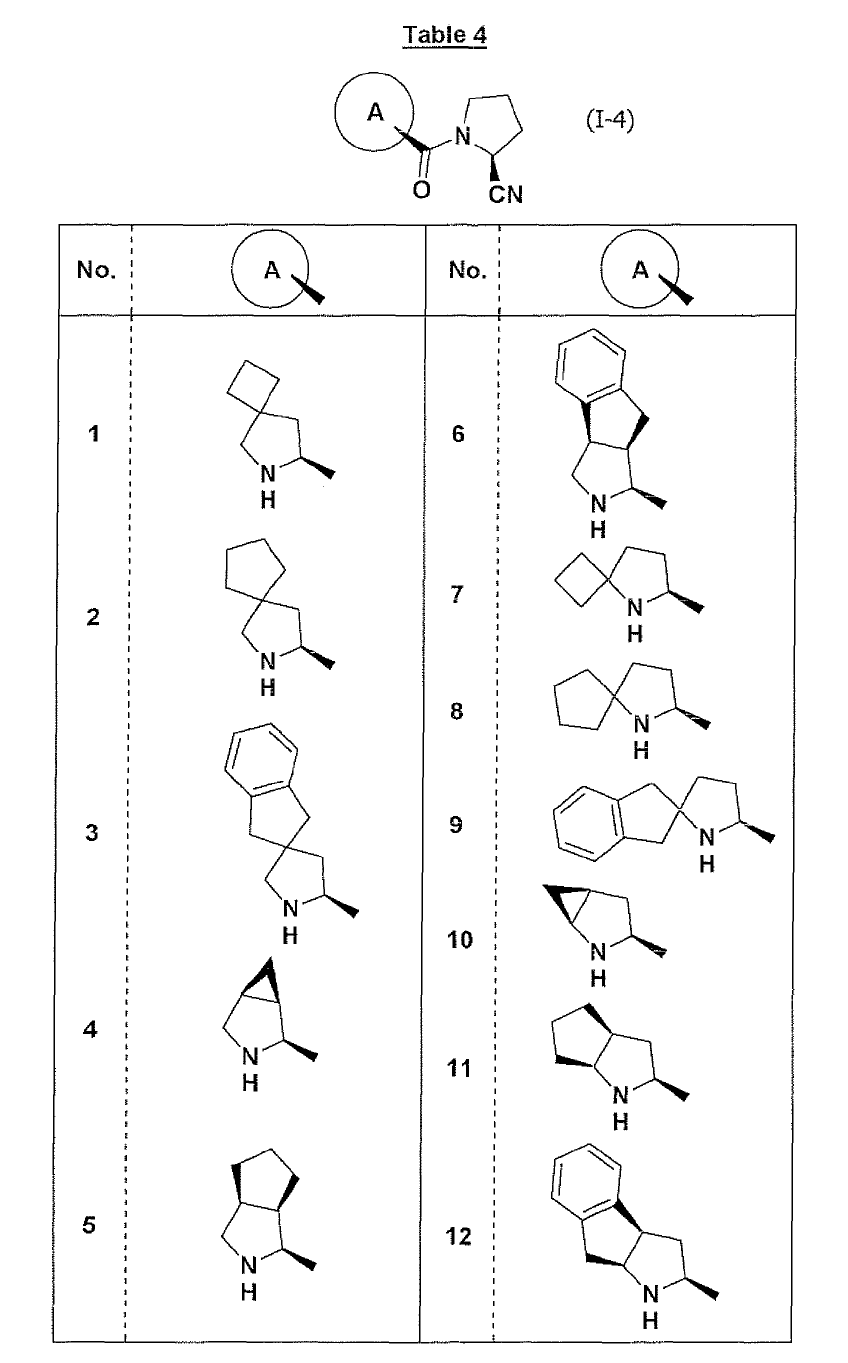

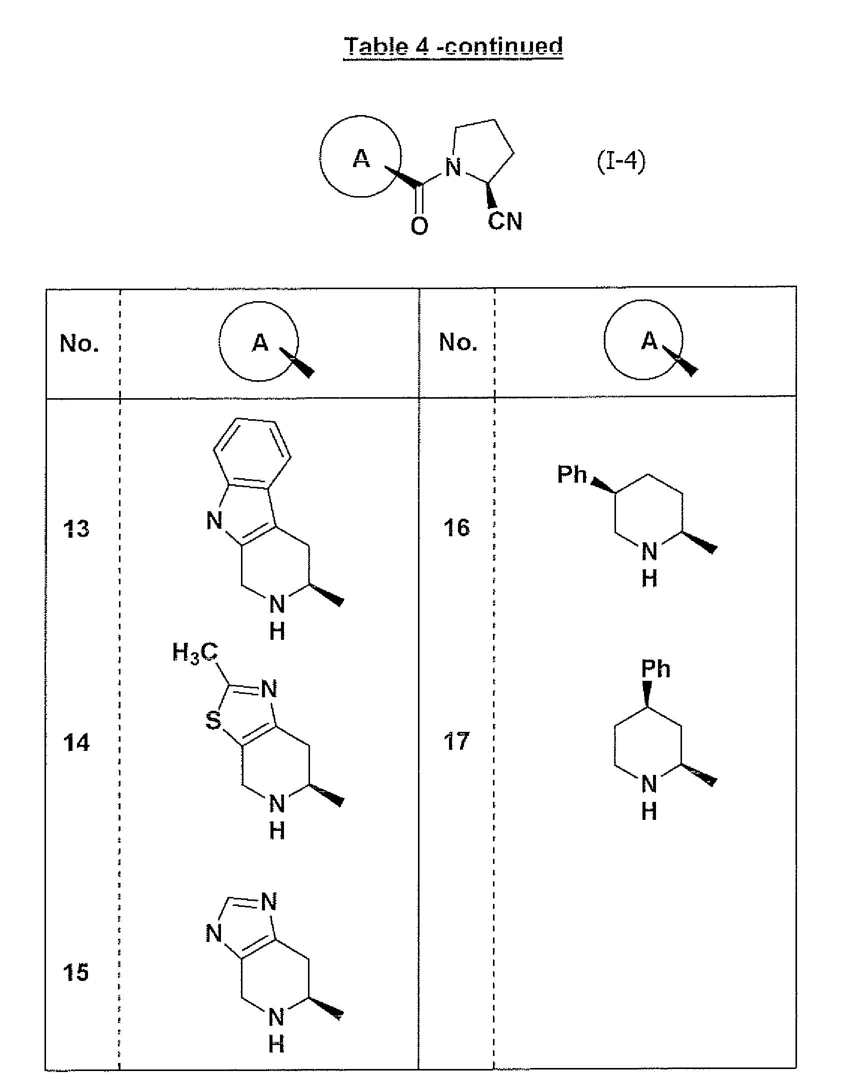

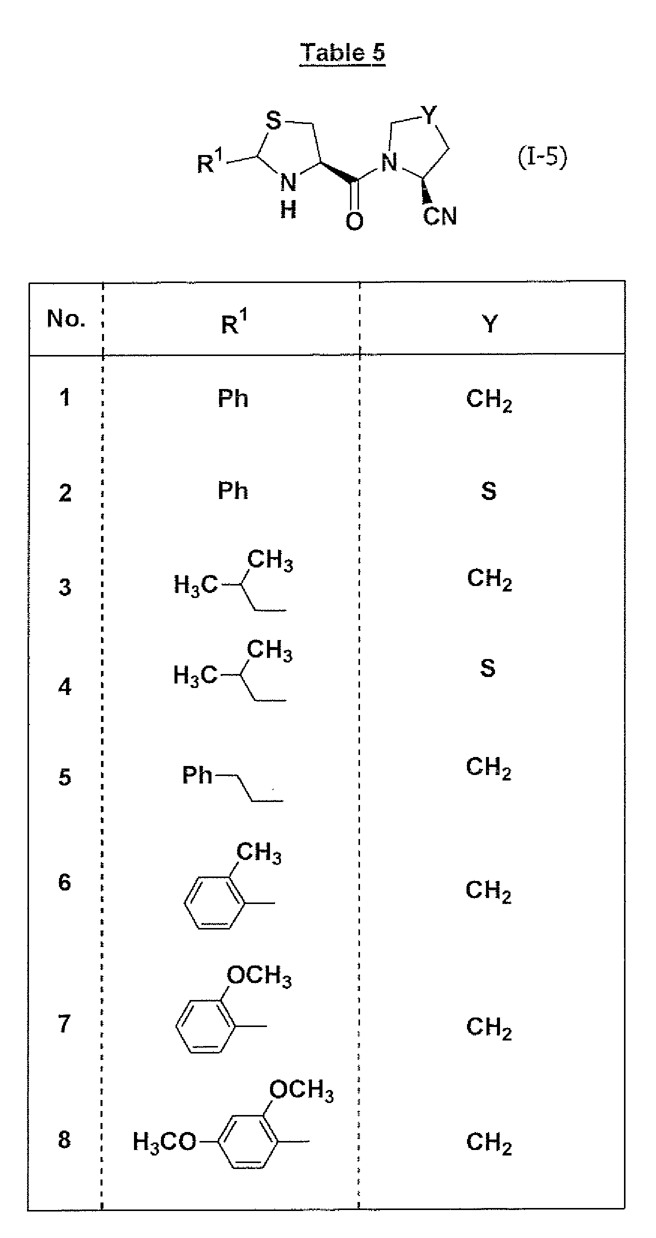

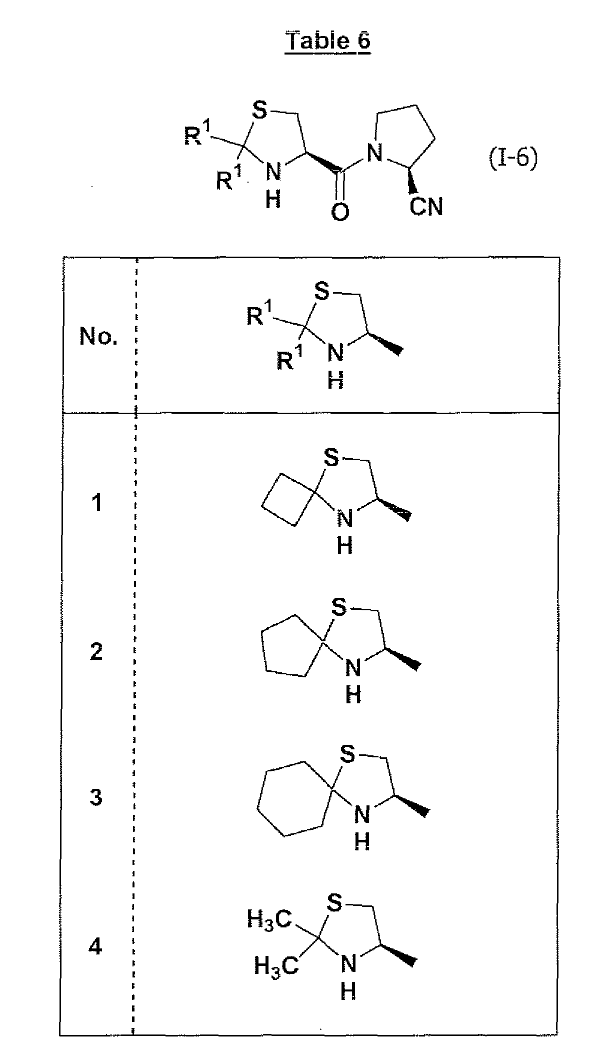

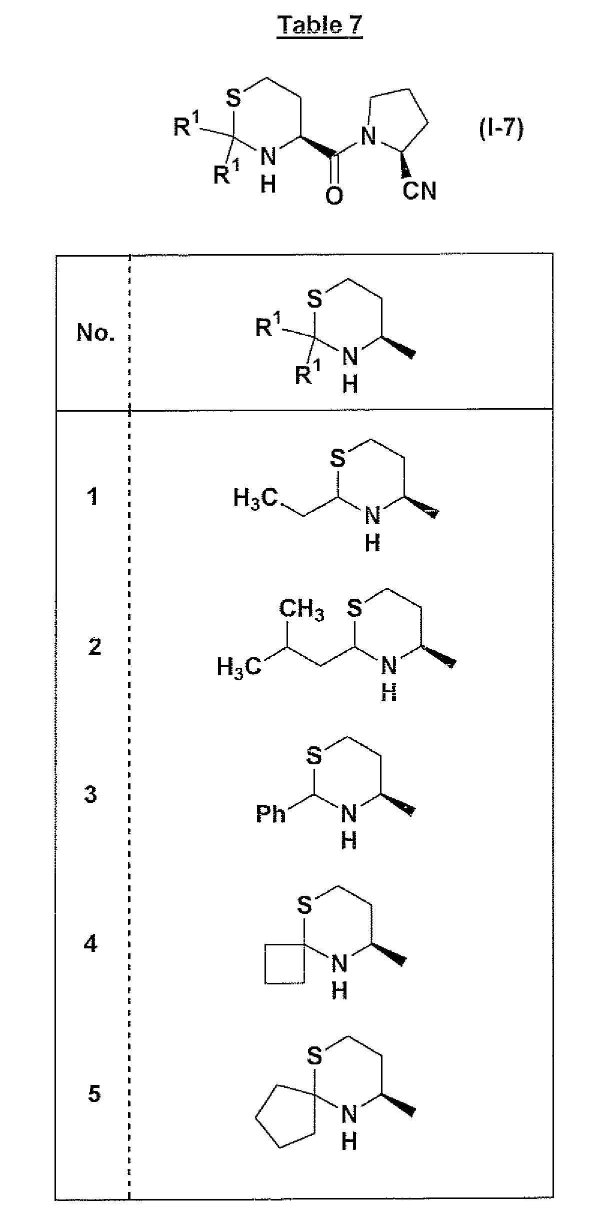

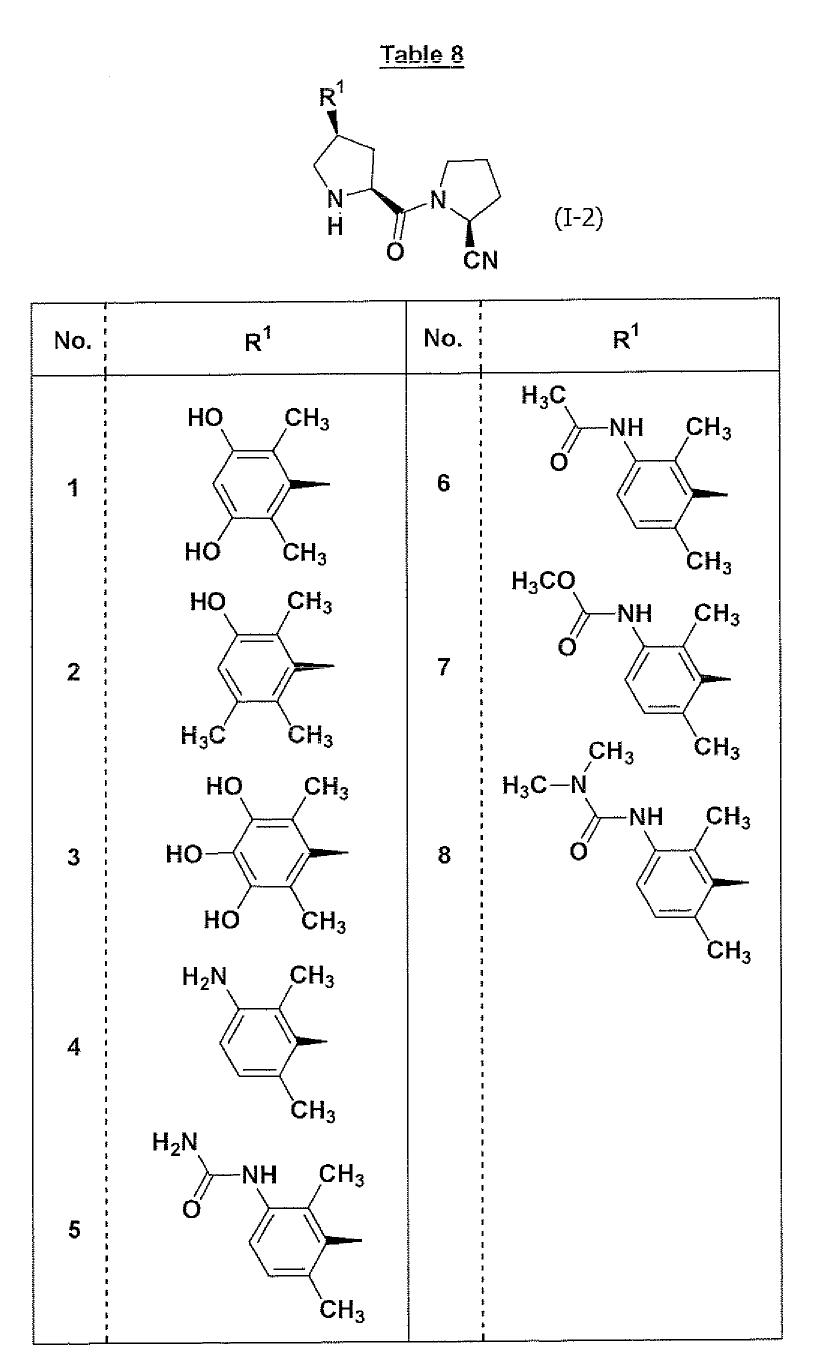

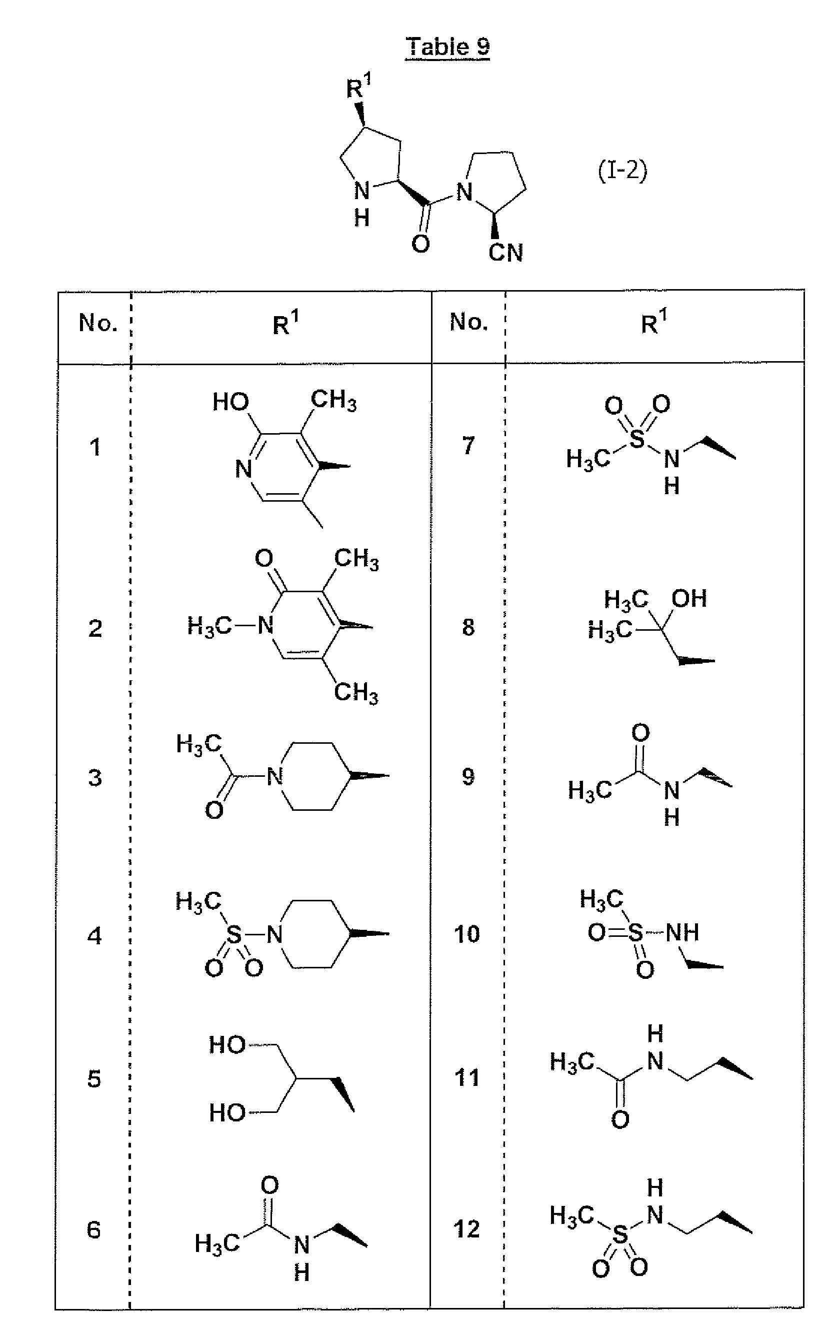

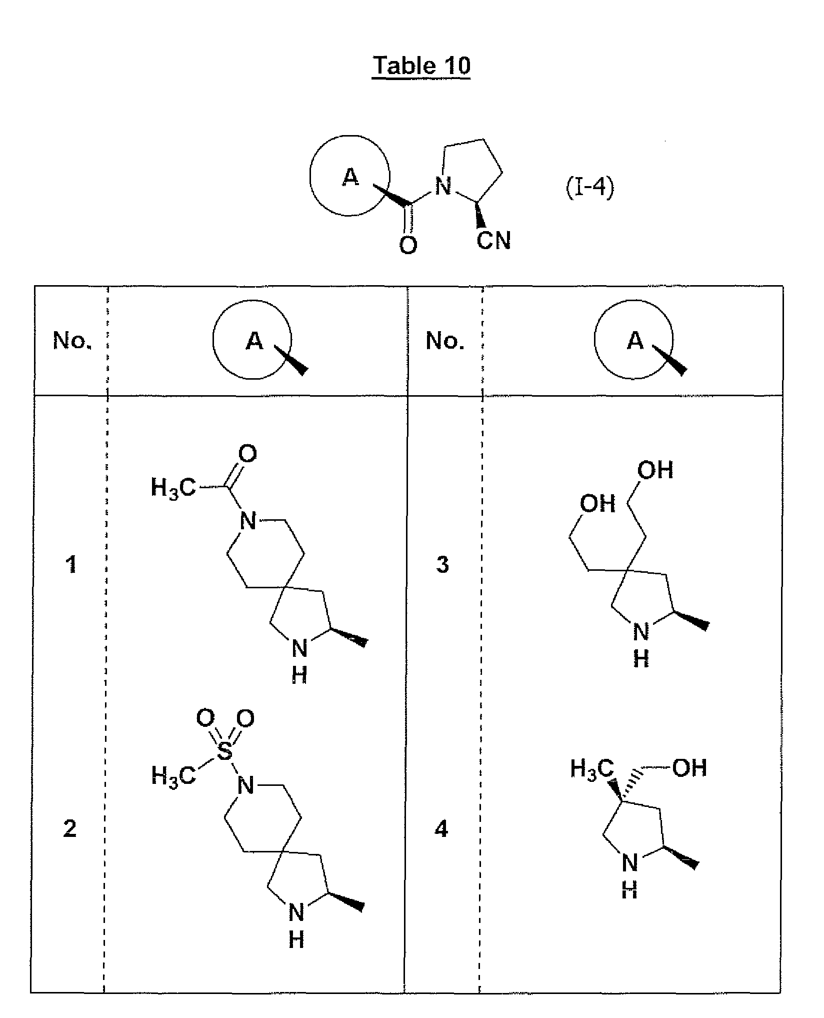

In the present invention, all isomers are included unless otherwise specified. For example, alkyl, alkoxy, alkylthio, alkenyl, alkynyl, alkylene, alkylidene, alkenylidene, alkynylidene includes straight or branched one. In addition, isomers on double bond, ring, fused ring (E-, Z-, cis-, trans-isomer), isomers generated from asymmetric carbon atom(s) (R-, S-, α-, β-configuration, enantiomer, diastereomer), optically active isomer (D-, L-, d-, I-configuration, (+)-, (-)-configuration), polar compounds generated by chromatographic separation (more polar compound, less polar compound), equilibrium compounds, rotational isomer, mixtures thereof at voluntary ratio and racemic mixtures are also included in the present invention.

Unless otherwise specifically described in the present specification,
a symbol 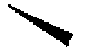 means a bond to front side of the paper (i.e., β-configuration),
a symbol 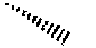 means a bond to the opposite side of the paper (i.e., α-configuration),
a symbol 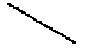 means a mixture of α- and β-configurations, and
a symbol 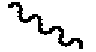 means either a bond to front side (i.e., β-confguration) or the opposite side (i.e., α-configuration) of the paper, but absolute configuration thereof is undecided.
Especially, 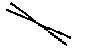 expressing double bonds, means a mixture of E- and Z-isomer among geometrical isomer of double bond.

### [Salt]

The compounds represented by formula (I) may be converted into the pharmaceutically acceptable salts by conventional means. In the present description, pharmaceutically acceptable salts includes salts of alkali metals, salts of alkaline earth metals, salts of amines, acid addition salts, and the like. In the case of including amino acid residue within a general formula (I), quaternary ammonium salts corresponding to thereof are included.

As salts, nontoxic and water-soluble salts are preferred. Suitable salts include salts of alkali metals (potassium, sodium, and the like), salts of alkaline earth metals (calcium, magnesium, and the like), ammonium salts, pharmaceutically acceptable salts of organic amines (tetramethylammonium, triethylamine, methylamine, dimethylamine, cyclopentylamine, benzylamine, phenethylamine, piperidine, monoethanolamine, diethanolamine, tris(hydroxylmethyl)methylamine, lysine, arginine, N-methyl-D-glucamine, and the like) and preferable ones are salts of alkali metals thereof.

As acid addition salts, nontoxic and water-soluble salts are preferred. Adequate acid addition salts includes salts of inorganic acid (hydrochloride, hydrobromide, sulfate, phosphate, nitrate, and the like), and salts of organic acid (acetate, trifluoroacetate, lactate, tartrate, oxalate, fumarate, maleate, citrate, benzoate, methanesulfonate, ethane sulfonate, benzensuplhonate, toluenesulfonate, ise-thionate, glucuronic acid salt, gluconate, and the like).

The compounds represented by formula (I) and salt thereof also can be converted into solvates thereof by conventional means.

As solvates, nontoxic and water-soluble salts are preferred. The appropriate solvates include hydrates, solvate of ethanol etc, and preferably is hydrates.

### [Preparation of the compound of the present invention]

### 1. The compound represented by formula (I) may be produced by subjecting the compound represented by formula (II)

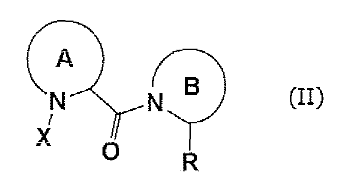

wherein X represents a protecting group of nitrogen atom and the other symbols represent same meaning as described above; to a deprotection reaction for a protective group of a nitrogen atom.

The protecting group for nitrogen atom includes, for example, benzyloxycarbonyl, t-butoxy carbonyl, trifluoro acetyl, and 9-fluorenylmethoxycarbonyl.

Each deprotection reaction of protecting group of nitrogen atom is well known, and it includes;
(1) deprotection reaction under alkaline conditions,
(2) deprotection reaction under acidic conditions,
(3) deprotection reaction by hydrogenolysis,
   and the like.

(1) The deprotection reaction under alkaline conditions is, for example, carried out in an organic solvent (methanol, tetrahydrofuran, dioxane, or dimethylformamide and the like) using a hydroxide of an alkali metal (sodium hydroxide, potassium hydroxide, or lithium hydroxide, and the like), a hydroxide alkaline earth metal (barium hydroxide, or calcium hydroxide and the like), organic amine (triethylamine, N-methylmorpholine, diisopropyl ethamine, piperidine and the like) or quaternary ammonium salt (tetrabutylammonium fluoride and the like), an aqueous solution thereof, or a mixture thereof at a temperature of 0 to 40 °C.
(2) The deprotection reaction under acidic conditions is carried out, for example, in an organic solvent (methylene chloride, chloroform, dioxane, ethyl acetate, or anisole and the like), in an organic acid (acetic acid, trifuloroacetic acid, or methansulfonic acid, and the like), an inorganic acid (hydrochloric acid, or sulfuric acid, and the like) or a mixture thereof (hydrogen bormide/acetic acid, and the like) at a temperature of 0 to 100 °C.
(3) The deprotection reaction by hydrogenolysis is carried out, for example, in a solvent (ethers (tetrahydrofuran, dioxane, dimethoxyethane, or diethyl ether, and the like), alcohols (methanol, or ethanol, and the like), benzenes (benzene, toluene and the like), ketones (acetone, methylethylketone, and the like), nitriles (actetonitrile and the like), amides (dimethylformamide and the like), water, ethyl acetate, acetic acid, a mixed solvent of at least two of these and the like) in the presence of a catalyst (palladium-carbon, palladium black, palladium hydroxide-carbon, platinum oxide, Raney nickel, and the like) under the hydrogen atomosphere at normal pressure or under pressurization, or in the presence of ammonium formate at a temperature of 0 to 200 °C.

As well understood to the skilled persons in the art, the objective compounds of the present invention may be prepared easily by using these reactions.

### 2. The compound represented by formula (I) also may be produced by subjecting the compound represented by formula (III)

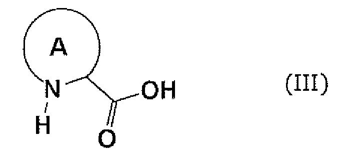

wherein all symbols represent same meaning as described above; to an amidation reaction with formula (IV) 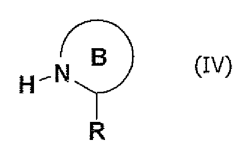 wherein all symbols represent same meaning as described above.

The amidation reaction is publicly known, and it includes;
(1) a process using acid halide,
(2) a process using a mixed acid anhydride,
(3) a process using a condensing agent,
   and the like.

Such processes will be specifically illustrated as follows.
(1) A process using an acid halide is carried out, for example, in such a manner that carboxylic acid reacts with an agent for producing an acid halide (such as oxalyl chloride and thionyl chloride and the like) in an organic solvent (chloroform, dichloromethane, diethyl ether and tetrahydrofuran) or without solvent at -20 °C to refluxing temperature and the resulting acid halide reacts with an amine in the presence of a base (pyridine, triethylamine, dimethylaniline, dimethylaminopyridine and diisopropylethylamine and the like) in an inert organic solvent (chloroform, dichloromethane, diethyl ether and tetrahydrofuran and the like) at the temperature of 0 to 40 °C. It is also possible to conduct the reaction with an acid halide at 0 to 40 °C in an organic solvent (dioxane and tetrahydrofuran and the like) using an aqueous solution of alkali (aqueous solution of sodium bicarbonate and an aqueous solution of sodium hydroxide and the like).
(2) A process using a mixed acid anhydride is carried out, for example, in such a manner that carboxylic acid is made to react with an acid halide (pivaloyl chloride, tosyl chloride or mesyl chloride and the like) or with an acid derivative (ethyl chloroformate and isobutyl chloroformate and the like) at 0 to 40 °C in an organic solvent (chloroform, dichloromethane, diethyl ether, tetrahydrofuran and the like) or without a solvent in the presence of a base (pyridine, triethylamine, dimethylaniline, dimethylaminopyridine, diisopropylethylamine and the like) and the resulting mixed acid anhydride is made to react with an amine at 0 to 40 °C in an organic solvent (chloroform, dichloromethane, diethyl ether, tetrahydrofuran and the like).
(3) A process using a condensing agent is carried out, for example, in such a manner that carboxylic acid and an amine are subjected to a reaction at 0 to 40 °C with or without 1-hydroxybenztriazole (HOBt) using a condensing agent (1,3-dicyclohexylcarbodiimide (DCC), 1-ethyl-3-[3-(dimethylamino)propyl]carbodiimide (EDC), 1,1'-carbonyldiimidazole (CDI), 2-chloro-1-methylpyridinium iodide, 1-propanephosphonic acid cyclic anhydride (PPA), and the like) in the presence or absence of a base (pyridine, triethylamine, dimethylanilin, dimethylaminopyridine, and the like) in an organic solvent (chloroform, dichloromethane, dimethylformamide, diethyl ether and tetrahydrofuran, and the like) or without a solvent.

It is preferred that all the reactions of (1), (2) and (3) are carried out in an atmosphere of inert gas (argon and nitrogen, and the like) under an anhydrous condition.

The compound represented by formula (II) may be produced by subjecting the compound represented by formula (IV) to an amidation reaction with formula (V)

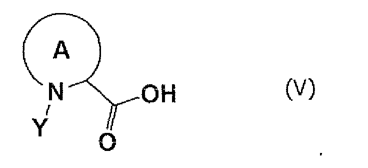

The compound represented by formula (V), in other words, the compounds represented by formulae (V-1), (V-2), (V-3), (V-4), (V-5), (V-6), (\/-7), and (V-8) may be produced by the process shown in the following schemes 1-4.

In the following schemes, R^{5a}, R^{5b}, R^{5c}, R^{5d} represents same meaning as R⁵ respectively, R^{6a}, R^{6b}, R^{6c}, R^{6d}, R^{6e}, R^{6f} express represents same meaning as R⁶ respectively ,with the proviso that at least one of R^{6a}, R^{6b}, R^{6c}, R^{6d}, R^{6e}, R^{6f} is hydrogen; R^{1A} represents C1-8 alkyl optionally with substituents among R¹, Boc represents *t*-butoxycarbonyl group, Me represents methyl group, tBu represents *t*-butyl group, Et represents ethyl group, Ts represents p-toluene sulfonyl group, Tf represents trifluoromethane sulfonyl, and NaHMDS represents sodium hexamethyldisilazane.

In addition, in scheme 2, when another R¹ exists, k1 represents 0 or an integer of 1 to 4, and when another R¹ doesn't exist, k1 represents 0 or an integer of 1 to 5.

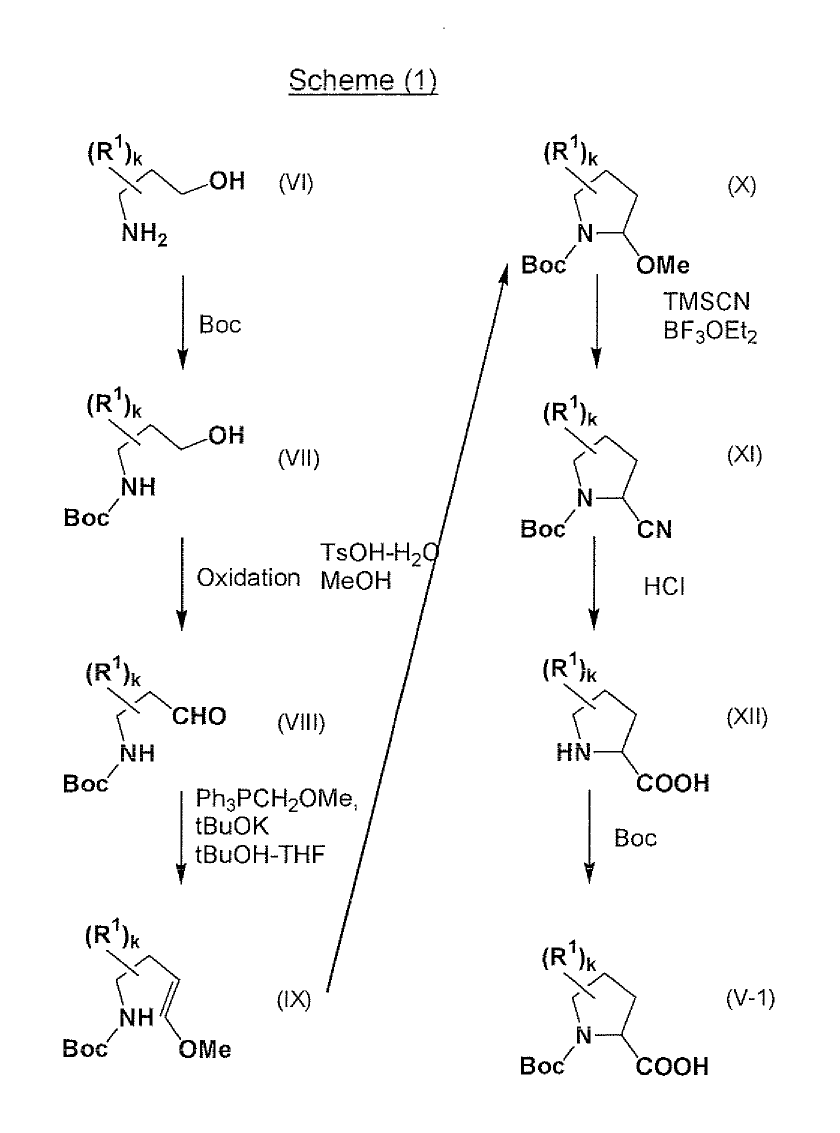

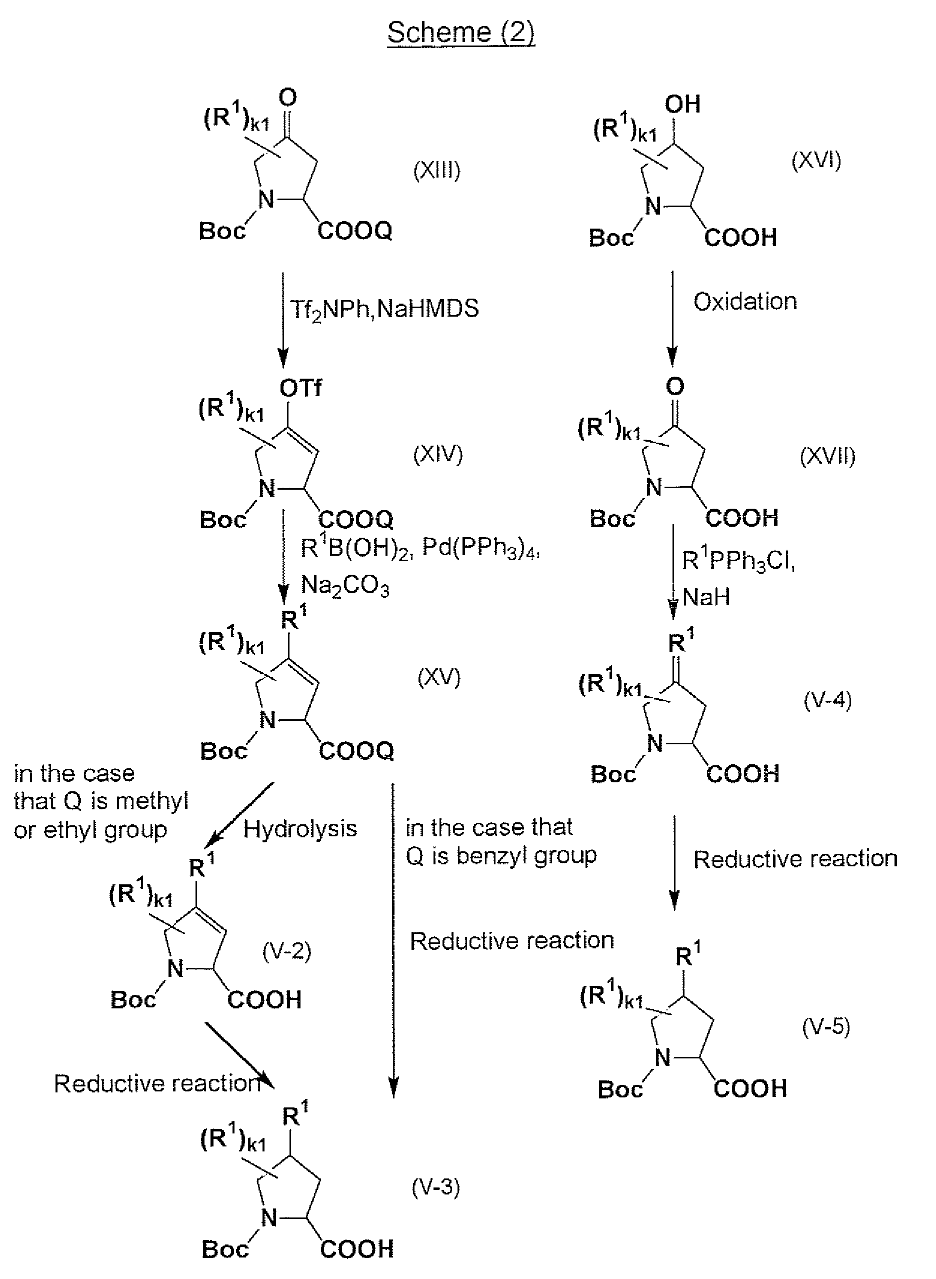

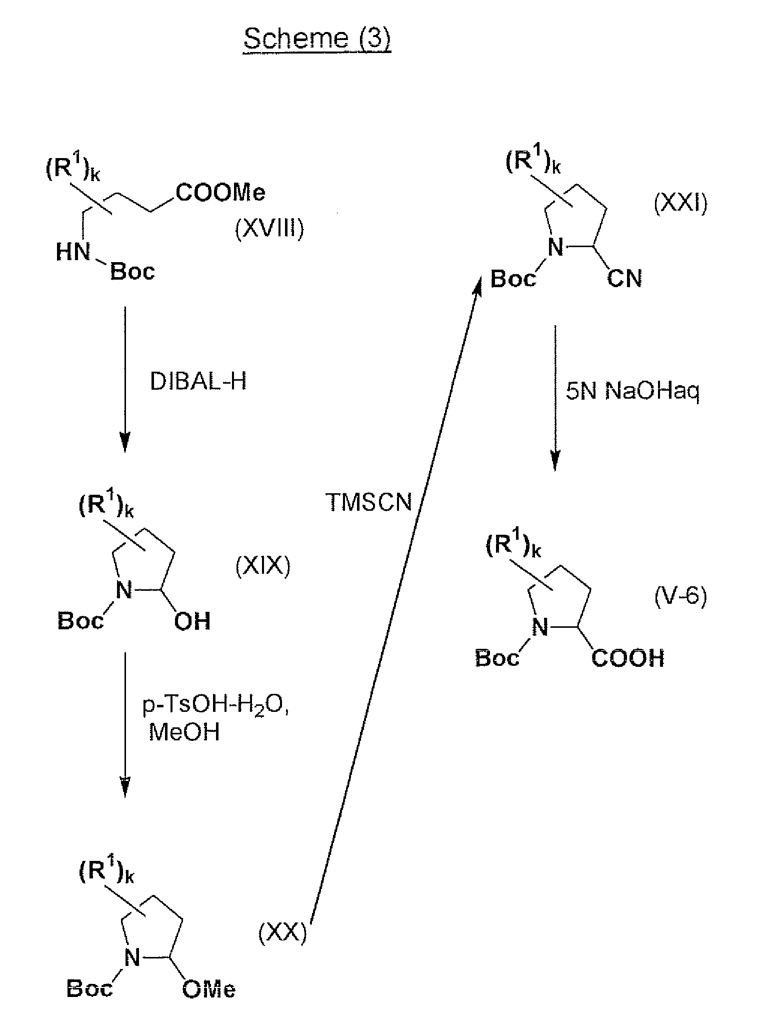

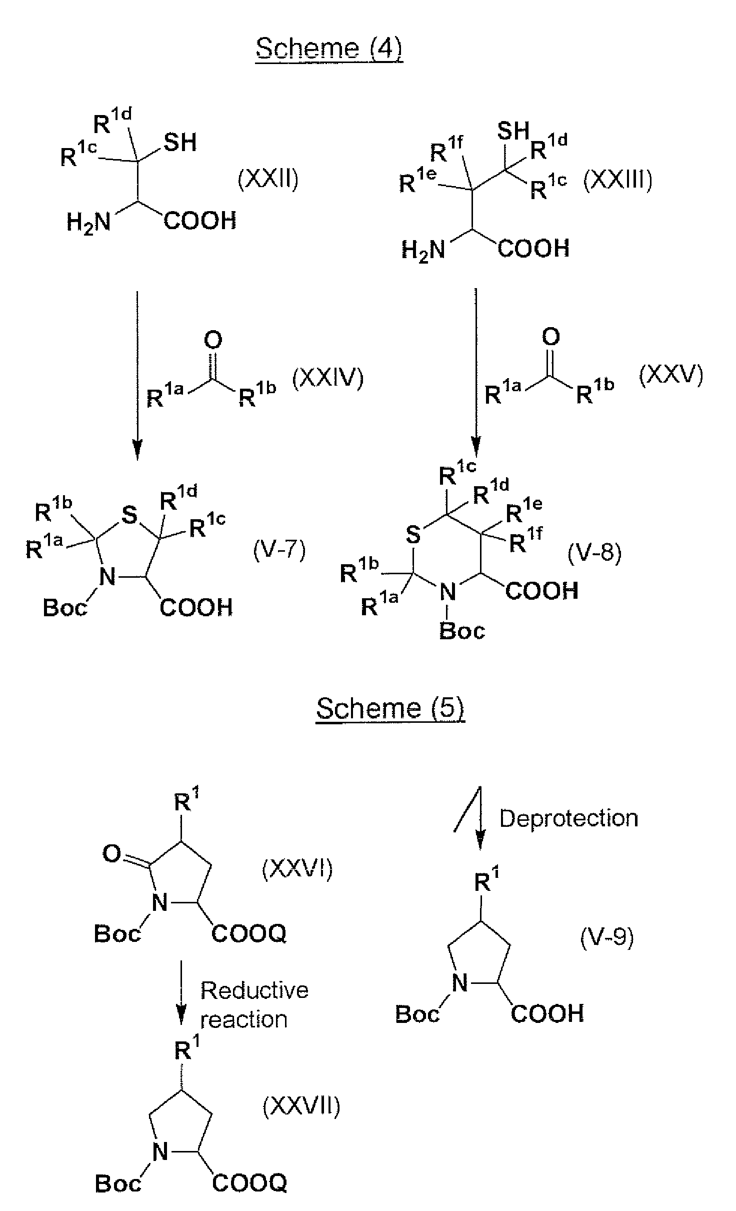

The compounds described in the above scheme 1-5 used as starting materials and reagents have been known or may be prepared from known compounds by known methods.

For example, the compounds represented by formula (XXVI) may be produced by the method described in Tetrahedron Letters, 1998, 39, 5887-5890 or J.Org.Chem, 1995, 60, 2925-2930.

Moreover, other compounds also may be produced by the above scheme 1-5, the method described in the following Examples, or the like thereof.

For example, the compound represented by formula (V-10) 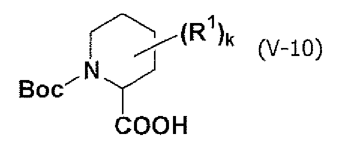 , which is a intermediate of the compound of formula (I),
wherein 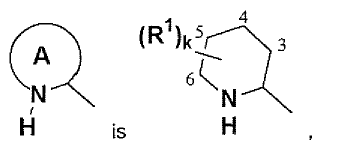 may be produced by same method in scheme 3.

Furthermore, the compound represented by formula (V-11) 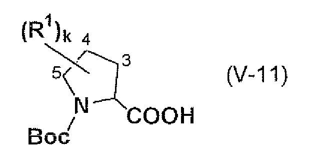 , which is a intermediate of the compound of formula (I), wherein 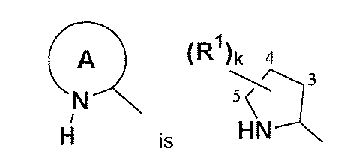 may be produced by same method in scheme 1 or 3, using a corresponding compound that is the compound represented by formula (VI-a) or (XVIII-a) in stead of the compound represented by above formula (VI) or (XVIII).

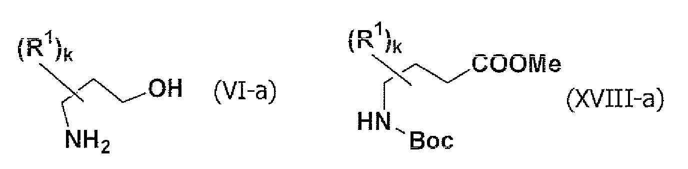

The compounds of the present invention also may be produced by subjecting the compounds produced by the above methods to a deprotection reaction of protecting group for amino group, hydroxyl group, mercapto group and/or carboxyl group if necessary.

A protective group for carboxyl includes, for example, methyl, ethyl, allyl, *t*-butyl, trichloroethyl, benzyl (Bn), phenacyl, and the like.

A protective group for hydroxyl includes, for example, methyl, trityl, methoxymethyl (MOM), 1-ethoxyethyl (EE), methoxyethoxymethyl (MEM), 2-tetrahydropyranyl (THP), trimethylsilyl (TMS), triethylsilyl (TES), *t*-butyldimethylsilyl (TBDMS), *t*-butyldiphenylsilyl (TBDPS), acetyl (Ac), pivaloyl, benzoyl, benzyl (Bn), p-methoxybenzyl, allyloxycarbonyl (Alloc), 2,2,2-trichloroethoxycarbonyl (Troc), and the like.

A protective group for amino includes, for example, benzyloxycarbonyl, *t*-butoxycarbonyl, allyloxycarbonyl (Alloc), 1-methyl-1-(4-biphenyl)ethoxycarbonyl (Bpoc), trifluoroacetyl, 9-fluororenylmethoxycarbonyl, benzyl (Bn), *p*-methoxybenzyl, benzyloxymethyl (BOM), 2-(trimethylsilyl)ethoxymethyl (SEM), and the like.

A protective group for mercapto includes, for example, benzyl, methoxybenzyl, methoxymethyl (MOM), 2-tetrahydropyranyl (THP), diphenylmethyl and acetyl (Ac).

A protective group for carboxyl, hydroxyl, amino or thiol is not particularly limited in addition to the above-described groups as long as it can be deprotected easily and selectively. For example, those described in *Protective Groups in Organic Synthesis* (T. W. Greene, John Wiley & Sons Inc., 1999) may be used.

The deprotection of the protective group for carboxyl, hydroxyl, amino or thiol is well known. For example, it is
(1) alkaline hydrolysis,
(2) deprotection of a protective group in acidic conditions,
(3) deprotection of a protective group by hydrogenolysis,
(4) deprotection of a protective group containing silyl,
(5) deprotection of a protective group using a metal,
(6) deprotection of a protective group using an organometal,
   and the like.

In the following, these methods are specifically described.
(1) The deprotection of the protective group by alkaline hydrolysis condition may be carried out, for example, in an organic solvent (methanol, tetrahydrofuran, dioxane, and the like) with alkaline metal hydroxide (sodium hydroxide, potassium hydroxide, lithium hydroxide, and the like), alkaline earth metal hydroxide (barium hydroxide, calcium hydroxide, and the like), carbonate (sodium carbonate or potassium carbonate, and the like), an aqueous solution thereof or a mixture thereof at 0 to 40°C.
(2) The deprotection of the protective group in acidic conditions may be carried out, for example, in an organic solvent (dichloromethane, chloroform, 1,4-dioxane, ethyl acetate, anisole, and the like), organic acid (acetic acid, trifluoroacetic acid, methanesulfonic acid, *p*-toluenesulfonic acid, and the like), inorganic acid (hydrochloric acid, sulfuric acid, and the like), or a mixture thereof (hydrogen bromide/acetic acid, and the like) at 0 to 100°C.
(3) The deprotection of the protective group by hydrogenolysis may be carried out, for example, in a solvent (ethers (tetrahydrofuran, 1,4-dioxane, 1,2-dimethoxyethane, diethyl ether, and the like), alcohols (methanol, ethanol, and the like), benzenes (benzene, toluene, and the like), ketones (acetone, methylethylketone, and the like), nitriles (acetonitrile, and the like), amides (N,N-dimethylformamide, and the like), water, ethyl acetate, acetic acid, a mixture of two or more thereof, and the like) in the presence of a catalyst (palladium on carbon, palladium black, palladium hydroxide, platinum oxide, Raney nickel, and the like) under hydrogen atmosphere at a normal pressure or elevated pressure, or in the presence of ammonium formate at 0 to 200°C.
(4) The deprotection of the protective group for silyl may be carried out, for example, in a water-miscible organic solvent (tetrahydrofuran, acetonitrile, and the like) which can be, with tetrabutylammonium fluoride at 0 to 40°C.
(5) The deprotection of the protective group a using metal may be carried out, for example, in an acidic solvent (acetic acid, a pH 4.2 to 7.2 buffer, a mixed solution of the buffer and an organic solvent such as tetrahydrofuran, and the like) in the presence of powder zinc, with or without an ultrasonic wave at a temperature of 0 to 40°C.
(6) The deprotection of the protective group using a metal complex may be carried out, for example, in an organic solvent (dichloromethane, dimethylformamide, tetrahydrofuran, ethyl acetate, acetonitrile, dioxane, ethanol, and the like), water or a mixed solvent thereof in the presence of a trap reagent (tributyltin hydride, triethylsilane, dimedone, morpholine, diethylamine, pyrrolidine, and the like), an organic acid (acetic acid, formic acid, 2-ethylhexanic acid, and the like) and/or an organic acid salt (sodium 2-ethylhexanate, potassium 2-ethylhexanate, etc) in the presence or absence of a phosphine reagent (triphenylphosphine, and the like) using a metal complex (tetrakis(triphenylphosphine)palladium (O), dichlorobis(triphenylphosphine)palladium (II), palladium acetate (II), chlorotris(triphenylphosphine)rhodium (I), and the like) at 0 to 40°C.

In addition to the above methods, the deprotection may be carried out by the method described in *Protective Groups in Organic Synthesis* (T. W. Greene, John Wiley & Sons Inc., 1999),

As well understood to the skilled persons in the art, the objective compounds of the present invention may be prepared easily by using these deprotection reactions.

Each reaction of all the above schemes can be done with known methods. Furthermore, other compounds used as starting materials or reagents are known per se or may be prepared by known methods. For example, the compound represented by formula (IV) is known and the compound represented by formula (III) may be produced from known compounds by known methods.

In each of the reactions in the present specification, the reaction product may be purified by a conventional purifying method such as distillation under ordinary or reduced pressure, high performance liquid chromatography, thin-layer chromatography or column chromatography using silica gel or magnesium silicate and recrystallization. Purification may be carried out for each reaction or after completion of some reactions.

### [Toxicity]

The toxicity of the compound of the present invention is very low, and it is believed that the compound is safe enough for pharmaceutical use.

### INDUSTRIAL APPLICABILITY

### [Application to Pharmaceuticals]

Since the compound of the present invention of formula (I) or salts thereof has an inhibitory activity of DPP IV, it is considered that it is useful as a preventive and/or therapeutic agent for type 2 diabetes mellitus, obesity, autoimmune disease, cancer metastasis, AIDS virus infection, dermatosis and benign prostatic hypertrophy.

A combination agent obtained by combining the compound of formula (I) or a non-toxic salt thereof with other medicaments may be administered to accomplish the following purposes:
1) to supplement and/or enhance the preventive and/or therapeutic effect of the present compound,
2) to improve the kinetics and/or absorption and reduce the dose of the present compound, and/or
3) to eliminate the side effects of the present compound.

A combination of the compound of formula (I) and other medicaments may be administered in the form of the formulations having these components incorporated in one preparation, or may be administered in separate preparations. In the case where these medicaments are administered in separate preparations, they may be administered simultaneously or at different times. In the latter case, the compound represented by formula (I) may be administered before the other medicaments. Alternatively, the other medicaments may be administered before the compound represented by formula (I). The method for the administration of these medicaments are the same or different.

The diseases on which the preventive and/or therapeutic effect of the above described combination preparations works are not specifically limited but may be those for which the preventive and/or therapeutic effect of the compound represented by formula (I) is supplemented and/or enhanced.

Furthermore, the other medicaments for supplementing and/or enhancing the action of the compound of formula (I) and for potentiation of therapeutic efficacy of diabetic complication remedy include, for example, hypoglycaemic agent of sulfonylurea, biguanide, α-glucosidase inhibitor, acute effect type insulin secretagogue, insulin sensitivity promoter, insulin formulation, PPAR agonist, β3 adrenoceptor agonist, aldose reductase.

Examples of sulfonylurea include acetohexamide, glibenclamide, gliclazide, glyclopyramide, chlorpropamide, tolazamide, tolbutamide, Glimepiride, and the like.

Examples of biguanide include Buformin Hydrochloride, Metformin Hydrochloride and the like.

Examples of α-glucosidase inhibitor include acarbose, voglibose and the like.

Examples of fast-acting insulin secretagogue include nateglinide, repaglinide and the like.

Examples of insulin sensitivity promoter include ONO-5816, YM-440, JTT-501, NN-2344 and the like.

Examples of PPAR agonist include pioglitazone, troglitazone, rosiglitazone and the like.

Examples of β3 adrenoceptor agonist include AJ9677, troglitazone, L750355, CP331648 and the like.

Examples of aldose reductase inhibitor include Epalrestat, Fidarestat, Zenarestat.

The compounds represented by formula (I) and salts thereof may be combined with for example MTP (Microsomal Triglyceride Transfer Protein) inhibitor, HMG-CoA reductase inhibitor, squalene synthetase inhibitor, fibrate (fibrin acid derivative), ACAT (acyl-CoA:cholesterol O-acyl transferase) inhibitor, 5 - lipoxygenase inhibitor, cholesterol uptake inhibitor, bile acid uptake inhibitor, ileum Na+/bile acid cotransporter (ileal Na+/bile acid transporter ; IBAT) inhibitor, LDL receptor activator/expression promoter, lipase inhibitor, probucol formulation, nicotinic acid formulation, ohter antihypercholesterolemia therapeutic agent in order of complement or potentiation of lipid lowering action.

Examples of MTP inhibitor include BMS-201038, BMS-212122, BMS-200150, GW-328713, R-103757 and the like.

Examples of HMG-CoA reductase inhibitor include atorvastatin, fluvastatin, lovastatin, pravastatin, simvastatin, pitavastatin, Rosuvastatin and the like.

Examples of ACAT inhibitor include F-12511, F-1394, CI-1011, melinamide and the like.

Examples of squalene synthetase inhibitor include TAK-475 and the like.

Examples of fibrate include gemfibrozil, clofibrate, clofibrate aluminum, clinofibrate, simfibrate, bezafibrate, fenofibrate and the like.

Examples of ACAT inhibitor include CI-1011, FCE27677, RP73163 and the like.

Examples of cholesterol uptake inhibitor include SCH48461 and the like.

Examples of bile acid uptake inhibitor include colestyramine, colesevelam and the like.

Examples of LDL receptor activator/enhanced expression agent include MD-700, LY295427 and the like.

Examples of lipase inhibitor include orlistat and the like. The ratio by mass of the compounds of formula (I) to other drugs is not particularly limited.

Two or more of other drugs optionally selected can be used in combination.

Other drugs to be used for complementing and/or enhancing the preventive and/or therapeutic effects of the compounds of formula (I) involve not only those which have been found out hitherto based on the above-described mechanism but also those which will found out in future.

To employ the compounds of formula (I) or combination drugs of the compounds of formula (I) with other drugs for the above-described purposes, they are usually administered systemically or topically, and orally or parenterally.

Although the administration dose varies depending on the age, body weight and conditions of a patient, therapeutic effect, administration route, treatment time, and the like, the single administration dose to an adult usually ranges from 1 ng to 100 mg and the administration is made once to several times per day in the case of oral administration. Alternatively, the single administration dose ranges from 0.1 ng to 10 mg and the administration is made once to several times per day in the case of parenteral administration. Alternatively, intravenous administration is continuously made for 1 hour to 24 hours per day.

Needless to say, the administration dose varies depending on various factors as described above. Thus, an administration dose smaller than the lower limit as defined above is enough in some cases, while an administration dose exceeding the upper limit is needed in other cases.

To administrate the compounds of formula (I) or combination drugs of the compounds of formula (I) with other drugs, use is made of solid preparations for internal use and liquid preparations for internal use for oral administration as well as injections, preparations for external use, suppositories, and the like for parenteral administration.

Examples of the solid preparations for internal use include tablets, pills, capsules, dusts, granules and the like. The capsules include hard capsules and soft capsules.

Such a solid preparation for internal use is prepared by a formulation method commonly employed by using one or more active substances either as such or as a mixture with an excipient (lactose, mannitol, glucose, microcrystalline cellulose, starch, and the like), a binder (hydroxypropylcellulose, polyvinylpyrrolidone, magnesium metasilicate aluminate, and the like) a disintegrating agent (calcium cellulose glycolate, and the like), a lubricant (magnesium stearate, and the like), a stabilizer, and a dissolution aid (glutamic acid, aspartic acid, and the like). If necessary, it may be coated with a coating agent (sucrose, gelatin, hydroxypropylcellulose, hydroxypropylmethylcellulose phthalate, and the like). It may be coated with two or more layers. Moreover, capsules made of an absorbable material such as gelatin are involved in the scope thereof.

The liquid preparations for internal use involve pharmaceutically acceptable solutions, suspensions, emulsions, syrups, elixirs and the like. Such a liquid preparation is prepared by dissolving, suspending or emulsifying one or more active substances in a diluent commonly employed (purified water, ethanol, a mixture thereof, and the like). The liquid preparation may further contain a moistening agent, a suspending agent, an emulsifier, a sweetener, a flavor, a perfume, a preservative, a buffer and the like.

Injections for use in parenteral administration include sterile aqueous, suspension, emulsion and solid forms used by dissolving or suspending in solvent before use. Injection is used by dissolving, suspending, or emulsifying one or more active substances in solvent. Examples of solvent include distilled water for injection, physiological saline, vegetable oil, propylene glycol, polyethylene glycol, alcohols such as ethanol and combinations thereof. Furthermore, this injection may contain stabilizer, solubilizer (glutamic acid, aspartic acid, polysorbate 80 (registered trademark), and the like), suspending agent, emulsifying agent, analgesic agent, buffering agent, preservative agent, and the like. They may also be manufactured in the form of sterile solid forms, for example, freeze-dried products, which may be dissolved in sterile water or some other sterile diluent(s) for injection immediately before use.

The dosage forms of the parenteral administration preparations for external use involve ointments, gels, creams, fomentations, patches, liniments, atomized agents, inhalations, sprays, aerosols, nasal drops and the like. Such a preparation contains one or more active substances and is prepared by a publicly known method or in accordance with a formulation commonly employed.

Ointments are prepared in accordance with a publicly known formulation or a formulation commonly employed. For example, they are prepared by levigating or melting one or more active substances in a base. The ointment base is selected from among publicly known ones or those commonly employed. For example, use may be made of one base or a mixture of two or more thereof selected from higher fatty acids or higher fatty acid esters (adipic acid, myristic acid, palmitic acid, stearic acid, oleic acid, adipic acid esters, myristic acid esters, palmitic acid esters, stearic acid esters, oleic acid esters, and the like), waxes (beeswax, whale wax, ceresin, and the like), surfactants (polyoxyethylene alkyl ether phosphoric acid esters, and the like), higher alcohols (cetanol, stearyl alcohol, cetostaryl alcohol, and the like), silicone oils (dimethylpolysiloxane, and the like), hydrocarbons (hydrophilic vaseline, white vaseline, refined lanolin, liquid paraffin, and the like), glycols (ethylene glycol, diethylene glycol, propylene glycol, polyethylene glycol, macrogol, and the like), vegetable oils (castor oil, olive oil, sesame oil, turpentine oil, and the like), animal oils (mink oil, yolk oil, squalane, squalene, and the like), water, absorption promoters and skin irritation inhibitors. The ointments may further contain a humectant, a preservative, a stabilizer, an antioxidant, a flavor, and the like

Gels are prepared in accordance with a publicly known formulation or a formulation commonly employed. For example, they are prepared by melting one or more active substances in a base. The gel base is selected from among publicly known ones or those commonly employed. For example, use may be made of one base or a mixture of two or more thereof selected from among lower alcohols (ethanol, isopropyl alcohol, and the like), gelling agents (carboxymethylcellulose, hydroxyethylcellulose, hydroxypropylcellulose, ethylcellulose, and the like), neutralizing agents (triethanolamine, diisopropanolamine, and the like), surfactants (polyethylene glycol monostearate, and the like), gums, water, absorption promoters and skin irritation inhibitors. The gels may further contain a preservative, an antioxidant, a flavor, and the like.

Creams are prepared in accordance with a publicly known formulation or a formulation commonly employed. For example, they are prepared by melting or emulsifying one or more active substances in a base. The cream base is selected from among publicly known ones or those commonly employed. For example, use may be made of one base or a mixture of two or more thereof selected from among higher fatty acid esters, lower alcohols, hydrocarbons, polyhydric alcohols (propylene glycol, 1,3-butylene glycol, and the like), higher alcohols (2-hexyldecanol, cetanol, and the like), emulsifiers (polyoxyethylene alkyl ethers, fatty acid esters, and the like), water, absorption promoters and skin irritation inhibitors. The creams may further contain a preservative, an antioxidant, a flavor, and the like.

Fomentations are prepared in accordance with a publicly known formulation or a formulation commonly employed. For example, they are prepared by melting one or more active substances in a base, kneading and then applying and spreading the kneaded matter on a substrate. The fomentation base is selected from among publicly known ones or those commonly employed. For example, use may be made of one base or a mixture of two or more thereof selected from among thickeners (polyacrylic acid, polyvinylpyrrolidone, acacia, starch, gelatin, methylcellulose, and the like), moistening agents (urea, glycerol, propylene glycol, and the like), fillers (kaolin, zinc oxide, talc, calcium, magnesium, and the like), water, dissolution aids, tackifiers and skin irritation inhibitors. The fomentations may further contain a preservative, an antioxidant, a flavor, and the like.

Patches are prepared in accordance with a publicly known formulation or a formulation commonly employed. For example, they are prepared by melting one or more active substances in a base and then applying and spreading on a substrate. The patch base is selected from among publicly known ones or those commonly employed. For example, use may be made of one base or a mixture of two or more thereof selected from among polymer bases, fats and oils, higher fatty acids, tackifiers and skin irritation inhibitors. The patches may further contain a preservative, an antioxidant, a flavor, and the like.

Liniments are prepared in accordance with a publicly known formulation or a formulation commonly employed. For example, they are prepared by dissolving, suspending or emulsifying one or more active substances in one or more media selected from among water, alcohols (ethanol, polyethylene glycol, and the like), higher fatty acids, glycerol, soap, emulsifiers, suspending agents and the like. The liniments may further contain a preservative, an antioxidant, a flavor, and the like

Atomized agents, inhalations, sprays and nasal drop may contain, in addition to a diluent commonly employed, a stabilizer such as sodium hydrogen sulfite, a buffer for imparting isotonicity, for example, an isotonic agent such as sodium chloride, sodium citrate or citric acid. Methods for producing a spray are described in detail in, for example, U.S. Patent 2,868,691 and U.S. Patent 3,095,355.

When a nasal drop is administered, it is usually administered by spraying (atomizing) solution or powder containing drug into nasal cavity with a dedicated nasal drip apparatus or atomizer quantitatively.

Eye drops for parenteral administration may be in the form of liquid, suspension, emulsion, liquid dissolved in a solvent in use or ointment.

These eye drops are prepared by any known method. For Example, one or more active substances are dissolved, suspended or emulsified in a solvent. As such a solvent for eye drops, there may be used sterilized purified water, physiological saline and other aqueous solvents or nonaqueous solvents for injection (vegetable oils, and the like), singly or in combination thereof. The eye drops may contain ones selected from an isotonic agent (sodium chloride, concentrated glycerin, and the like), a buffering agent (sodium phosphate, sodium acetate, and the like), a surfactant (Polysolvate 80 (trade name), Polyoxyl stearate 40, polyoxyethylene-hydrogenated castor oil, and the like), a stabilizer (sodium citrate, sodium edetate, and the like), a preservative (benzalconium chloride, paraben, and the like), and the like. The eye drops are sterilized at the final step or prepared by an aseptic process. Alternatively, an aseptic solid agent such as freeze-dried product which has previously been prepared may be rendered aseptic or dissolved in aseptic distilled water for injection or other solvent before use.

The dosage of inhalations for parenreral administration include aerosol, powders for inhalation or liquids for inhalation. The liquids for inhalation may be dissolved or suspended in water or the other appropriate solvent as needed.

Such inhalations are prepared in a known method. For example, a liquid for inhalation is prepared by selecting proper additives from an antiseptic (benzalkonium chloride, *p*-aminobenzonic acid and the like), a coloring agent, a buffering agent (sodium phosphate, sodium acetate and the like), an isotonizing agent (sodium chloride, concentrated glycerin and the like), thickening agent (carboxyvinylpolymer and the like), or an accelerator of absorption, and the like, if necessary.

A powder for inhalation is prepared by selecting proper additives from a lubricant agent (such as stearin acid and the salt thereof), a binding agent, (starch, dextrin and the like), a diluting agent (lactose, cellulose and the like), a coloring agent, an antiseptic (benzalkonium chloride, p-aminobenzonic acid and the like), an accelerator of absorption, and the like, if necessary.

In case of administration of liquid for inhalation, spray (atomizer, nebulizer) is usually used and in case of administration of powder for inhalation, inhalation administration apparatus for powder agents is usually used.

The other compositions for parenteral administration include suppositories for intrarectal administration and pessaries for vaginal administration which comprise one or more of the active substance(s) and may be prepared by methods known per se.

### BEST MODE FOR CARRYING OUT THE INVENTION

The present invention is explained below in detail based on Reference Examples and Examples, however, the present invention is not limited thereto.

The solvents in the parentheses show the developing or eluting solvents and the ratios of the solvents used are by volume in chromatographic separations or TLC. The solvents in the parentheses in NMR show the solvents for measurement. In addition, dimethyl sulfoxide represents dimethyl sulfoxide, DMF represents N,N-dimethylformamide and THF represents tetrahydrofuran.

The name of the following compounds is designated according to ACD Labs 6.0 Name.

### Reference Example 1

### (2S)-1-[(2S,4S)-N-(t-butoxycarbonyl)-4-phenylpyrrolidin-2-ylcarbonyl]pyrrolidine-2-carbonitrile

To a solution of (2S,4S)-*t*-butylcarbonyl-4-phenylpyrrolidine-2-carboxylic acid (524 mg), benzotriazolylmethanesulfonate (426 mg) and 2-cyanopyrrolidine hydrochloride (264 mg) in DMF (3 ml), triethylamine (0.56 ml) was added dropwise at 0 °C. The mixture was stirred for 3 hours at room temperature. The reaction mixture was diluted with ethyl acetate, washed with 1 M hydrochloric acid, 1 M aqueous solution of sodium hydroxide, a saturated aqueous solution of sodium bicarbonate and a saturated aqueous solution of sodium chloride, dried and concentrated. The residue was purified by column chromatography to give the title compound (440 mg).

### Reference Example 2

### (2RS,4R)-2-phenylthiazolidine-4-carboxylic acid

To a solution of benzaldehyde (1.06 g) in methanol, (12 ml), a solution of L-cysteine (1.21 g) in water (10 ml) was added. The mixture was stirred for 1 hour. The precipitate filtrated from the reaction mixture was washed with methanol-water (1:1) and dried to give the title compound (1.80 g).

### Reference Example 3

### (2RS,4R)-3-t-butoxycarbonyl-2-phenylthiazolidine-4-carboxylic acid

To a solution of the compound prepared in Reference Example 2 (1.79 g) in ethanol (9 ml) and 1 M aqueous solution of sodium hydroxide (9 ml), di-*t*-butyl dicarbonate (1.87 g) was added. The mixture was stirred for 5 hours at room temperature. The reaction mixture was concentrated. 1N hydrochloric acid (9.5 ml) was added to the residue. The mixture extracted with ethyl acetate. The organic layer was dried and concentrated. The residue was washed with diisoprpyl ether and dried to give the title compound (2.40 g).

### Reference Example 4

### (2E,4R)-4-(t-butoxycarbonylamino)-4-phenylbutenbutenoic acid methyl ester

A solution of (2-(N-*t*-butoxycarbonylamino)-2-phenylethanol) (14.1 g) and Dess Martin reagent (1,1,1-triacetoxy)-1,1-dihydro-1,2-benziodoxol-3(1H)-on) (25.3 g) in dichloromethane (200 ml) was stirred for 1 hour at room temperature. (Methoxycarbonylmethylene)triphenylphosphorane was added to the mixture and the mixture was stirred for 1 hour. The reaction mixture was concentrated. Toluene was added to the residue. The mixture was filtrated. The filtrate was concentrated. The residued was purified by column chromatography to give the title compound (14.7 g).

### Reference Example 5

### (4R)-4-(t-butoxycarbonylamino)-4-phenylbutanoic acid methyl ester

To the solution of the compound (14.7 g) prepared in Reference Example 4 in ethanol (250 ml), 10% palladium carbon (1.5 g) was added. Under an atmosphere of hydrogen, the mixture was stirred for 6 hours at room temperature. The reaction mixture was filtrated. The filtrate was concentrated to give the title compound (11.05 g).

### Reference Example 6

### (2RS,5R)-2-hydroxy-5-phenylpyrrolidine-1-carboxylic acid t-butyl ester

To the solution of the compound prepared in Reference Example 5 (5.19 g) in toluene (100 ml), diisobutylaluminum hydride (1.01 N toluene solution; 17.5 ml) was slowly added at -70 °C. The mixture was stirred for 2 hours. A saturated aqueous solution of sodium sulfate was added to the reaction mixture, and then the mixture was filtrated at room temperature. The filtrate was extracted with toluene. The organic layer was dried and concentrated to give the title compound.

### Reference Example 7

### (2RS,5R)-2-methoxy-5-phenylpyrrolidine-1-carboxylic acid t-butyl ester

To the solution of the compound prepared in Reference Example 6 in methanol (80 ml), *p*-toluenesulfonic acid monohydrate (168 mg) was added. The mixture was stirred for 40 minutes. A saturated aqueous solution of sodium bicarbonate was added to the reaction solution. The mixture was concentrated, extracted with ethyl acetate, and dried and concentrated. The residue was purified by column chromatography to give the title compound (2.56 g).

### Reference Example 8

### (2RS,5R)-2-cyano-5-phenylpyrrolidine-1-carboxylic acid t-butyl ester

To the solution of the compound prepared in Reference Example 7 (2.56 g) and trimethylsilyl cyanide (1.83 g) in dichloromethane (100 ml), boron trifluoride ether complex (2.62 g) was added dropwise at -78 °C. The mixture was stirred for 15 minutes. A saturated aqueous solution of sodium bicarbonate was added to the reaction mixture. The mixture was warmed to room temperature. The aqueous layer was extracted with ethyl acetate. The organic layer was dried and concentrated to give the title compound (2.42 g).

### Reference Example 9

### (2RS,5R)-1-(t-butoxycarbonyl)-5-phenylpyrrolidine-2-carboxylic acid

To the solution of the compound prepared in Reference Example 8 (1.59 g) in methanol (50 ml), 5N aqueous solution of sodium hydroxide (30 ml) was added. The mixture was stirred for 3 hours at 75 °C, overnight at room temperature and then for 3 hours at 75 °C. The reaction mixture was concentrated. The residue was extracted with ethyl acetate. The organic layer was dried and concentrated to give the title compound (2.42 g).

### Reference Example 10

### (3R)-1-thia-4-azaspiro[4.4]nonane-3-carboxylic acid methyl ester

To a solution of L-cysteine methyl ester hydrochloride (3.00 g) in ethanol (20 ml), cyclopentanone (1.70 ml) was added. The mixture was stirred for 3 hours at 70 °C. The reaction mixture was concentrated. A saturated aqueous solution of sodium bicarbonate was added to the residue. The mixture was extracted with ethyl acetate. The organic layer was washed with water and a saturated aqueous solution of sodium chloride successively, dried and concentrated. The residue was purified by column chromatography to give the title compound (2.01 g).

### Reference Example 11

### (3R)-1-thia-4-azaspiro[4.4]nonane-3-carboxylic acid

To the solution of the compound prepared in Reference Example 10 (2.01g) in methanol (20 ml), 1 N aqueous solution of sodium hydroxide (15 ml) was added at 0 °C. The mixture was stirred for 3 hours. After 1N hydrochloric acid (15 ml) was added to the reaction mixture, the mixture was concentrated to give the title compound (2.74 g).

### Reference Example 12

### (2-aminomethyl-indan-2-yl)methanol

To a suspension of lithium aluminum hydride (1.71 g) in diethyl ether (50 ml), a solution of 2-cyanoindan-2-carboxylic acid ethyl ester (6.02 g) in tetrahydrofuran (20 ml) was added dropwise. The mixture was refluxed for 3 hours. The reaction mixture was cooled to 0 °C. Water (1.7 ml), 15% aqueous solution of sodium hydroxide aqueous solution (1.7 ml) and water (5.1 ml) were dropped successively to the reaction mixture. The mixture was stirred for 30 minutes at room temperature. The reaction mixture was filtrated. The filtrate was concentrated to give the title compound (4.45 g).

### Reference Example 13

### 2-t-butoxycarbonylaminomethylindan-2-ylmethanol

To the suspension of the compound prepared in Reference Example 12 (4.45 g) in 1,4-dioxane (40 ml), di-*t*-butyl dicarbonate (5.49g) was added. The mixture was stirred for 4 hours at room temperature. The reaction mixture was diluted with ethyl acetate. The mixture was washed with water and a saturated aqueous solution of sodium chloride successively, dried and concentrate. The residue was recrystallized from *t*-butylmethyl ether to give the title compound (3.63 g).

### Reference Example 14

### 2-t-butoxycarbonyl aminomethylindan-2-ylcarbaldehyde

To the solution of the compound prepared in Reference Example 13 (4.60 g) in DMSO (80 ml), triethylamine (14 ml) was added little by little. The mixture was stirred for 2 hours at room temperature. The reaction mixture was poured into iced water and the mixture was extracted with ethyl acetate. The organic layer was washed with a saturated aqueous solution of sodium chloride, dried and concentrated. The residue was purified by column chromatography to give the title compound (4.21 g).

### Reference Example 15

### [2-(2-methoxyvinyl)indan-2-yl]methylcarbamic acid t-butyl ester (a mixture of E, Z isomer)

To a suspension of (methoxymethyl)triphenylphosphonium chloride (26.2 g) in THF (75 ml), a solution of potassium-*t*-butoxide (8.57 g) in *t*-butanol (95 ml) was dropped. The mixture was stirred for 1 hour. In addition, a solution of the compound prepared in Reference Example 14 (4.20 g) in THF.(10 ml) was added dropwise to the mixture. The mixture was stirred for 1 hour at 0 °C. Water (50 ml) was added to the reaction mixture. The mixture was extracted with ethyl acetate (20 ml). The organic layer was washed with water and a saturated aqueous solution of sodium chloride successively, dried and concentrated. The residue was purified by column chromatography to give the title compound (3.87 g).

### Reference Example 16

### (2'RS)-2'-methoxy spiro[indan-2,4'-pyrrolidine]-1'-carboxylic acid t-butyl ester

To the solution of the compound prepared in Reference Example 15 (3.87 g) in methanol (16 ml) and water (2 ml), *p*-toluenesulfonic acid monohydrate (300 mg) was added. The mixture was stirred for 15 hours at room temperature. A saturated aqueous solution of sodium bicarbonate (10 ml) was added to the reaction mixture. The mixture was concentrated. The residue was extracted with ethyl acetate. The organic layer was washed with a saturated aqueous solution of sodium chloride, dried and concentrated. The residue was recrystallized from *t*-butylmethyl ether to give the title compound (2.84 g).

### Reference Example 17

### (2'RS)-2'-cyanospiro[indan-2,4'-pyrrolidine]-1'-carboxylic acid t-butyl ester

To a solution of the compound prepared in Reference Example 16 (3.47 g) in dichloromethane (30 ml), cyanotrimethylsilane (3.4 ml) and boron trifluoride diethyl ether complex (3.2 ml) were successively added at -78 °C. The mixture was stirred for 90 minutes at -78 °C. A saturated aqueous solution of sodium bicarbonate (20 ml) was added to the reaction mixture. The organic layer was washed with a saturated aqueous solution of sodium chloride, dried and concentrated. The residue was purified by column chromatography to give the title compound (2.97 g).

### Reference Example 18

### (2'RS)-spiro[indan-2,4'-pyrrolidine]-2'-carboxylic acid hydrochloride

The suspension of the compound prepared in Reference Example 17 (2.96 g) in concentrated hydrochloric acid (25 ml) was refluxed for 15 hours. The reaction mixture was concentrated. Ethanol was added to the residue and the mixture was concentrated. Ethanol was also added to the residue and the mixture was filtrated. The filtrate was concentrated to give the title compound (2.80 g).

### Reference Example 19

### (2'RS)-1'-(t-butoxycarbonyl)-spiro[indan-2,4'-pyrrolidine]-2'-carboxylic acid

In 1,4-dioxane (20 ml) and 1M sodium hydroxide aqueous solution, the compound prepared in Reference Example 18 (2.80 g) was dissolved (20 ml). A solution of di-*t*-butyl dicarbonate (2.18 g) in 1,4-dioxane (5 ml)to the solution. The mixture was stirred for 6 hours at room temperature. The mixture was adjusted pH 2 by adding 5% aqueous solution of potassium bisulfate. The mixture was extracted with ethyl acetate. The organic layer was dried and concentrated. The residue was recrystallized from *t*-butylmethyl ether to give the title compound (2.75 g).

### Reference Example 20

### (2S)-1-(t-butoxycarbonyl)-4-oxopyrrolidine-2-carboxylic acid

The title compound (11.9 g) was obtained, using N-*t*-butoxycarbonylhydroxyproline (22.3 g), by the same procedure as a series of reactions of Reference Example 14.

### Reference Example 21

### (2S,4E)-4-benzylidene-N-(t-butoxycarbonyl)pyrrolidine-2-carboxylic acid

To a suspension of sodium hydride (1.15 g) in DMSO (30 ml), a solution of benzyltriphenylphosphonium chloride (11.7 g) in DMSO (30 ml) was added. The mixture was stirred at 70 °C. After the mixture became uniform, the mixture was cooled to room temperature. A solution of the compound prepared in Reference Example 20 (2.29 g) in DMSO (10 ml) was added dropwise to the mixture. The mixture was stirred for 3 hours at 70 °C and overnight at room temperature. The reaction mixture was added to an iced aqueous solution of potassium hydrogen carbonate (2 g). After the mixture was washed with diethyl ether, the aqueous layer was acidified by adding 1 N hydrochloric acid and was extracted with ethyl acetate. The organic layer was concentrated. The residue was dissolved into 1N aqueous solution of sodium hydride. The mixture was washed with diethyl ether. The aqueous layer was acidified by adding 1 N hydrochloric acid and the mixture was extracted with ethyl acetate. The organic layer was washed with a saturated aqueous solution of sodium chloride, dried and concentrated. The residue was dissolved in ethyl acetate and dicyclohexylamine was added to the mixture. The precipitate was obtained by filtration, washed with ethyl acetate and dried. Ethyl acetate and 1N hydrochloric acid were added to the obtained solid. The organic layer was washed with a saturated aqueous solution of sodium chloride, dried and concentrated to give the title compound (1.20 g).

### Reference Example 22

### (2S,4RS)-4-benzyl-1-(t-butoxycarbonyl)pyrrolidine-2-carboxylic acid

The title compound was obtained, using the compound prepared in Reference Example 21 by the same procedure as in Reference Example 5.

### Reference Example 23

### (2S)-1-(t-butoxycarbonyl)-4-trifluoromethylsulfonyloxy-2,5-dihydro-1H-pyrrole carboxylic acid methyl ester

Under an atmosphere of argon, to a solution of sodium hexamethyldisilazane (27.3 g) in THF (150 ml), a solution of (2S)-N-(*t*-butoxycarbonyl)-4-oxopyrrolidin-2-carboxymethyl (Synthesis, 1986, 81) (30.2 g) in THF (50 ml) was dropped at -78 °C. The mixture was stirred for 30 minutes at -78 °C. A solution of N-phenyl trifluoromethanesulfone imide (48.7 g) in THF (120 ml) was added dropwise to the mixture. The mixture was stirred for 4 hours at the same temperature. A saturated aqueous solution of sodium bicarbonate was added to the reaction mixture. The mixture was warmed to room temperature and extracted with ethyl acetate. The organic layer was washed with water and a saturated aqueous solution of sodium chloride successively, dried and concentrated. The residue was purified by column chromatography to give the title compound (12.0 g).

### Reference Example 24

### (2S)-1-(t-butoxycarbonyl)-4-(4-trifluoromethylphenyl)-2,5-dihydro-1H-pyrrole-2- carboxylic acid methyl ester

To a solution of the compound prepared in Reference Example 23 (1.00 g) in dioxane (27 ml), 4-trifluoromethylbenzeneboronic acid (392 mg), 2M potassium carbonate solution (3 ml) and tetrakis(triphenylphosphine)palladium (0) (77 mg) were added. The mixture was refluxed for 30 minutes. Water was added to the reaction mixture. The mixture was extracted with ethyl acetate. The organic layer was washed with water and a saturated aqueous solution of sodium chloride successively, dried and concentrated. The residue was purified by column chromatography to give the title compound (1.00 g).

### Reference Example 25

### (2S,4R)-1-(t-butoxycarbonyl)-4-(4-trifluoromethylphenyl)pyrrolidine-2-carboxylic acid methyl ester

The title compound (684mg) was obtained, using the compound prepared in Reference Example 24 (1.00 g), by the same procedure as in Reference Example 5.

### Reference Example 26

### (2S,4R)-1-(t-butoxycarbonyl)-4-(4-trifluoromethylphenyl)pyrrolidine-2-carboxylic acid

The title compound (552 mg) was obtained, using the compound prepared in Reference Example 25 (684 mg), by the same procedure as in Reference Example 11.

### Reference Example 27

### (4R)-3-[(4R)-3-(t-butoxycarbonyl)-2-phenylthiazolidin-4-ylcarbonyl]thiazolidine-4-carboxamide

To a solution of (2RS,4R)-3-*t*-butoxycarbonyl-2-phenylthiazolidine-4-carboxylic acid (1.55 g) in DMF (10 ml), triethylamine (607 mg), thiazolidine-4-carbamide hydrochloride (Bioorg. Med. Chem. Lett. Vol.6, No.22, 2745-2748, 1996 ; 843mg), 1-hydroxybenzotriazole monohydrate (919 mg) and 1-ethyl-3-[3-(dimethylamino)propyl]carbodiimide hydrochloride (1.15 g) were successively added at room temperature. The mixture was stirred overnight. The reaction mixture was poured into iced water and the mixture and was extracted with ethyl acetate. The organic layer was washed with 1 N hydrochloric acid, a saturated aqueous solution of sodium bicarbonate and a saturated aqueous solution of sodium chloride successively, dried and concentrated. The residue was purified by column chromatography to give the title compound (1.62 g).

### Reference Example 28

### (4R)-3-[(2RS,4R)-3-(t-butoxycarbonyl)-2-phenylthiazolidin-4-ylcarbonyl]thiazolidine-4-carbonitrile

Under an atmosphere of argon, to a solution of the compound prepared in Reference Example 27 (1058 mg) and imidazole (340 mg) in pyridine (10 ml), phosphorus oxychloride (0.93 ml) was added dropwise slowly at -40 °C. The mixture was stirred for 30 minutes at the same temperature. The reaction mixture was poured into ice, the mixture was acidified by adding 2N hydrochloric acid and extracted with ethyl acetate. The organic layer was washed with 1N hydrochloric acid, a saturated aqueous solution of sodium bicarbonate and a saturated aqueous solution of sodium chloride successively, dried and concentrated. The residue was purified by column chromatography to give the title compound (729 mg).

### Reference Example 29

### (2S,4S)-4-allyl-1-(t-butoxycarbonyl)-pyrrolidine-2-carboxylic acid methyl ester

To a solution of (2S,4S)-4-allyl-1-(*t*-butoxycarbonyl)-5-oxo-pyrrolidine-2-carboxylic acid methyl ester (Tetrahedron Letters, 1998, 39, 5887-5890) (2.88 g) in THF (55 ml), lithium triethylborohydride (1M THF solution; 12.2 ml) was added at -78 °C. The mixture was stirred for 40 minutes. A saturated aqueous solution of sodium bicarbonate (20 ml) was added to the reaction mixture and raised the temperature at 0 °C. 35% hydrogen peroxide water (2 ml) was added to the mixture and stirred for 20 minutes at 0 °C. The reaction mixture was concentrated. The residue was extracted with dichloromethane, dried and concentrated. The residue was dissolved again into dichloromethane. Triethylsilane (1.7 ml) and boron trifluoride diethyl ether complex (1.5 ml) were added to the mixture at -78 °C. The mixture was stirred for 150 minutes. A saturated aqueous solution of sodium bicarbonate (50 ml) was added to the reaction mixture. The mixture was extracted with dichloromethane at room temperature, dried and concentrated. The residue was purified by column chromatography to give the title compound (2.45 g).

### Reference Example 30

### (2RS)-1-(t-butoxycarbonyl)-4-benzyloxycarbonyl-2,5-dihydro-1H-pyrrole-2-carboxylate methyl ester

Under an atmosphere of carbon monoxide, a solution of the compound prepared in Reference Example 23 (6.54 g), benzylalcohol (2.7 ml), triethylamine (5 ml), triphenylphosphine (548 mg) and palladium acetate (234 mg) in DMF (30 ml) were stirred for 15 hours at room temperature. The reaction mixture was poured into water. The mixture was extracted with ethyl acetate three times. The combined organic layer was washed with 1N hydrochloric acid, a saturated aqueous solution of sodium bicarbonate and a saturated aqueous solution of sodium chloride successively, dried and concentrated. The residue was purified by column chromatography to give the title compound (3.22 g).

### Reference Example 31

### cis-(2RS,4RS)-1-(t-butoxycarbonyl)-2-methoxycarbonyl-pyrrolidine-4-carboxylic acid

The compound prepared in Reference Example 30 (3.21 g) was dissolved in methanol (30 ml), and 10% palladium carbon (50% water content; 850 mg) was added to the mixture. The mixture was stirred for 90 minutes under an atmosphere of hydrogen gas. The reaction mixture was filtrated. The filtrate was concentrated to give the title compound (2.55 g). The title compound was racemate mixture whose relative configuration was cis.

### Reference Example 32

### cis-(2RS,4RS)-1-(t-butoxycarbonyl)-4-(5-methyl-oxadiazol-2-yl)-pyrrolidine-2- carboxylic acid methyl ester

To a mixture of the compound (819 mg) prepared in Reference Example 31 and triethylamine (0.46 ml), chloroethyl formate (0.32 ml) was added dropwise at 0 °C. The mixture was for 30 minutes. Furthermore acetohydrazide (244 mg) was added to the mixture. The mixture was stirred for 2 hours at 0 °C and then for 15 hours at room temperature. The reaction mixture was diluted with ethyl acetate, washed with a saturated aqueous solution of sodium bicarbonate and a saturated aqueous solution of sodium chloride successively, dried and concentrated. The residue was dissolved in THF (30 ml). Pyridine (0.51 ml) and thionyl chloride (0.23 ml) were added to the solution at 0 °C. The mixture was stirred for 2 hours at 0 °C. The reaction mixture was filtrated. The filtrate was concentrated. The residue was dissolved into toluene (30 ml) and the solution was refluxed for 2 hours. The reaction mixture was concentrated. The residue was purified by column chromatography to give the title compound (398 mg).

### Reference Example 33

### cis-(2RS,4RS)-1-(t-butoxycarbonyl)-4-diazoacetylpyrrolidine-2-carboxylic acid methyl ester

To the mixture of the compound prepared in Reference Example 31 (1.64 g) and triethylamine (0.92 ml), isobutyl chloroformate (0.86 ml) was added at -10 °C. The mixture was stirred for 90 minutes at -10 °C. The mixture was filtrated. The filtrate was added to a solution of diazomethane in ether at 0 °C. The mixture was stirred for 2 hours at the same temperature. The reaction mixture was concentrated. The residue was purified by column chromatography to give the title compound (1.10 g).

### Reference Example 34

### cis-(2RS,4RS)-1-(t-butoxycarbonyl)-4-(2-methyl-1,3-thiazol-4-yl)pyrrolidine-2-carboxylic acid methyl ester

To a solution of the compound prepared in Reference Example 33 (1.08 g) in THF (20 ml), 4N hydrogen chloride-dioxane solution (1 ml) was added at 0 °C. The mixture was stirred for 2 hours at the same temperature. Ethyl acetate (20 ml) was added to the reaction mixture. The mixture was washed with a saturated aqueous solution of sodium bicarbonate and a saturated aqueous solution of sodium chloride successively, dried and concentrated. The residue was dissolved in ethanol (7 ml). Thioacetamide (263 mg) was added to the solution. The mixture was stirred for 15 hours at room temperature. The reaction mixture was concentrated. A saturated aqueous solution of sodium bicarbonate was added to the residue. The reaction solution was extracted with ethyl acetate. The organic layer was washed with a saturated aqueous solution of sodium chloride, dried and concentrated. The residue was purified by column chromatography to give the title compound (870 mg).

### Reference Example 35

### t-butyl (2S,4R)-2-{[(2S)-2-cyanopyrrolidin-1-yl]carbonyl}-4-[2-(methoxycarbonyl) phenyl]pyrrolidine-1-carboxylic acid

The title compound was obtained, using its benzyl ester instead of the compound prepared in Reference Example 34 (methyl ester), by the same procedure as in Reference Example 24 →Reference Example 5 →Reference Example 1.

### Reference Example 36

### 2-((3R,5S)-1-(t-butoxycarbonyl)-5-{[(2S)-2-cyanopyrrolidin-1-yl]carbonyl}pyrrolidin-3-yl)benzoic acid

To a solution of the compound prepared in Reference Example 35 (540 mg) in dimethylformamide (90 ml), lithium iodide (3.0 g) was added. The mixture was refluxed for 15 hours. The reaction mixture was poured into water, washed with ethyl acetate. The aqueous layer was neutralized and extracted with ethyl acetate. The organic layer was dried, concentrated and purified by column chromatography on silica gel (chloroform/methanol) to give the title compound (198 mg).

### Reference Example 37

### t-butyl (2S,4R)-4-(2-t-butoxy-2-oxoethyl)-2-{[(2S)-2-cyanopyrrolidin-1-yl]carbonyl}pyrrolidine-1-carboxylic acid

The title compound was obtained, by the same procedure as in Reference Example 11 →Reference Example 1, using *t*-butyl N-*t*-butoxycarbonyl-5-methoxycarbonylpyrrolidin-3-ylacetic acid.

### Reference Example 38

### [4-((3R,5S)-5-{[(2S)-2-cyanopyrrolidin-1-yl]carbonyl}pyrrolidin-3-yl)phenoxy]butanoic acid benzyl ester

(a) The compound prepared in Example19 (28) was carried out, by the same procedure as Reference Example 3 (protective reaction of Boc) to give the title compound protected by N-Boc.
(b) The compound prepared in (a) (300 mg) was dissolved into benzene (8 ml). 4-hydroxybutanoic acid (182 mg), N,N,N',N'-tetramethylazo dicarboxamide (402 mg) and tributylphosphine (0.58 ml) were added to the mixture. The mixture was stirred for 15 hours at 60 °C. The reaction mixture was filtrated. The filtrate was diluted with ethyl acetate, washed with an aqueous solution of sodium hydroxide and a saturated aqueous solution of sodium chloride successively, dried and concentrated. The residue was purified by column chromatography on silica gel (hexane/ethyl acetate) to give the title compound (310 mg).

### Reference Example 39

### 1-t-butyl 2-methyl (2S, 4R)-4-(2-hydroxyethyl)pyrrolidine-1,2-dicarboxylate

To a solution of [(3R,5S)-1-(*t*-butoxycarbonyl)-5-(methoxycarbonyl) pyrrolidin-3-yl]acetic acid (J.Org.Chem.1989, 54, 109-115) (575 mg) in tetrahydrofuran (10 ml), triethylamine (0.42 ml) and chloroethyl formate (0.23 ml) were added at 0 °C. The mixture was stirred for 2 hours at room temperature. The reaction mixture was filtrated. The filtrate was concentrated. The residue was dissolved into tetrahydrofuran (5 ml). The solution was added dropwise to a solution of sodium borohydride (378 mg) in water (5 ml) which was cooled at 0 °C. The mixture was stirred for 1 hour at 0 °C. Water was poured into the reaction solution. The mixture was extracted with ethyl acetate, washed with water and a saturated aqueous solution of sodium chloride successively, dried and concentrated to give the title compound (540 mg).

### Reference Example 40

### 1-t-butyl 2-methyl

### (2S,4R)-4-[2-(tetrahydro-2H-pyran-2-yloxy)-ethyl]pyrrolidine-1,2-dicarbaxylate

To a solution of the compound prepared in Reference Example 39 (540 mg) in dichloromethane (4 ml), 3,4-dihydro-2H-pyran (0.27 ml) and pyridinium p-toluenesulfonate (50 mg) were added. The mixture was stirred for 3 hours at room temperature. A saturated aqueous solution of sodium bicarbonate was added to the reaction mixture. The solution was extracted with ethyl acetate, wash with water and a saturated aqueous solution of sodium chloride successively, dried and concentrated to give the title compound (710 mg).

### Reference Example 41

### t-butyl (2S,4R)-2-{[(2S)-2-cyanopyrrolidin-1-yl]carbonyl}-4-(2-hydroxyethyl) pyrrolidine-1-carboxylate

The following compounds were obtained, using the compound prepared in Reference Example 40, by the same procedure as in Reference Example 11 (NaOH) →Reference Example 1 (amidation). *p*-toluenesulfonic acid monohydrate (25 mg) was added to a solution of the obtained compound (548 mg) in methanol (3 ml). The mixture was stirred for 4 hours at room temperature. A saturated aqueous solution of sodium bicarbonate was added to the reaction mixture. The mixture was extracted with ethyl acetate, washed with water and a saturated aqueous solution of sodium chloride, dried and concentrated. The residue was purified by column chromatography on silica gel (hexane/ethyl acetate) to give the title compound (307 mg).

### Example 1

### (2S)-1-{[(2S,4S)-4-phenylpyrrolidin-2-yl]carbonyl}pyrrolidine-2-carbonitrile hydrochloride

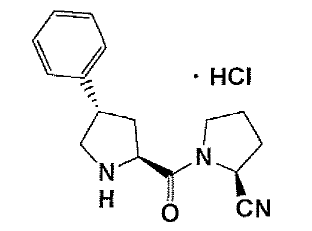

To a solution of the compound prepared in Reference Example 1 (421 mg) in ethyl acetate (4 ml), 4N hydrogen chloride-ethyl acetate solution (2 ml) was added and the mixture was stirred for 6 hours at room temperature. The precipitation was obtained by centrifugation. Its precipitation was washed with ethyl acetate to give the compound present invention (205 mg).
TLC: Rf 0.43(dichloromethane:methanol= 9:1);
NMR (DMSO-d₆): δ 2.16 (m, 4H), 2.44 (m, 1 H), 3.16 (m, 1 H), 3.43 (m, 1H), 3.62 (m, 4H), 4.75 (s, 1H), 4.84 (dd, J=7.97, 4.67Hz, 1 H), 7.33 (m, 5H), 8.93 (s, 1H), 10.76 (s, 1H).

### Example 1 (1) - Example 1 (4)

The following compound of present invention was obtained, by the same procedure as Example 1.

### Example 1 (1)

### (2S)-1-[(2S)-2,3-dihydro-1H-indol-2-ylcarbonyl]pyrrolidine-2-carbonitrile hydrochloride

### Example 1 (2)

### (2S)-1-[(2S)-piperidin-2-ylcarbonyl] pyrrolidine-2-carbonitrile trifluoroacetate

### Example 1 (3)

### (2S)-1-[(3S)-2,3,4,9-tetrahydro-1H-β-carbolin-3-ylcarbonyl]pyrrolidine-2-carbonitrile hydrochloride

### Example 1 (4)

### (2S)-1-[(6S)-4,5,6,7-[tetrahydro-3H-imidazo[4,5-c]pyridin-6-ylcarbonyl]pyrrolidine-2-carbonitrile

### hydrochloride

### Example 2

### (2S)-1-{[(4R)-2-phenyl-1,3-thiazolidin-4-yl]carbonyl}pyrrolidine-2-carbonitrile trifluoroacetate

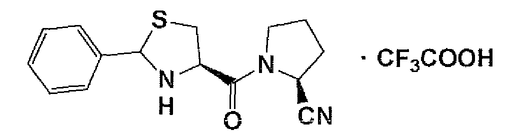

The compound of the present invention was obtained, using the compound prepared in Reference Example 3, by the same procedure as Reference Example 1 →Example 1 (Trifluoroacetic acid was used instead of hydrogen chloride-ethyl acetate which was used in Example 1.).
TLC: Rf 0.62 (dichloromethane:methanol= 19:1);
NMR (CDCl₃): δ 7.66-7.30 (m, 5H), 5.88 (s, 0.2H), 5.69 (s, 0.8H), 4.70 (m, 1H), 4.49 and 4.42 (t, J = 7.2Hz, 1H), 3.76-3.60 (m, 2H), 3.59 (dd, J = 10.8, 7.8Hz, 0.8H), 3.44 (dd, J = 10.8, 6.9Hz, 0.2H), 3.28 (dd, J = 10.8, 6.9Hz, 0.8H), 3.20 (dd, J = 10.8, 7.8Hz, 0.2H), 2.40-2.10 (m, 4H).

### Example 2 (1) - Example 2 (46)

The compound of the present invention was obtained, using a corresponding compound, by the same procedure as Reference Example 2 →Example 3 →Example 2 (in some case, hydrogen chloride-ethyl acetate solution, hydrogen chloride-dioxane solution may be used instead of trifluoroacetic acid).

### Example 2 (1)

### (2S)-1-{[(4R)-2-isobutyl-1,3-thiazolidin-4-yl]carbonyl}pyrrolidine-2-carbonitrile trifluoroacetate

### Example 2 (2)

### (2S)-1-{[(4R)-2-(2-phenylethyl-1,3-thiazolidin-4-yl]carbonyl}pyrrolidine-2-carbonitrile

### Example 2 (3)

### (2S)-1-{[(4R)-2-pyridine-3-yl-1,3-thiazolidin-4-yl]carbonyl}pyrrolidine-2-carbonitrile trifluoroacetate

### Example 2 (4)

### (2S)-1-{[(4R)-2-isopropyl-1,3-thiazolidin-4-yl]carbonyl}pyrrolidine-2-carbonitrile trifluoroacetate

### Example 2 (5)

### (2S)-1-{[(4R)-2-cyclohexyl-1,3-thiazolidin-4-yl]carbonyl}pyrrolidine-2-carbonitrile trifluoroacetate

### Example 2 (6)

### (2S)-1-{[(4R)-2-(1-ethylpropyl)-1,3-thiazolidin-4-yl]carbonyl}pyrrolidine-2-carbonitrile trifluoroacetate

### Example 2 (7)

### (2S)-1-{[(4R)-2-butyl-1,3-thiazolidin-4-yl]carbonyl}pyrrolidine-2-carbonitrile trifluoroacetate

### Example 2 (8)

### (2S)-1-{[(4R)-2-benzyl-1,3-thiazolidin-4-yl]carbonyl}pyrrolidine-2-carbonitrile trifluoroacetate

### Example 2 (9)

### (2S)-1-{[(4R)-2-ethyl-1,3-thiazolidin-4-yl]carbonyl}pyrrolidine-2-carbonitrile trifluoroacetate

### Example 2 (10)

### (2S)-1-{[(4R)-2-(1-adamanthyl)-1,3-thiazolidin-4-yl]carbonyl}pyrrolidine-2-carbonitrile

### Example 2 (11)

### (2S)-1-{[(4R)-2-pyridin-2-yl-1,3-thiazolidin-4-yl]carbonyl}pyrrolidine-2-carbonitrile hydrochloride

### Example 2 (12)

### (2S)-1-{[(4R)-2-pyridin-4-yl-1,3-thiazolidin-4-yl]carbonyl}pyrrolidine-2-carbonitrile dihydrochloride

### Example 2 (13)

### (2S)-1-{[(4R)-2-(1,3-thiazol-2-yl)-1,3-thiazolidin-4-yl]carbonyl}pyrrolidine-2-carbonitrile dihydrochloride

### Example 2 (14):

### (2S)-1-{(4R)-2-([2,3-dimethoxyphenyl)-1,3-thiazolidin-4-yl]carbonyl}pyrrolidine-2-carbonitrile

### hydrochloride

### Example 2 (15)

### (2S)-1-{(4R)-2-(1,3-benzodioxol-5-yl)-[1,3-thiazolidin-4-yl]carbonyl}pyrrolidine-2-carbonitrile

### hydrochloride

### Example 2 (16)

### (2S)-1-{[(4R)-2-(phenoxymethyl)-1,3-thiazolidin-4-yl]carbonyl}pyrrolidine-2-carbonitrile hydrochloride

### Example 2 (17)

### (2S)-1-{[(4R,5R)-5-methyl-2-phenyl-1,3-thiazolidin-4-yl]carbonyl}pyrrolidine-2-carbonitrile

### hydrochloride

### Example 2 (18)

### (2S)-1-{[(4R)-2-(1,3-benzodioxol-4-yl)-1,3-thiazolidin-4-yl]carbonyl}pyrrolidine-2-carbonitrile

### hydrochloride

### Example 2 (19)

### (2S)-1-{[(4R)-2-(2,5-dimethoxyphenyl)-1,3-thiazolidin-4-yl]carbonyl}pyrrolidine-2-carbonitrile

### hydrochloride

### Example 2 (20)

### (2S)-1-({(4R)-2-[(phenylthio)methyl]-1,3-thiazolidin-4-yl}carbonyl)pyrrolidine-2-carbonitrile

### hydrochloride)

### Example 2 (21)

### (2S)-1-({(4R)-2-[2-(phenylthio)ethyl]-1,3-thiazolidin-4-yl}carbonyl)pyrrolidine-2-carbonitrile

### hydrochloride

### Example 2 (22)

### (2S)-1-{[(4R)-2-(2,6-dimethoxyphenyl)-1,3-thiazolidin-4-yl]carbonyl}pyrrolidine-2-carbonitrile

### hydrochloride

### Example 2 (23)

### (2S)-1-{(4R)-2-(3,4-dimethoxyphenyl)-[1,3-thiazolidin-4-yl]carbonyl}pyrrolidine-2-carbonitrile

### hydrochloride

### Example 2 (24)

### (2S)-1-{(4R)-2-([3,5-dimethoxyphenyl)-1,3-thiazolidin-4-yl]carbonyl}pyrrolidine-2-carbonitrile

### hydrochloride

### Example 2 (25)

### (2S)-1-{[(4R)-2-(2,4-dimethoxyphenyl)-1,3-thiazolidin-4-yl]carbonyl}pyrrolidine-2-carbanitrile

### hydrochloride

### Example 2 (26)

### (2S)-1-{[(4R)-2-(4-phenoxyphenyl)-1,3-thiazolidin-4-yl]carbonyl}pyrrolidine-2-carbonitrile hydrochloride

### Example 2 (27)

### (2S)-1-{[(4S)-2-phenyl-1,3-thiazinan-4-yl]carbonyl}pyrrolidine-2-carbonitrile hydrochloride

### Example 2 (28)

### (2S)-1-{[(4R)-2-(2-methoxyphenyl)-1,3-thiazolidin-4-yl]carbonyl}pyrrolidine-2-carbonitrile hydrochloride

### Example 2 (29)

### (2S)-1-{[(4R)-2-(3-methoxyphenyl)-1,3-thiazolidin-4-yl]carbonyl}pyrrolidine-2-carbonitrile hydrochloride

### Example 2 (30)

### (2S)-1-{[(4R)-2-(4-methoxyphenyl)-1,3-thiazolidin-4-yl]carbonyl}pyrrolidine-2-carbonitrile hydrochloride

### Example 2 (31)

### (2S)-1-{[(4R)-2-(3-phenoxyphenyl)-1,3-thiazolidin-4-yl]carbonyl}pyrrolidine-2-carbonitrile hydrochloride

### Example 2 (32)

### (2S)-1-{[(4R)-2-(2,3,4-trimethoxyphenyl)-1,3-thiazolidin-4-yl]carbonyl}pyrrolidine-2-carbonitrile

### hydrochloride

### Example 2 (33)

### (2S)-1-{[(4R)-2-(2,4,5-trimethoxyphenyl)-1,3-thiazolidin-4-yl]carbonyl}pyrrolidine-2-carbonitrile

### hydrochloride

### Example 2 (34)

### (2S)-1-{[(4R)-2-(2-ethoxyphenyl)-1,3-thiazolidin-4-yl]carbonyl}pyrrolidine-2-carbonitrile hydrochloride

### Example 2 (35)

### (2S)-1-{[(4R)-2-(3-ethoxyphenyl)-1,3-thiazolidin-4-yl]carbonyl}pyrrolidine-2-carbonitrile hydrochloride

### Example 2 (36)

### (2S)-1-{[(4R)-2-(4-ethoxyphenyl)-1,3-thiazolidin-4-yl]carbonyl}pyrrolidine-2-carbonitrile hydrochloride

### Example 2 (37)

### (2S)-1-{[(4R)-2-(3,4,5-trimethoxyphenyl)-1,3-thiazolidin-4-yl]carbonyl}pyrrolidine-2-carbonitrile

### hydrochloride

### Example 2 (38)

### (2S)-1-{[(4R)-2-(4-propoxyphenyl)-1,3-thiazolidin-4-yl]carbonyl}pyrrolidine-2-carbonitrile hydrochloride

### Example 2 (39)

### (2S)-1-{[(4R)-2-(3-cyanophenyl)-1,3-thiazolidin-4-yl]carbonyl}pyrrolidine-2-carbonitrile hydrochloride

### Example 2 (40)

### (2S)-1-{[(4R)-2-(4-cyanophenyl)-1,3-thiazolidin-4-yl]carbonyl}pyrrolidine-2-carbonitrile hydrochloride

### Example 2 (41)

### (2S)-1-{[(4R)-2-(4-pyridin-2-ylphenyl)-1,3-thiazolidin-4-yl]carbonyl}pyrrolidine-2-carbonitrile

### dihydrochloride

### Example 2 (42)

### 3-((4R)-4-{[(2S)-2-cyanopyrrolidin-1-yl]carbonyl}-1,3-thiazolidin-2-yl)benzoic acid hydrochloride

### Example 2 (43)

### 4-((4R)-4-{[(2S)-2-cyanopyrrolidin-1-yl]carbonyl}-1,3-thiazolidin-2-yl)benzoic acid hydrochloride

### Example 2 (44)

### 3-((4R)-4-{[(2S)-2-cyanopyrrolidin-1-yl]carbonyl}-1,3-thiazolidin-2-yl)-N,N-dimethylbenzamide

### hydrochloride

### Example 2 (45)

### 4-((4R)-4-{[(2S)-2-cyanopyrrolidin-1-yl]carbonyl}-1,3-thiazolidin-2-yl)-N,N-dimethylbenzamide

### hydrochloride

### Example 2 (46)

### (2S)-1-[(4R)-spiro[1,3-thiazolidin-2,2'-tricyclo[3.3.1.1^{3,7}]decan]-4-ylcarbonyl]pyrrolidine-2-carbonitrile

### hydrochloride

### Example 3

### (2S)-1-{[(5R)-5-phenylpyrrolidin-2-yl]carbonyl}pyrrolidine-2-carbonitrile hydrochloride

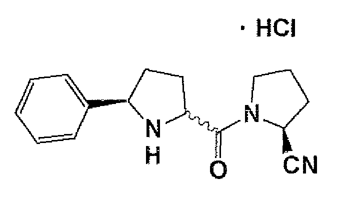

The compound of the present invention was obtained, using the compound prepared in Reference Example 9, by the same procedure as Reference Example 1 →Example 1.

### More polar compound

TLC: Rf 0.57 (chloroform:methanol=5:1);
NMR (CDCl₃): δ 1.80-2.80 (m, 8H), 3.50-3.80 (m, 2H), 4.60-5.00 (m, 3H), 6.93 (s, 1 H), 7.46 (m, 3H) 7.61 (m, 2H).
Less polar compound
TLC: Rf 0.59 (chloroform:methanol=5:1);
NMR (CDCl₃): δ 2.00-2.60 (m, 8H), 3.30-4.00 (m, 2H), 4.70-5.20 (m, 3H), 6.80 (s, 1H), 7.42 (m, 3H) 7.58 (m, 2H).

### Example 3 (1) - Example 3 (5)

The following compounds of the present invention were obtained, by the same procedure as Reference Example 4 →Reference Example 5 →Reference Example 6 →Reference Example 7 →Reference Example 8 →Reference Example 9 →Example 3.

### Example 3 (1)

### (2S)-1-(1-azaspiro[4.4]nona-2-ylcarbonyl)pyrrolidine-2-carbonitrile hydrochloride

### Example 3 (2)

### (2S)-1-{[(5S)-5-phenylpyrrolidin-2-yl]carbonyl}pyrrolidine-2-carbonitrile hydrochloride

### Example 3 (3)

### (2S)-1-{[(2S)-5-vinylpyrrolidin-2-yl]carbonyl}pyrrolidine-2-carbonitrile hydrochloride

### Example 3 (4)

### (2S)-1-{[(2S)-5-ethylpyrrolidin-2-yl]carbonyl}pyrrolidine-2-carbonitrile hydrochloride

### Example 3 (5)

### (2S)-1-(1-azaspiro[4.5]deca-2-ylcarbonyl)pyrrolidine-2-carbonitrile hydrochloride

### Example 4

### (2S)-1-[(3R)-1-thia-4-azaspiro[4.4]nona-3-ylcarbonyl]pyrrolidine-2-carbonitrile hydrochloride

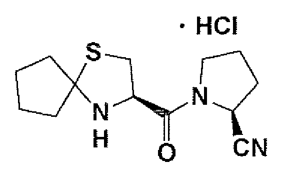

The compound of the present invention was obtained, using the compound of Reference Example 11, by the same procedure as Reference Example 1 (if necessary converting into corresponding salt by known methods.).
TLC: Rf 0.20 (toluene: ethyl acetate =1:1);
NMR (DMSO-d₆): δ 4.81 (dd, J = 8.1, 4.8Hz, 1H), 4.75 (dd, J = 9.0, 8.1 Hz, 1 H), 3.81 (ddd, J = 9.6, 6.9, 6.9Hz, 1H), 3.70 (dd, J = 11.4, 8.1Hz, 1H), 3.63 (ddd, J = 9.6, 6.9, 6.9Hz, 1H), 3.18 (dd, J = 11.4, 9.0Hz, 1 H), 2.45-1.95 (m, 8H), 1.87-1.55 (m, 4H).

### Example 4 (1) -Example 4 (14)

The following compounds of the present invention were obtained, by the same procedure as Reference Example 10 →Reference Example 11 →Example 4.

### Example 4 (1)

### (2S)-1-[(7R)-5-thia-8-azaspiro[3.4]octa-7-ylcarbonyl]pyrrolidine-2-carbonitrile hydrochloride

### Example 4 (2)

### (2S)-1-{[(3R)-8-(phenylsulfonyl)-1-thia-4,8-diazaspiro[4.5]deca-3-yl]carbonyl}pyrrolidine-2-carbonitrile

### Example 4 (3)

### (2S)-1-{[(3R)-8-phenyl-1-thia-4,8-diazaspiro[4.5]deca-3-yl]carbonyl}pyrrolidine-2-carbonitrile

### Example 4 (4)

### (2S)-1-{[(3R)-8-benzoyl-1-thia-4,8-diazaspiro[4.5]deca-3-yl]carbonyl}pyrrolidine-2-carbonitrile

### Example 4 (5)

### (2S)-1-[(4'R)-spiro[fluorene-9,2'-[1,3]thiazolidin]-4'-ylcarbonyl]pyrrolidine-2-carbonitrile hydrochloride]

### Example 4 (6)

### (2S)-1-{[(3R)-8-acetyl-1-thia-4,8-diazaspiro[4.5]deca-3-yl]carbonyl}pyrrolidine-2-carbonitrile

### Example 4 (7)

### (2S)-1-{[(3R)-8-(methylsulfonyl)-1-thia-4,8-diazaspiro[4.5]deca-3-yl]carbonyl}pyrrolidine-2-carbonitrile

### Example 4 (8)

### (2S)-1-[(11R)-1,4-dioxan-9-thia-12-azadispiro[4.2.4.2]tetradeca-11-ylcarbonyl]pyrrolidine-2-carbonitrile

### Example 4 (9)

### (2S)-1-{[(3R)-8-phenyl-1-thia-4-azaspiro[4.5]deca-3-yl]carbonyl}pyrrolidine-2-carbonitrile hydrochloride

### Example 4 (10)

### (2S)-1-{[(3R)-8,8-dimethyl-1-thia-4-azaspiro[4.5]deca-3-yl]carbonyl}pyrrolidine-2-carbonitrile

### hydrochloride

### Example 4 (11)

### (2S)-1-[(3R)-1-thia-4-azaspiro[4.5]deca-3-ylcarbonyl]pyrrolidine-2-carbonitrile trifluoroacetate

### Example 4 (12)

### (3R)-3-{[(2S)-2-cyanopyrrolidin-1-yl]carbonyl}-1-thia-4,8-diazaspiro[4.5]decane-8-carboxylic acid t-butyl ester

### Example 4 (13)

### (2S)-1-{[(4R)-2,2-diphenyl-1,3-thiazolidin-4-yl]carbonyl}pyrrolidine-2-carbonitrile hyd rochloride

### Example 4 (14)

### (2S)-1-{[(4R)-2,2-dimethyl-1,3-thiazolidin-4-yl]carbonyl}pyrrolidine-2-carbonitrile hydrochloride

### Example 5

### (2S)-1-(1,3-dihydrospiro[indene-2,3'-pyrrolidine]-5'-ylcarbonyl)pyrrolidine-2-carbonitrile hydrochloride

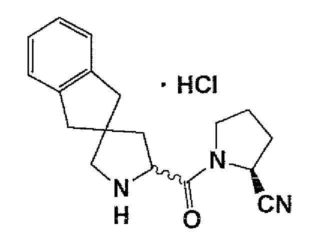

The compound of the present invention was obtained, using the compound of Reference Example 17, by the same procedure as Reference Example 1 →Example 1 and then being separated by column chromatography on silica gel.

### More polar compound

TLC: Rf 0.32 (dichloromethane:methanol=9:1);
NMR (DMSO-d₆): δ 1.97 (m, 4H), 2.18 (m, 2H), 2.99 (m, 4H), 3.25 (s, 2H), 3.35 (m, 1 H), 3.78 (m, 1H), 4.66 (s, 1H), 4.80 (m, 1H), 7.17 (m, 4H), 8.82 (s, 1H), 10.65 (s, 1H).
Less polar compound
TLC: Rf 0.43 (dichloromethane:methanol=9:1);
NMR (DMSO-d₆) : δ 1.97 (m, 4H), 2.18 (m, 2H), 2.99 (m, 4H), 3.25 (s, 2H), 3.35 (m, 1 H), 3.78 (m, 1H), 4.66 (s, 1 H), 4.80 (m, 1H), 7.17 (m, 4H), 8.82 (s, 1 H), 10.65 (s, 1H).

### Example 5 (1)

### (2S)-1-(2-azaspiro[4.4]nona-3-ylcarbonyl)pyrrolidine-2-carbonitrile hydrochloride

The compound of the present invention was obtained, by the same procedure as Reference Example 12 →Reference Example 13 →Reference Example 14 →Reference Example 15 →Reference Example 16 →Reference Example 17 →Reference Example 18 →Reference Example 19 →Example 5.
TLC: Rf 0.32, 0.23 (dichloromethane:methanol=9:1);
NMR (CDCl₃): δ 1.50-2.50 (m, 14H), 3.30-4.30 (m, 4H), 4.70-5.43 (m, 2H).

### Example 6

### (2S)-1-{[(2S)-4-benzylpyrrolidin-2-yl]carbonyl}pyrrolidine-2-carbonitrile hydrochloride

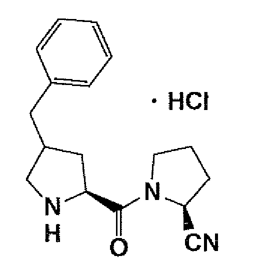

The compound of the present invention was obtained, using the compound of Reference Example 22, by the same procedure as Reference Example 1 →Example 1.
TLC: Rf 0.36 (chloroform:methanol=9:1);
NMR (DMSO-d₆): δ 1.60 (m, 1 H), 2.19 (m, 4H), 2.85 (m, 4H), 3.53 (m, 4H), 4.59 (m, 2H), 7.27 (m, 5H).

### Example 7

### (2S)-1-{[(2S,4E)-4-benzylidenepyrrolidin-2-yl]carbonyl}pyrrolidine-2-carbonitrile hydrochloride

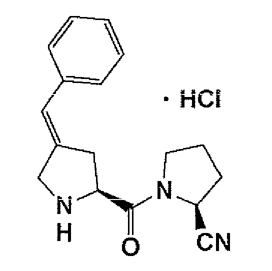

The compound of the present invention was obtained, using the compound of Reference Example 21, by the same procedure as Reference Example 1 →Example 1.
TLC: Rf 0.32 (chloroform:methanol=9:1);
NMR (DMSO-d₆): δ 2.15 (m, 4H), 2.78 (dd, J=17.31, 7.97Hz, 1H), 3.44 (m, 1H), 3.65 (m, 2H), 4.02 (m, 2H), 4.74 (t, J=8.24Hz, 1H), 4.84 (dd, J=7.83, 4.81 Hz, 1H), 6.61 (s, 1H), 7.34 (m, 5H).

### Example 8

### (2S)-1-({(2S,4R)-4-[4-(trifluoromethyl)phenyl)pyrrolidin-2-yl}carbonyl)pyrrolidine-2-carbonitrile hydrochloride

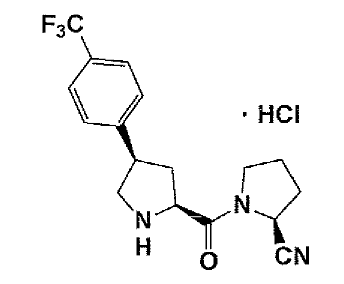

The compound of the present invention was obtained, using the compound of Reference Example 26, by the same procedure as Reference Example 1 →Example 1.
TLC: Rf 0.55 (chloroform:methanol=5:1);
NMR (DMSO-d₆): δ 1.80-2.30 (m, 5H), 3.00 (m, 1H), 3.26 (m, 1H), 3.67 (m, 4H), 4.63 (dd, J=10.16, 7.14Hz, 1H), 4.85 (dd, J=7.97, 4.94Hz, 1 H), 7.59 (d, J=8.52Hz, 2H), 7.72 (d, J=8.52Hz, 2H), 9.61 (m, 2H).

### Example 8 (1) - Example 8 (17)

The compound of the present invention was obtained, by the same procedure as Reference Example 23 →Reference Example 24 →Reference Example 25 →Reference Example 26 →Example 8.

### Example 8 (1)

### (2S)-1-{[(2S,4R)-4-phenylpyrrolidin-2-yl]carbonyl}pyrrolidine-2-carbonitrile hydrochloride

### Example 8 (2)

### (2S)-1-{[(2S,4R)-4-(4-methoxyphenyl)pyrrolidin-2-yl]carbonyl}pyrrolidine-2-carbonitrile hydrochloride

### Example 8 (3)

### (2S)-1-{[(2S,4S)-4-(2-furyl)pyrrolidin-2-yl]carbonyl}pyrrolidine-2-carbonitrile hydrochloride

### Example 8 (4)

### (2S)-1-{[(2S,4R)-4-(4-fluorophenyl)pyrrolidin-2-yl]carbonyl}pyrrolidine-2-carbonitrile hydrochloride

### Example 8 (5)

### (2S)-1-{[(2S,4R)-4-(4-methylphenyl)pyrrolidin-2-yl]carbonyl}pyrrolidine-2-carbonitrile hydrochloride

### Example 8 (6)

### (2S)-1-{[(2S,4R)-4-pyridin-4-ylpyrrolidin-2-yl]carbonyl}pyrrolidine-2-carbonitrile dihydrochloride

### Example 8 (7)

### (2S)-1-{[(2S,4R)-4-pyridin-3-ylpyrroildin-2-yl]carbonyl}pyrrolidine-2-carbonitrile dihydrochloride

### Example 8 (8)

### (2S)-1-{[(2S,4R)-4-(3-methylphenyl)pyrrolidin-2-yl]carbonyl}pyrrolidine-2-carbonitrile hydrochloride

### Example 8 (9)

### (2S)-1-{[(2S,4R)-4-(2-methylphenyl)pyrrolidin-2-yl]carbonyl}pyrrolidine-2-carbonitrile hydrochloride

### Example 8 (10)

### (2S)-1-{[(2S,4S)-4-(1-benzofuran-2-yl)pyrrolidin-2-yl]carbonyl}pyrrolidine-2-carbonitrile hydrochloride

### Example 8 (11)

### (2S)-1-{[(2S,4R)-4-(4-isopropylphenyl)pyrrolidin-2-yl]carbonyl}pyrrolidine-2-carbonitrile hydrochloride

### Example 8 (12)

### (2S)-1-{[(2S,4R)-4-(3-furyl)pyrrolidin-2-yl]carbonyl}pyrrolidine-2-carbonitrile hydrochloride

### Example 8 (13)

### (2S)-1-{[(2S,4R)-4-(4-cyanophenyl)pyrrolidin-2-yl]carbonyl}pyrrolidine-2-carbonitrile hydrochloride

### Example 8 (14)

### 3-{[(2S,4R)-4-phenylpyrrolidin-2-yl]carbonyl}-1,3-thiazolidine/hydrochloride

### Example 8 (15)

### (2S)-1-{[(2S,4R)-4-(2,6-dimethylphenyl)pyrrolidin-2-yl]carbonyl}pyrrolidine-2-carbonitrile hydrochloride

### Example 8 (16)

### (2S)-1-{[(2S,4R)-4-(1-naphthyl)pyrrolidin-2-yl]carbonyl}pyrrolidine-2-carbonitrile hydrochloride

### Example 8 (17)

### (2S)-1-{[(2S,4R)-4-2-naphthyl)pyrrolidin-2-yl]carbonyl}pyrrolidine-2-carbonitrile hydrochloride

### Example 9

### (2S)-1-[(4-phenyl-2,5-dihydro-1H-pyrrol-2-yl)carbonyl]pyrrolidine-2-carbonitrile hydrochloride

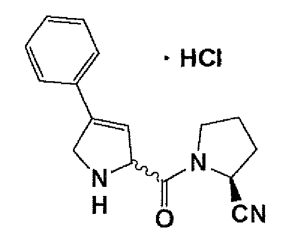

The compound of the present invention was obtained, using the compound of Reference Example 23, by the same procedure as Reference Example 24 →Reference Example 26 →Example 8.
TLC: Rf 0.44, 0.36 (chloroform:methanol=9:1);
NMR (DMSO-d₆): δ 2.19 (m, 4H), 3.82 (m, 2H), 4.50 (m, 2H), 4.83 (m, 1H), 5.44 (m, 1H), 6.56 (m, 1 H), 7.48 (m, 5H), 10.13 (m, 1H).

### Example 10

### (4R)-3-{[(4R)-2-phenyl-1,3-thiazolidin-4-yl]carbonyl}-1,3-thiazolidine-4-carbonitrile hydrochloride

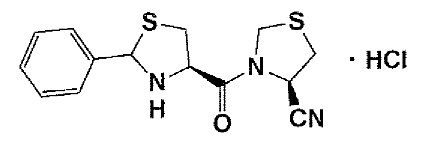

The compound of the present invention was obtained, using the compound of Reference Example 28, by the same procedure as Example 1.
TLC: Rf 0.60 (ethyl acetate: n-hexane =2:1);
NMR (DMSO-d₆): δ 7.63-7.52 (m, 2H), 7.45-7.30 (m, 3H), 5.84-5.58 (m, 1H), 5.34-5.24 (m, 1H), 4.92-4.44 (m, 3H), 4.04 (bs, 2H), 3.62-3.14 (m, 4H).

### Example 10 (1) - Example 10 (13)

The compound of the present invention was obtained, by the same procedure as Reference Example 27 →Reference Example 28 →Example 10.

### Example 10 (1)

### (4R)-3-{[(4R)-2-(2-phenylethyl)-1,3-thiazolidin-4-yl]carbonyl}-1,3-thiazolidine-4-carbonitrile hydrochloride

### Example 10 (2)

### (4R)-3-{[(4R)-2-isobutyl-1,3-thiazolidin-4-yl]carbonyl}-1,3-thiazolidine-4-carbanitrile hydrochloride

### Example 10 (3)

### (4R)-3-{[(4R)-2-(2-methylphenyl)-1,3-thiazolidin-4-yl]carbonyl}-1,3-thiazolidine-4-carbonitrile hydrochloride

### Example 10 (4)

### (4R)-3-{[(4R)-2-(2-Chlorophenyl)-1,3-thiazolidin-4-yl]carbonyl}-1,3-thiazolidine-4-carbonitrile hydrochloride

### Example 10 (5)

### (4R)-3-{[(4R)-2-(3-methylphenyl)-1,3-thiazolidin-4-yl]carbonyl}-1,3-thiazolidine -4-carbonitrile hydrochloride

### Example 10 (6)

### (4R)-3-{[(4R)-2-(4-methylphenyl)-1,3-thiazolidin-4-yl]carbonyl}-1,3-thiazolidine -4-carbonitrile hydrochloride

### Example 10 (7)

### (4R)-3-{[(4R)-2-(3-chlorophenyl)-1,3-thiazolidin-4-yl]carbonyl}-1,3-thiazolidine -4-carbonitrile hydrochloride

### Example 10 (8)

### (4R)-3-{[(4R)-2-(4-chlorophenyl)-1,3-thiazolidin-4-yl]carbonyl}-1,3-thiazolidine -4-carbonitrile hydrochloride

### Example 10 (9)

### (4R)-3-{(4R)-2-(2-methoxyphenyl)-[1,3-thiazolidin-4-yl]carbonyl}-1,3-thiazolidine-4-carbonitrile hydrochloride

### Example 10 (10)

### (4R)-3-{[(4R)-2-(3-methoxyphenyl)-1,3-thiazolidin-4-yl]carbonyl}-1,3-thiazolidine-4-carbonitrile hydrochloride

### Example 10 (11)

### (4R)-3-{[(4R)-2-(4-methoxyphenyl)-1,3-thiazolidin-4-yl]carbonyl}-1,3-thiazolidine-4-carbonitrile hydrochloride

### Example 10 (12)

### (4R)-3-{[(4R)-2-benzyl-1,3-thiazolidin-4-yl]carbonyl}-1,3-thiazolidine-4-carbonitrile hydrochloride

### Example 10 (13)

### (4R)-3-{[(4R)-2-(3-phenylpropyl)-1,3-thiazolidin-4-yl]carbonyl}-1,3-thiazolidine-4-carbonitrile hydrochloride

### Example 11

### (2S)-1-{[(2S,4S)-4-allylpyrrolidin-2-yl]carbonyl}pyrrolidine-2-carbonitrile hydrochloride

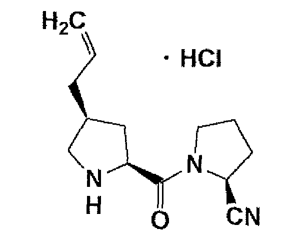

The compound of the present invention was obtained, using the compound of Reference Example 29, by the same procedure as Reference Example 11 →Reference Example 1 →Example 1.
TLC: Rf 0.40 (dichloromethane:methanol=9:1);
NMR (DMSO-d₆): δ 1.44 (m, 1H), 1.90-2.50 (m, 8H), 2.60 (m, 1H), 2.87 (t, J=10.03Hz, 1 H), 3.57 (m, 2H), 4.45 (t, J=8.52Hz, 1 H), 4.82 (dd, J=7.97, 4.67Hz, 1 H), 5.05 (m, 2H), 5.77 (m, 1H), 8.69 (s, 1 H), 10.37 (s, 1H).

### Example 11 (1):

### (2S)-1-{[(2S,4S)-4-propylpyrrolidin-2-yl]carbonyl}pyrrolidine-2-carbonitrile hydrochloride

The compound of the present invention was obtained, using the compound of Reference Example 29, by the same procedure as Reference Example 5 →Example 11.
TLC: Rf 0.34 (dichloromethane:methanol=9:1);
NMR (DMSO-d₆): δ 0.86 (t, J = 7.00 Hz, 3H), 1.20-1.40 (m, 5H), 1.90-2.30 (m, 5H), 3.59 (m, 2H), 4.43 (m, 1 H), 4.82 (dd, J = 7.83, 4.81 Hz, 1H), 8.66 (s, 1H), 10.38 (s, 1H).

### Example 12

### (2S)-1-{[4-(5-methyl-1,3,4-oxadiazol-2-yl)-1-pyrrolidin-2-yl]carbonyl}pyrrolidine-2-carbonitrile hydrochloride

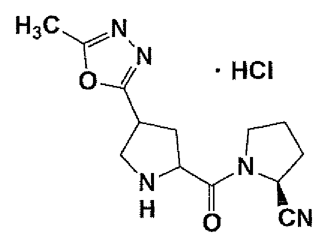

The compound of the present invention was obtained, using the compound of Reference Example 32, by the same procedure as Reference Example 11 →Reference Example 1 →Example 1.
TLC: Rf 0.42 (dichloromethane:methanol=9:1);
NMR (DMSO-d₆): δ 2.13 (m, 5H), 2.46 (s, 3H), 2.93 (m, 1 H), 3.43 (m, 2H), 3.86 (m, 3H), 4.70 (s, 1H), 4.77 (dd, J=6.59, 4.12 Hz, 1H), 9.15 (s, 1H), 10.49 (s, 1H).

### Example 13

### (2S)-1-{[4-(2-methyl-1,3-thiazole-4-yl)-1-pyrrolidin-2-yl]carbonyl}pyrrolidine-2-carbonitrile dihydrochloride

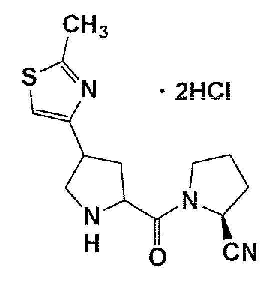

The compound of the present invention was obtained, using the compound of Reference Example 34, by the same procedure as Reference Example 11 →Reference Example 1 →Example 1. TLC: Rf 0.45 (dichloromethane:methanol: ethyl acetate =17:2:1);
NMR (DMSO-d₆): δ 2.03 (m, 3H), 2.21 (m, 2H), 2.62 and 2.63 (s, 3H), 2.86 (m, 1H), 3.27 (m, 1H), 3.42 (m, 1 H), 3.69 (m, 3H), 4.63 (m, 1 H), 4.81 (m, 1H), 7.39 and 7.41 (s, 1 H), 8.91 (s, 1H), 10.64 and 10.82 (s, 1 H).

### Example 14

### 2-((3R,5S)-5-{[(2S)-2-cyanopyrrolidin-1-yl]carbonyl}pyrrolidin-3-yl)benzoic acid 4-methylbenzene sulfonate

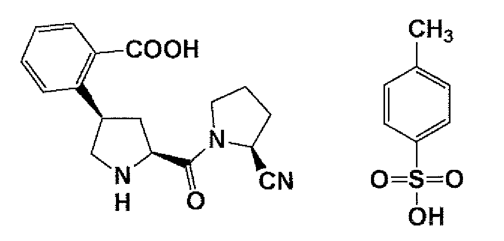

To a solution of the compound prepared on Reference Example 36 (198 mg) in 1,4-dioxane (2.4ml), *p*-toluenesulfonic acid (99.6 mg) was added. The mixture was stirred for 10 hours at 80 °C. The reaction mixture was concentrated. The residue was washed with diethyl ether to give the title compound (161 mg).
TLC: Rf 0.29 (chloroform:methanol: acetic acid:water=80:20:3:3);
NMR (DMSO-d₆): δ 1.99 (m, 3H), 2.18 (m, 2H), 2.28 (s, 3H), 2.89 (m, 1H), 3.23 (m, 1H), 3.54 (m, 3H), 4.37 (m, 1H), 4.67 (m, 1H), 4.86 (dd, J=8.06, 5.13 Hz, 1H), 7.10 (d, J=8.06 Hz, 2H), 7.39 (m, 1 H), 7.46 (d, J=8.06 Hz, 2H), 7.56 (m, 2H), 7.80 (d, J=7.32 Hz, 1H), 8.98 (s, 1H), 9.57 (s, 1H), 13.16 (s, 1H).

### Example 14 (1) - Example 14 (3)

The following compounds were obtained, by the same procedure as Reference Example 14.

### Example 14 (1)

### 5-((3R,5S)-5-{[(2S)-2-cyanopyrrolidin-1-yl]carbonyl}pyrrolidin-3-yl)-2-hydroxybenzoic acid 4-methylbenzene sulfonate

### Example 14 (2)

### 3-((3R,5S)-5-{[(2S)-2-cyanopyrrolidin-1-yl]carbonyl}pyrrolidin-3-yl)benzoic acid 4-methylbenzene sulfonate

### Example 14 (3)

### 4-((3R,5S)-5-{[(2S)-2-cyanopyrrolidin-1-yl]carbonyl}pyrrolidin-3-yl)acetic acid 4-methylbenzene sulfonate

### Example 15

### ((3R,5S)-5-{[(2S)-2-cyanopyrrolidin-1-yl]carbonyl}pyrrolidin-3-yl)acetic acid 4-methylbenzene sulfonate

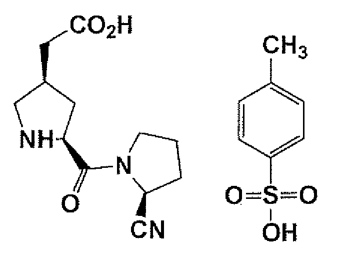

The title compound was obtained, using the compound prepared in Reference Example 37, by the same procedure as Example 14.
TLC: Rf 0.17 (chloroform:methanol:acetic acid=5:1:1);
NMR (DMSO-d₆): δ 1.45 (m, 1 H), 2.13 (m, 4H), 2.28 (s, 3H), 2.55 (m, 4H), 2.94 (m, 1 H), 3.40 (m, 1H), 3.55 (m, 2H), 4.48 (m, 1H), 4.82 (dd, J=7.78, 4.85 Hz, 1 H), 7.10 (d, J=7.87 Hz, 2H), 7.46 (m, 2H), 8.70 (s, 1H), 9.34 (s, 1 H) .

### Example 16

### (2S)-1-{[(2S,4R)-4-(2-morpholin-4-yl-2-oxoethyl)pyrrolidin-2-yl]carbonyl}pyrrolidine-2-carbonitrile 4-methylbenzene sulfonate

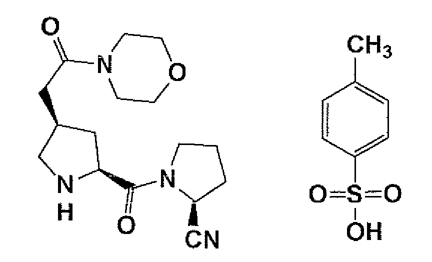

The title compound was obtained, using the compound prepared in Example 15, by the same procedure as Reference Example 3 (protective reaction of Boc) →Reference Example 1 with morpholine (amidation) →Example 14.
TLC: Rf 0.21 (ethyl acetate: acetic acid:water=3:1:1);
NMR (DMSO-d₆) : δ 1.46 (m, 1H), 2.08 (m, 5H), 2.28 (s, 3H), 2.56 (m, 1H), 2.65 (m, 2 H), 2.90 (m, 1H), 3.45 (m, 11H), 4.48 (m, 1H), 4.82 (dd, J = 7.78, 4.85 Hz, 1H), 7.10 (d, J = 8.06 Hz, 2H), 7.46 (d, J = 8.06 Hz, 2H), 8.67 (m, 1H), 9.32 (m, 1H).

### Example 16 (1) ― Example 16 (10)

The following compounds were obtained, by the same procedure as Example16 (with the proviso that, Example 16 was prepared using the compound in Reference Example 31 as a material).

### Example 16 (1)

### 5-{[(2S)-2-cyanopyrrolidin-1-yl]carbonyl}-N-phenylpyrrolidine-3-carboxamido hydrochloride

### Example 16 (2)

### 2-((3R,5S)-5-{[(2S)-2-cyanopyrrolidin-1-yl]carbonyl}pyrrolidin-3-yl)acetamide 4-methylbenzene sulfonate

### Example 16 (3)

### 2-((3R,5S)-5-{[(2S)-2-cyanopyrrolidin-1-yl]carbonyl}pyrrolidin-3-yl)-N-(methylsulfonyl)acetamide 4-methylbenzene sulfonate

### Example 16 (4)

### 2-((3R,5S)-5-{[(2S)-2-cyanopyrrolidin-1-yl]carbonyl}pyrrolidin-3-yl)-N-methylacetamide 4-methylbenzene sulfonate

### Example 16 (5)

### 2-((3R,5S)-5-{[(2S)-2-cyanopyrrolidin-1-yl]carbonyl}pyrrolidin-3-yl)-N,N-bis(2-methoxyethyl)acetamide 4-methylbenzene sulfonate

### Example 16 (6)

### 2-((3R,5S)-5-{[(2S)-2-cyanopyrrolidin-1-yl]carbonyl}pyrrolidin-3-yl)-N-(4-hydroxybutyl)acetamide 4-methylbenzene sulfonate

### Example 16 (7)

### (2S)-1-({(2S,4R)-4-[2-(4-hydroxypiperidin-1-yl)-2-oxoethyl)pyrrolidin-2-yl}carbonyl)pyrrolidine-2-carbonitrile 4-methylbenzene sulfonate

### Example 16 (8)

### 2-((3R,5S)-5-{[(2S)-2-cyanopyrrolidin-1-yl]carbonyl}pyrrolidin-3-yl)-N,N-dimethylacetamide 4-methylbenzene sulfonate

### Example 16 (9)

### 2-((3R,5S)-5-{[(2S)-2-cyanopyrrolidin-1-yl]carbonyl}pyrrolidin-3-yl)-N-(phenylsulfonyl)acetamide 4-methylbenzene sulfonate

### Example 16 (10)

### 2-((3R,5S)-5-{[(2S)-2-cyanopyrrolidin-1-yl]carbonyl}pyrrolidin-3-yl)-N-[(1r,5R,7S)-3-hydroxy-1-adamanthyl]acetamide 4-methylbenzene sulfonate

### Example 17

### {[((3R,5S)-5-{[(2S)-2-cyanopyrrolidin-1-yl]carbonyl}pyrrolidin-3-yl)acetyl]amino}acetic acid 4-methylbenzene sulfonate

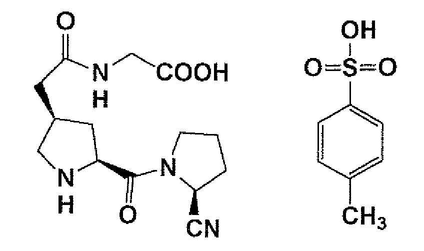

The title compound was obtained, using the compound prepared in Example 15, by the same procedure as Reference Example 3 →Reference Example 1 (glycine benzyl ester was used.) →catalytic reduction (it was carried out, by the same procedure as Reference Example 5, using hydroxylation palladium in ethyl acetate.) →Example 14.
TLC: Rf 0.19 (ethyl acetate: acetic acid:water=3:1:1);
NMR (DMSO-d₆): δ 1.44 (m, 1H), 2.13 (m, 5H), 2.28 (s, 3H), 2.34 (m, 1 H), 2.65 (m, 2H), 2.94 (m, 1H), 3.39 (m, 1 H), 3.55 (m, 2H), 3.73 (d, J=5.95 Hz, 2H), 4.46 (m, 1H), 4.82 (dd, J=7.87, 4.76 Hz, 1H), 7.10 (d, J=8.24 Hz, 2H), 7.46 (d, J=8.24 Hz, 2H), 8.28 (t, J=5.95 Hz, 1 H), 8.69 (s, 1 H), 9.30 (s, 1H).

### Example 17 (1) - Example 17 (2)

The following compounds were obtained, by the same procedure as Example17.

### Example 17 (1)

### [[((3R,5S)-5-{[(2S)-2-cyanopyrrolidin-1-yl]carbonyl}pyrrolidin-3-yl)acetyl](methyl)amino]acetic acid 4-methylbenzene sulfonate

### Example 17 (2)

### 3-{[((3R,5S)-5-{[(2S)-2-cyanopyrrolidin-1-yl]carbonyl}pyrrolidin-3-yl)acetyl]amino}propanoate 4-methylbenzene sulfonate

### Example 18

### (2S)-1-[(4R)-4-(cyanomethyl)-L-prolyl]pyrrolidine-2-carbonitrile trifluoroacetate

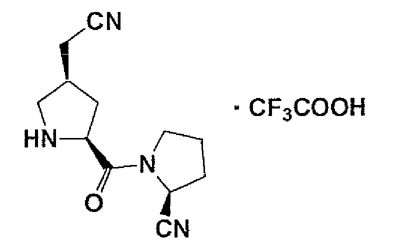

The compound prepared in Example 16 (2) was protected by Boc, by the same procedure as Reference Example 3. The obtained compound (250 mg) was dissolved into THF (2 ml). Pyridine (0.18ml) and trifluoroacetic anhydride (0.12ml) were added to the reaction solution at 0 °C and the mixture was stirred for 2 hours at 0 °C. Water was added to the reaction mixture. The mixture was extracted with ethyl acetate, washed with 0.1N hydrochloric acid, water and a saturated solution of sodium chloride successively, dried and concentrated. The residue was washed with ether-ethyl acetate (5/1) to give the title compound protected by Boc (156 mg).

The title compound was obtained using the obtained Boc protected compound, by the same procedure as Example 14.
TLC: Rf 0.35 (ethyl acetate: acetic acid: aqua =3:1:1);
NMR DMSO-d₆): δ 1.56 (m, 1 H) 2.03 (m, 2 H) 2.20 (m, 3 H) 2.64 (m, 3 H) 2.99 (m, 1 H) 3.57 (m, 3 H) 4.50 (m, 1 H) 4.84 (dd, J=7.81, 4.88 Hz, 1 H) 8.83 (s, 1 H) 9.46 (s, 1 H).

### Example 18 (1):

### (2S)-1-[(4R)-4-(4-cyano-2,6-dimethylphenyl)-L-prolyl]pyrrolidine-2-carbonitrile 4-methylbenzene sulfonate

The title compound was obtained, by the same procedure as Example18.

### Example 19 (1) - Example 19 (64)

The following compounds were obtained, by the same procedure as Reference Example 24 →Reference Example 5 →Reference Example 11 →Reference Example 1 →Example 1. In addition, deprotection reaction of protecting group was carried out if necessary.

### Example 19 (1)

### (2S)-1-{[(2S,4R)-4-(2,6-dimethoxyphenyl)pyrrolidin-2-yl]carbonyl}pyrrolidine-2-carbonitrile hydrochloride

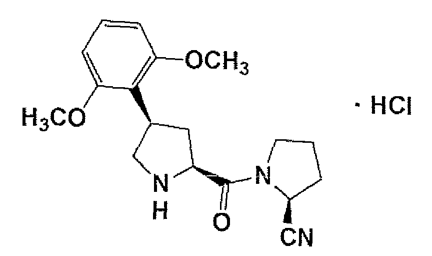

TLC: Rf 0.44 (chloroform:methanol=9:1);
NMR (DMSO-d₆): δ 2.01 (m, 2H), 2.19 (m, 3H), 2.61 (m, 1 H), 3.34 (m, 2H), 3.56 (m, 2H), 3.76 (s, 6H), 4.13 (m, 1H), 4.61 (t, J=8.65 Hz, 1H), 4.85 (m, 1H), 6.66 (d, J=8.24 Hz, 2H), 7.23 (t, J=8.38 Hz, 1H).

### Example 19 (2)

### (2S)-1-{[(2S,4R)-4-mesitylpyrrolidin-2-yl]carbonyl}pyrrolidine-2-carbonitrile hydrochloride

### Example 19 (3)

### (2S)-1-{[(2S,4R)-4-(3-methoxyphenyl)pyrrolidin-2-yl]carbonyl}pyrrolidine-2-carbonitrile hydrochloride

### Example 19 (4)

### (2S)-1-{[(2S,4R)-4-(2-methoxyphenyl)pyrrolidin-2-yl]carbonyl}pyrrolidine-2-carbonitrile hydrochloride

### Example 19 (5)

### (2S)-1-{[(2S,4R)-4-biphenyl-3-ylpyrrolidin-2-yl]carbonyl}pyrrolidine-2-carbonitrile hydrochloride

### Example 19 (6)

### (2S)-1-{[(2S,4R)-4-(2,5-dimethoxyphenyl)pyrrolidin-2-yl]carbonyl}pyrrolidine-2-carbonitrile hydrochloride

### Example 19 (7)

### (2S)-1-{[(2S,4R)-4-biphenyl-4-ylpyrrolidin-2-yl]carbonyl}pyrrolidine-2-carbonitrile hydrochloride

### Example 19 (8)

### (2S)-1-{[(2S,4R)-4-(3,4-dimethylphenyl)pyrrolidin-2-yl]carbonyl}pyrrolidine-2-carbonitrile hydrochloride

### Example 19 (9)

### (2S)-1-{[(2S,4R)-4-(2,5-dimethylphenyl)pyrrolidin-2-yl]carbonyl}pyrrolidine-2-carbonitrile hydrochloride

### Example 19 (10)

### (2S)-1-{[(2S,4R)-4-(2,4-dimethoxyphenyl)pyrrolidin-2-yl]carbonyl}pyrrolidine-2-carbonitrile hydrochloride

### Example 19 (11)

### (2S)-1-{[(2S,4R)-4-(3,4-dimethoxyphenyl)pyrrolidin-2-yl]carbonyl}pyrrolidine-2-carbonitrile hydrochloride

### Example 19 (12)

### (2S)-1-{[(2S,4R)-4-biphenyl-2-ylpyrrolidin-2-yl]carbonyl}pyrrolidine-2-carbanitrile hydrochloride

### Example 19 (13)

### (2S)-1-{[(2S,4R)-4-(2-isopropylphenyl)pyrrolidin-2-yl]carbonyl}pyrrolidine-2-carbonitrile hydrochloride

### Example 19 (14)

### (2S)-1-{[(2S,4R)-4-(2,3,4-trimethoxyphenyl)pyrrolidin-2-yl]carbonyl}pyrrolidine-2-carbonitrile hydrochloride

### Example 19 (15)

### (2S)-1-{[(2S,4R)-4-phenethylpyrrolidin-2-yl]carbonyl}pyrrolidine-2-carbonitrile 4-methylbenzene sulfonic acid

### Example 19 (16)

### (2S)-1-{[(2S,4R)-4-(1,3-benzodioxol-5-yl)pyrrolidin-2-yl]carbonyl}pyrrolidine-2-carbonitrile 4-methylbenzene sulfonic acid

### Example 19 (17)

### N-[2-((3R,5S)-5-{[(2S)-2-cyanopyrrolidin-1-yl]carbonyl}pyrrolidin-3-yl)phenyl]methane sulfonamide 4-methylbenzene sulfonic acid

### Example 19 (18)

### 4-((3R,5S)-5-{[(2S)-2-cyanopyrrolidin-1-yl]carbonyl}pyrrolidin-3-yl)-N,N-dimethylbenzamide 4-methylbenzene sulfonic acid

### Example 19 (19)

### (2S)-1-{[(2S,4R)-4-(4-hydroxy-2,6-dimethylphenyl)pyrrolidin-2-yl]carbonyl}pyrrolidine-2-carbonitrile 4-methylbenzene sulfonate

### Example 19 (20)

### (2S)-1-{[(2S,4R)-4-(4-methoxy-2,6-dimethylphenyl)pyrrolidin-2-yl]carbonyl}pyrrolidine-2-carbonitrile 4-methylbenzene sulfonate

### Example 19 (21)

### (2S)-1-({(2S,4R)-4-[4-(benzyloxy)-2,6-dimethylphenyl)pyrrolidin-2-yl}carbonyl)pyrrolidine-2-carbonitrile 4-methylbenzene sulfonate

### Example 19 (22)

### 4-((3R,5S)-5-{[(2S)-2-cyanopyrrolidin-1-yl]carbonyl}pyrrolidin-3-yl)-3,5-dimethylphenylmethanesulfonate 4-methylbenzene sulfonate

### Example 19 (23)

### (2S)-1-({(2S,4R)-4-[2-(benzyloxy)phenyl)pyrrolidin-2-yl}carbanyl)pyrrolidine-2-carbonitrile 4-methylbenzene sulfonate

### Example 19 (24)

### (2S)-1-{[(2S,4R)-4--(2-hydroxyphenyl)pyrrolidin-2-yl]carbonyl}pyrrolidine-2-carbonitrile 4-methylbenzene sulfonate

### Example 19 (25)

### (2S)-1-({(2S,4R)-4-[2-hydroxy-5-(methylsulfonyl)phenyl)pyrrolidin-2-yl}carbonyl)pyrrolidine-2-carbonitrile 4-methylbenzene sulfonate

### Example 19 (26)

### 4-((3R,5S)-5-{[(2S)-2-cyanopyrrolidin-1-yl]carbonyl}pyrrolidin-3-yl)-N,3,5-trimethylbenzamide 4-methylbenzene sulfonate

### Example 19 (27)

### 4-((3R,5S)-5-{[(2S)-2-cyanopyrrolidin-1-yl]carbonyl}pyrrolidin-3-yl)-N,N,3,5-tetramethylbenzamide 4-methylbenzene sulfonate

### Example 19 (28)

### (2S)-1-{[(2S,4R)-4-(4-hydroxyphenyl)pyrrolidin-2-yl]carbonyl}pyrrolidine-2-carbonitrile 4-methylbenzene sulfonate

### Example 19 (29)

### (2S)-1-{[(2S,4R)-4-(3-hydroxyphenyl)pyrrolidin-2-yl]carbonyl}pyrrolidine-2-carbonitrile 4-methylbenzene sulfonate

### Example 19 (30)

### 4-((3R,5S)-5-{[(2S)-2-cyanopyrrolidin-1-yl]carbonyl}pyrrolidin-3-yl)-3,5-dimethylbenzamide 4-methylbenzene sulfonate

### Example 19 (31)

### (2S)-1-{[(2S,4R)-4-(2,6-difluorophenyl)pyrrolidin-2-yl]carbonyl}pyrrolidine-2-carbonitrile 4-methylbenzene sulfonate

### Example 19 (32)

### (2S)-1-{[(2S,4R)-4-(2-fluoro-6-methoxyphenyl)pyrrolidin-2-yl]carbonyl}pyrrolidine-2-carbonitrile 4-methylbenzene sulfonate

### Example 19 (33)

### (2S)-1-({(2S,4R)-4-[4-(hydroxymethyl)-2,6-dimethylphenyl)pyrrolidin-2-yl}carbonyl)pyrrolidine-2-carbonitrile 4-methylbenzene sulfonate

### Example 19 (34)

### (2S)-1-({(2S,4R)-4-[4-(methoxymethyl)-2,6-dimethylphenyl)pyrrolidin-2-yl}carbonyl)pyrrolidine-2-carbonitrile 4-methylbenzene sulfonate

### Example 19 (35)

### (2S)-1-{[(2S,4R)-4-(2-methoxy-4,6-dimethylphenyl)pyrrolidin-2-yl]carbonyl}pyrrolidine-2-carbonitrile 4-methylbenzene sulfonate

### Example 19 (36)

### (2S)-1-{[(2S,4R)-4-(4-hydroxy-2,3,6-trimethylphenyl)pyrrolidin-2-yl]carbonyl}pyrrolidine-2-carbonitrile 4-methylbenzene sulfonate

### Example 19 (37)

### (2S)-1-{[(2S,4R)-4-(2-hydroxy-4-methoxy-6-methylphenyl)pyrrolidin-2-yl]carbonyl}pyrrolidine-2-carbonitrile 4-methylbenzene sulfonate

### Example 19 (38)

### (2S)-1-{[(2S,4R)-4-(4-hydroxy-3-methoxyphenyl)pyrrolidin-2-yl]carbonyl}pyrrolidine-2-carbonitrile 4-methylbenzene sulfonate

### Example 19 (39)

### (2S)-1-{[(2S,4R)-4-(3-hydroxy-2,4,6-trimethylphenyl)pyrrolidin-2-yl]carbonyl}pyrrolidine-2-carbonitrile 4-methylbenzene sulfonate

### Example 19 (40)

### (2S)-1-{[(2S,4R)-4-(4-hydroxy-3,5-dimethylphenyl)pyrrolidin-2-yl]carbonyl}pyrrolidine-2-carbonitrile 4-methylbenzene sulfonate

### Example 19 (41)

### (2S)-1-{[(2S,4R)-4-(4-hydroxy-2-methoxy-6-methylphenyl)pyrrolidin-2-yl]carbonyl}pyrrolidine-2-carbonitrile 4-methylbenzene sulfonate

### Example 19 (42)

### (2S)-1-{[(2S,4R)-4-(2-ethyl-6-methylphenyl)pyrrolidin-2-yl]carbonyl}pyrrolidine-2-carbonitrile 4-methylbenzene sulfonate

### Example 19 (43)

### (2S)-1-{[(2S,4R)-4-(3-hydroxy-2,6-dimethylphenyl)pyrrolidin-2-yl]carbonyl}pyrrolidine-2-carbonitrile 4-methylbenzene sulfonate

### Example 19 (44)

### (2S)-1-({(2S,4R)-4-[4-(2-hydroxyethoxy)-2,6-dimethylphenyl)pyrrolidin-2-yl}carbonyl)pyrrolidine-2-carbonitrile 4-methylbenzene sulfonate

### Example 19 (45)

### (2S)-1-({(2S,4R)-4-[4-(2-methoxyethoxy)-2,6-dimethylphenyl)pyrrolidin-2-yl}carbonyl)pyrrolidine-2-carbonitrile 4-methylbenzene sulfonate

### Example 19 (46)

### (2S)-1-{[(2S,4R)-4-(4-hydroxy-2,6-dimethoxyphenyl)pyrrolidin-2-yl]carbonyl}pyrrolidine-2-carbonitrile 4-methylbenzene sulfonate

### Example 19 (47)

### (2S)-1-{[(2S,4R)-4-(4-ethoxy-2-hydroxy-6-methylphenyl)pyrrolidin-2-yl]carbonyl}pyrrolidine-2-carbonitrile 4-methylbenzene sulfonate

### Example 19 (48)

### (2S)-1-{[(2S,4R)-4-(2-ethyl-4-hydroxy-6-methylphenyl)pyrrolidin-2-yl]carbonyl}pyrrolidine-2-carbonitrile 4-methylbenzene sulfonate

### Example 19 (49)

### N-[4-((3R,5S)-5-{[(2S)-2-cyanopyrrolidine-1-yl]carbonyl}pyrrolidin-3-yl)phenyl]methane sulfonamide 4-methylbenzene sulfonate

### Example 19 (50)

### (2S)-1-({(2S,4R)-4-[4-(tetrahydro-2H-pyran-4-yloxy)phenyl)pyrrolidin-2-yl}carbonyl)pyrrolidine-2-carbonitrile 4-methylbenzene sulfonate

### Example 19 (51)

### (2S)-1-[((2S,4R)-4-{4-[3-hydroxy-2-(hydroxymethyl)propoxy]phenyl}pyrrolidin-2-yl]carbonyl] pyrrolidine-2-carbonitrile 4-methylbenzene sulfonate

### Example 19 (52)

### (2S)-1-{[(2S,4R)-4-(2,6-diethoxy-4-hydroxyphenyl)pyrrolidin-2-yl]carbonyl}pyrrolidine-2-carbonitrile 4-methylbenzene sulfonate

### Example 19 (53)

### (2S)-1-{[(2S,4R)-4-(2-ethoxy-4-hydroxy-6-methylphenyl)pyrrolidin-2-yl]carbonyl}pyrrolidine-2-carbonitrile 4-methylbenzene sulfonate

### Example 19 (54)

### (2S)-1-{[(2S,4R)-4-(2-hydroxy-4-isopropoxy-6-methylphenyl)pyrrolidin-2-yl]carbonyl}pyrrolidine-2-carbonitrile 4-methylbenzene sulfonate

### Example 19 (55)

### (2S)-1-{[(2S,4R)-4-(4-hydroxy-2-isopropoxy-6-methylphenyl)pyrrolidin-2-yl]carbonyl}pyrrolidine-2-carbonitrile 4-methylbenzene sulfonate

### Example 19 (56)

### (2S)-1-{[(2S,4R)-4-(2-methoxy-1-naphthyl)pyrrolidin-2-yl]carbonyl}pyrrolidine-2-carbonitrile 4-methylbenzene sulfonate

### Example 19 (57)

### (2S)-1-{[(2S,4R)-4-(4-hydroxy-2-methyl-6-propylphenyl)pyrrolidin-2-yl]carbonyl}pyrrolidine-2-carbonitrile 4-methylbenzene sulfonate

### Example 19 (58)

### (2S)-1-{[(2S,4R)-4-(3,4-dihydroxyphenyl)pyrrolidin-2-yl]carbonyl}pyrrolidine-2-carbonitrile 4-methylbenzene sulfonate

### Example 19 (59)

### (2S)-1-{[(2S,4R)-4-(3-hydroxy-2-methoxy-6-methylphenyl)pyrrolidin-2-yl]carbonyl}pyrrolidine-2-carbonitrile 4-methylbenzene sulfonate

### Example 19 (60)

### (2S)-1-({(2S,4R)-4-[4-(benzyloxy)-2-methoxy-6-methylphenyl)pyrrolidin-2-yl}carbonyl)pyrrolidine-2-carbonitrile 4-methylbenzene sulfonate

### Example 19 (61)

### (2S)-1-{[(2S,4R)-4-(5-hydroxy-2-methoxyphenyl)pyrrolidin-2-yl]carbonyl}pyrrolidine-2-carbonitrile 4-methylbenzene sulfonate

### Example 19 (62)

### (2S)-1-{[(2S,4R)-4-(5-hydroxy-2-methylphenyl)pyrrolidin-2-yl]carbonyl}pyrrolidine-2-carbonitrile 4-methylbenzene sulfonate

### Example 19 (63)

### (2S)-1-{[(2S,4R)-4-(3-hydroxy-2-methylphenyl)pyrrolidin-2-yl]carbonyl}pyrrolidine-2-carbonitrile 4-methylbenzene sulfonate

### Example 19 (64)

### (2S)-1-({(2S,4R)-4-[3-hydroxy-4-(2-hydroxyethoxy)phenyl)pyrrolidin-2-yl)carbonyl)pyrrolidine-2-carbonitrile 4-methylbenzene sulfonate

### Example 20

### [3-((3R,5S)-5-{[(2S)-2-cyanopyrrolidin-1-yl]carbonyl}pyrrolidin-3-yl)phenoxy]acetic acid 4-methylbenzene sulfonate

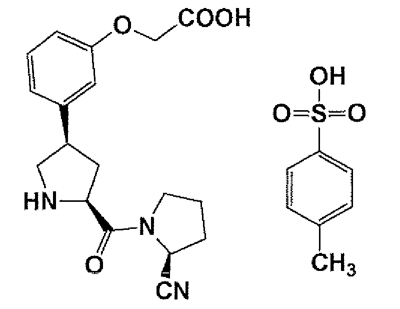

The compound prepared in Example 19 (29) (IA-255) was carried out, by the same procedure as Reference Example 3 to give Boc protected compound. The Boc protected compound (230 mg) was dissolved into methylethylketone (3 ml). Potassium carbonate (414 mg) and a bromoacetic acid benzyl (0.29 ml) were added to the solution and the mixture was refluxed for 1 hour. The reaction mixture was diluted with ethyl acetate, washed with water and a saturated solution of sodium chloride successively, dried with sulfuric anhydride magnesium and concentrated. The residue was purified by column chromatography on silica gel (hexane:ethyl acetate =1:1) to give the title compound (380 mg). The obtain compound was carried out, by the same procedure as Reference Example 5 →Example 14 to give the title compound.
TLC: Rf 0.39 (ethyl acetate: acetic acid:water=3:1:1);
NMR (DMSO-d₆): δ 1.81 (m, 1H), 2.14 (m, 4H), 2.28 (s, 3H), 2.94 (m, 1 H), 3.24 (m, 1H), 3.59 (m, 4H), 4.60 (m, 1H), 4.67 (s, 2H), 4.85 (dd, J=7.96, 4.85 Hz, 1H), 6.82 (dd, J=8.51, 2.29 Hz, 1H), 6.92 (m, 2H), 7.10 (d, J=8.06 Hz, 2H), 7.26 (t, J=7.96 Hz, 1 H), 7.47 (d, J=8.06 Hz, 2H), 8.95 (m, 1 H), 9.52 (m, 1H)).

### Example 20 (1) - Example 20 (6)

The following compounds were obtained, by the same procedure as Example 20.

### Example 20 (1)

### [4-((3R,5S)-5-{[(2S)-2-cyanopyrrolidin-1-yl]carbonyl}pyrrolidin-3-yl)phenoxy]acetic acid 4-methylbenzene sulfonate

### Example 20 (2)

### 2-[4-((3R,5S)-5-{[(2S)-2-cyanopyrrolidin-1-yl]carbanyl}pyrrolidin-3-yl)]-2-phenoxymethylpropane acid 4-methylbenzene sulfonate

### Example 20 (3)

### [4-((3R,5S)-5-{[(2S)-2-cyanopyrrolidin-1-yl]carbonyl}pyrrolidin-3-yl)-3,5-dimethylphenoxy]acetic acid 4-methylbenzene sulfonate

### Example 20 (4)

### 2,2'-[[4-((3R,5S)-5-{[(2S)-2-cyanopyrrolidin-1-yl]carbonyl}pyrrolidin-3-yl)-1,2-phenylene]bis(oxy)]diacetic acid 4-methylbenzene sulfonate

### Example 20 (5)

### [4-((3R,5S)-5-{[(2S)-2-cyanopyrrolidin-1-yl]carbonyl}pyrrolidin-3-yl)-3-methoxy -5-methylphenoxy]acetic acid 4-methylbenzene sulfonate

### Example 20 (6)

### [4-((3R,5S)-5-{[(2S)-2-cyanopyrrolidin-1-yl]carbonyl}pyrrolidin-3-yl)-3,5-dimethoxy phenoxy]acetic acid 4-methylbenzene sulfonate

### Example 21

### 4-[4-((3R,5S)-5-{[(2S)-2-cyanopyrrolidin-1-yl]carbonyl}pyrrolidin-3-yl)phenoxy]butanoic acid 4-methylbenzene sulfonate

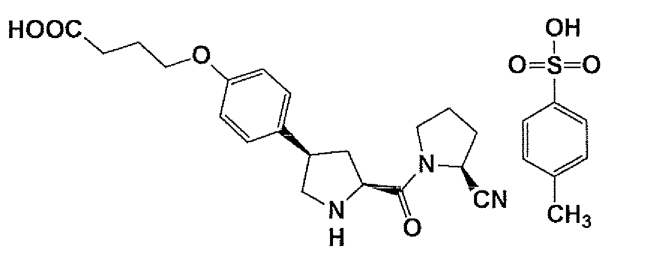

The title compound was obtained, using the compound prepared in Reference Example 38, by the same procedure as Reference Example 5 →Example 14.
TLC: Rf 0.24 (chloroform:methanol=9:1);
NMR (DMSO-d₆): δ 2.02 (m, 7H), 2.28 (s, 3H), 2.36 (t, J=7.32 Hz, 2H), 2.91 (m, 1H), 3.18 (m, 1H), 3.53 (m, 4H), 3.95 (t, J=6.50 Hz, 2H), 4.59 (m, 1 H), 4.85 (dd, J=7.87, 4.76 Hz, 1 H), 6.90 (d, J=8.79 Hz, 2H), 7.10 (d, J=8.06 Hz, 2H), 7.24 (d, J=8.79 Hz, 2H), 7.46 (d, J=8.06 Hz, 2H), 8.91 (m, 1H), 9.49 (m, 1H).

### Example 21 (1)

### trans-4-[4-((3R,5S)-5-{[(2S)-2-cyanopyrrolidin-1-yl]carbonyl}pyrrolidin-3-yl)phenoxy]cyclohexanecarboxylic acid 4-methylbenzene sulfonate

The title compound was obtained, by the same procedure as Example 21.

### Example 22

### ethyl ((3R,5S)-5-{[(2S)-2-cyanopyrrolidin-1-yl]carbonyl}pyrrolidin-3-yl)acetate 4-methylbenzene sulfonate

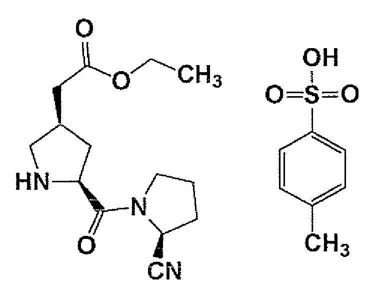

The compound prepared in Example 15 was operated, by the same procedure as Reference Example 3. *p*-toluenesulfonic acid monohydrate (119 mg) was added to a solution of the obtained compound (200 mg) in ethanol (2 ml) and the mixture was refluxed for 4 hours. The reaction mixture was concentrate. The residue was washed with ethyl acetate to give the title compound (184 mg).
TLC: Rf 0.50 (chloroform:methanol=5:1);
NMR (DMSO-d₆): δ 1.18 (t, J=7.14 Hz, 3H), 1.46 (m, 1H), 2.01 (m, 2H), 2.17 (m, 2H), 2.28 (s, 3H), 2.63 (m, 4H), 2.96 (m, 1H), 3.40 (m, 1H), 3.55 (m, 2H), 4.06 (q, J=7.14 Hz, 2H), 4.47 (m, 1H), 4.83 (dd, J=7.78, 4.85 Hz, 1H), 7.10 (d, J=8.06 Hz, 2H), 7.46 (d, J=8.06 Hz, 2H), 8.72 (s, 1 H), 9.31 (s, 1H).

### Example 22 (1)

### ethyl 4-[4-((3R,5S)-5-{[(2S)-2-cyanopyrrolidin-1-yl]carbonyl}pyrrolidin-3-yl)phenoxy]butanoate 4-methylbenzene sulfonate

The title compound was obtained, by the same procedure as Example 22.

### Example 23

### (2S)-1-[(4R)-4-(2-hydroxyethyl)-L-prolyl]pyrrolidine-2-carbonitrile 4-methylbenzene sulfonate

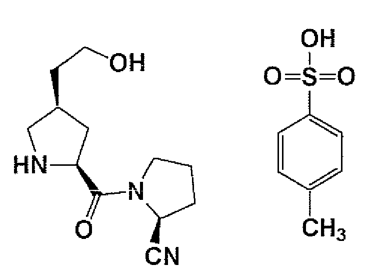

The title compound was obtained, using the compound prepared in Reference Example 41, by the same procedure as Example 14.
TLC: Rf 0.20 (chloroform:methanol:acetic acid=8:2:1);
NMR (DMSO-d₆): δ 1.39 (m, 1H), 1.55 (m, 2H), 1.72 (m, 1H), 2.02 (m, 2H), 2.18 (m, 2H), 2.28 (s, 3H), 2.67 (m, 1 H), 2.87 (m, 1H), 3.41 (m, 3H), 3.57 (m, 2H), 4.00 (m, 1 H), 4.43 (m, 1H), 4.82 (dd, J=7.69, 4.76 Hz, 1 H), 7.10 (d, J=8.06 Hz, 2H), 7.46 (d, J=8.06 Hz, 2H), 8.67 (s, 1H), 9.29 (s, 1H).

### Example 23 (1)

### (2S)-1-[(4S)-4-(3-hydroxypropyl)-L-prolyl]pyrrolidine-2-carbonitrile 4-methylbenzene sulfonate

The title compound was obtained by the same procedure as Reference Example 39 →Reference Example 40 →Reference Example 41 →Example 23.

### Example 24 (1) - Example 24 (2)

The following compounds were obtained, using the compound prepared in Example 41, by the same procedure as Reference Example 38 (b) (using a corresponding alcohol compound) →Reference Example 5 →Example 14.

### Example 24 (1)

### (2S)-1-{(4R)-4-[2-(4-hydroxyphenoxy)ethyl]-L-prolyl}pyrrolidine-2-carbonitrile 4-methylbenzene sulfonate

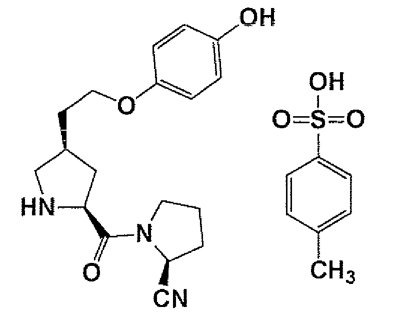

TLC: Rf 0.45 (chloroform:methanol:water=3:1:0.1);
NMR (DMSO-d₆): δ 1.48 (m, 1H), 1.82 (m, 2H), 2.02 (m, 2H), 2.16 (m, 3H), 2.28 (s, 3H), 2.70 (m, 1H), 2.95 (m, 1 H), 3.41 (m, 1H), 3.57 (m, 3H), 3.88 (m, 2H), 4.46 (m, 1H), 4.83 (dd, J =7.96, 4.85 Hz, 1H), 6.66 (m, 2H), 6.73 (m, 2H), 7.10 (dd, J=8.06, 0.55 Hz, 2H), 7.46 (d, J=8.06 Hz, 2H), 8.71 (s, 1 H), 9.29 (s, 1H).

### Example 24 (2)

### 4-[2-((3R,5S)-5-{[(2S)-2-cyanopyrrolidin-1-yl]carbonyl}pyrrolidin-3-yl)ethoxy]benzoic acid 4-methylbenzene sulfonate

### Example 25

### (2S)-1-[(4R)-4-(2-methoxyethyl)-L-prolyl]pyrrolidine-2-carbonitrile 4-methylbenzene sulfonate

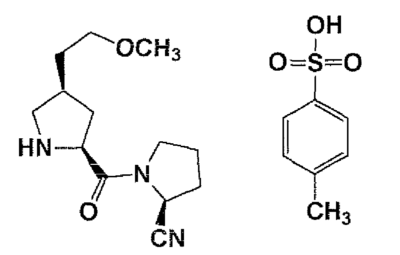

Iodomethane (0.14 ml) and silver oxide (I) (516 mg) were added to a solution of the compound prepared in Reference Example 41 in acetonitrile (2 ml) and the mixture was stirred for 15 hours at room temperature. The reaction mixture was filtrated using cellite. The filtrate was concentrated. The residue was purified by column chromatography on silica gel (hexane:ethyl acetate) to give the corresponding methyl ether compound (126 mg). The title compound was obtained using the compound, by the same procedure as Example 14.
TLC: Rf 0.35 (chloroform:methanol:acetic acid=8:2:1);
NMR (DMSO-d₆): δ 1.40 (m, 1H), 1.63 (m, 2H), 2.02 (m, 2H), 2.19 (m, 3H), 2.28 (s, 3H), 2.65 (m, 1H), 2.88 (m, 1 H), 3.22 (s, 3H), 3.33 (m, 3H), 3.54 (m, 2H), 4.44 (m, 1 H), 4.82 (dd, J =7.69, 4.76 Hz, 1 H), 7.10 (d, J=7.69 Hz, 2H), 7.46 (d, J=8.06 Hz, 2H), 8.68 (s, 1 H), 9.29 (s, 1H).

### Example 26

### [2-((3R,5S)-5-{[(2S)-2-cyanopyrrolidin-1-yl]carbonyl}pyrrolidin-3-yl)ethoxy]acetic acid 4-methylbenzene sulfonate

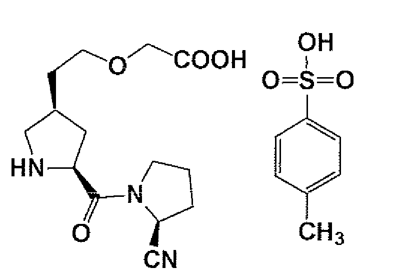

To a solution of the compound prepared in Reference Example 40 (460 mg) in dichloromethane (3 ml), bromoacetic acid *t*-butyl (0.57 ml), hydrogen sulfate tetrabutylammonium (44 mg) and 40% potassium hydroxide aqueous solution (1 ml) were was added. The mixture was stirred for 8 hours at room temperature. Water was added to the reaction mixture. The mixture was extracted with ethyl acetate. The organic layer was washed with water, a saturated aqueous solution of sodium chloride successively, dried and concentrated. The residue was purified by column chromatography on silica gel (chloroform/methanol) to give *t*-butyl ester (290mg). The title compound was obtained using the compound, by the same procedure as Example 14.
TLC: Rf 0.23 (chloroform:methanol:acetic acid=3:1:1)
NMR (DMSO-d₆): δ 1.42 (m, 1 H), 1.65 (m, 2H), 2.10 (m, 5H), 2.28 (s, 3H), 2.69 (m, 1 H), 2.89 (m, 1H), 3.50 (m, 5H), 3.99 (s, 2H), 4.44 (m, 1H), 4.82 (dd, J=7.87, 4.58 Hz, 1H), 7.10 (d, J=8.24 Hz, 2H), 7.46 (d, J=8.24 Hz, 2H), 8.67 (s, 1H), 9.29 (s, 1H).

### Example 27

### (2S)-1-[4-(phenoxymethyl)-prolyl]pyrrolidine-2-carbonitrile 4-methylbenzene sulfonate

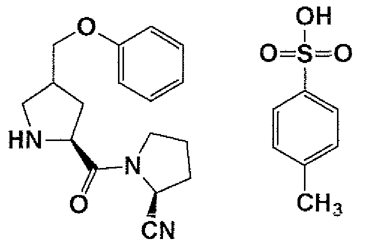

The title compound was obtained, by the same procedure as Reference Example 39 →Reference Example 40 →Reference Example 11 →Reference Example 41 →Reference Example 38 (b) (Phenol was used instead of 4-hydroxybutanoic acid) →Example 14.
TLC: Rf 0.60, 0.53 (chloroform:methanol=5:1);
NMR (DMSO-d₆): δ 1.76 (m, 1H), 2.10 (m, 4H), 2.29 (s, 3H), 2.82 (m, 2H), 3.27 (m, 1H), 3.45 (m, 2H), 3.60 (m, 1 H), 4.06 (m, 2H), 4.59 (m, 1H), 4.76 (m, 1H), 6.95 (m, 3H), 7.09 (m, 2H), 7.28 (m, 2H), 7.53 (d, J=8.24 Hz, 2H), 8.92 (s, 2H).

### Example 28

### (2S)-1-[4-(methoxymethyl)-L-prolyl]pyrrolidine-2-carbonitrile 4-methylbenzene sulfonate

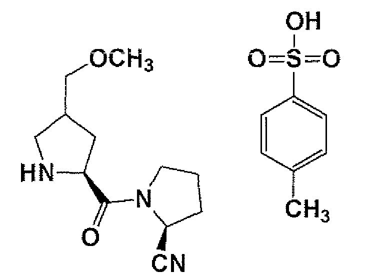

The title compound was obtained, using the compound prepared in Reference Example 31, by the same procedure as Reference Example 39 →Reference Example 40 →Reference Example 11 →Reference Example 41 →Example 25.
TLC: Rf 0.30 (chloroform:methanol=5:1);
NMR (DMSO-d₆): δ 1.63 (m, 1H), 2.06 (m, 2H), 2.24 (m, 2H), 2.29 (s, 3H), 2.65 (m, 2H), 3.13 (dd, J=11.44, 7.41 Hz, 1H), 3.29 (m, 3H), 3.38 (m, 3H), 3.57 (m, 2H), 4.50 (m, 1 H), 4.82 (m, 1H), 7.08 (d, J=7.69 Hz, 2H), 7.52 (d, J=8.06 Hz, 2H), 8.90 (s, 2H).

### Example 29 (1) - Example 29 (19)

The following compounds were obtained, by the same procedure as Reference Example 29 →Example 11.

### Example 29 (1)

### (2S)-1-{[(2S,4S)-4-(4-methylbenzyl)pyrrolidin-2-yl]carbonyl}pyrrolidine-2-carbonitrile hydrochloride

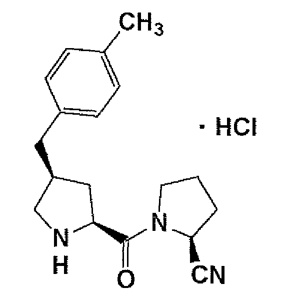

TLC: Rf 0.37 (chloroform:methanol=9:1);
NMR (DMSO-d₆): δ 1.49 (m, 1H), 1.99 (m, 2H), 2.16 (m, 2H), 2.25 (s, 3H), 2.55 (m, 2H), 2.72 (m, 1H), 2.95 (m, 1H), 3.23 (m, 1 H), 3.51 (m, 3H), 4.43 (m, 1H), 4.82 (dd, J=7.83, 4.81 Hz, 1H), 7.10 (m, 4H), 8.71 (m, 1H), 10.43 (m, 1 H).

### Example 29 (2)

### (2S)-1-{[(2S,4S)-4-(4-chlorobenzyl)pyrrolidin-2-yl]carbonyl}pyrrolidine-2-carbonitrile hydrochloride

### Example 29 (3)

### (2S)-1-{[(2S,4S)-4-but-2-yn-1-ylpyrrolidin-2-yl]carbonyl}pyrrolidine-2-carbonitrile hydrochloride

### Example 29 (4)

### (2S)-1-{[(2S,4S)-4-prop-2-yn-1-ylpyrrolidin-2-yl]carbonyl}pyrrolidine-2-carbonitrile hydrochloride

### Example 29 (5)

### (2S)-1-{[(2S,4S)-4-benzylpyrrolidin-2-yl]carbonyl}pyrrolidine-2-carbonitrile hydrochloride

### Example 29 (6)

### (2S)-1-{[(2S,4R)-4-cyclohexylpyrrolidin-2-yl]carbonyl}pyrrolidine-2-carbonitrile hydrochloride

### Example 29 (7)

### (2S)-1-{[(2S,4S)-4-(4-cyanobenzyl)pyrrolidin-2-yl]carbanyl}pyrrolidine-2-carbonitrile 4-methylbenzene sulfonate

### Example 29 (8)

### (2S)-1-{[(2S,4R)-4-(2-adamanthyl)pyrrolidin-2-yl]carbonyl}pyrrolidine-2-carbonitrile 4-methylbenzene sulfonate

### Example 29 (9)

### (2S)-1-{[4-(2,2-dimethylpropyl)pyrrolidin-2-yl]carbonyl}pyrrolidine-2-carbonitrile 4-methylbenzene sulfonate

### Example 29 (10)

### (2S)-1-{[4-(1,3-benzdioxol-5-ylmethyl)pyrrolidin-2-yl]carbonyl}pyrrolidine-2-carbonitrile 4-methylbenzene sulfonate

### Example 29 (11)

### (2S)-1-{[4-(1,3-benzdioxol-4-ylmethyl)pyrrolidin-2-yl]carbonyl}pyrrolidine-2-carbonitrile 4-methylbenzene sulfonate

### Example 29 (12)

### N-{4-chloro-2-[((3S,5S)-5-{[(2S)-2-cyanopyrrolidin-1-yl]carbonyl}pyrrolidin-3-yl)-methyl]phenyl} methane sulfonamide 4-methylbenzene sulfonate

### Example 29 (13)

### N-{2-[((3S,5S)-5-{[(2S)-2-cyanopyrrolidin-1-yl]carbonyl}pyrrolidin-3-yl)methyl]phenyl}methane sulfonamide 4-methylbenzene sulfonate

### Example 29 (14)

### (2S)-1-({(2S,4S)-4-[(2E)-3-phenylpropa-2-en-1-yl]pyrrolidin-2-yl)carbonyl)pyrrolidine-2-carbonitrile 4-methylbenzene sulfonic acid

### Example 29 (15)

### (2S)-1-{[(2S,4S)-4-(cyclohexa-1-en-1-ylmethyl)pyrrolidin-2-yl]carbonyl}pyrrolidine-2-carbonitrile 4-methylbenzene sulfonate

### Example 29 (16):

### (2S)-1-{[(2S,4S)-4-(cyclopenta-1-en-1-ylmethyl)pyrrolidin-2-yl]carbonyl}pyrrolidine-2-carbonitrile 4-methylbenzene sulfonate

### Example 29 (17)

### (2S)-1-{[(2S,4S)-4-(pentafluorobenzyl)pyrrolidin-2-yl]carbonyl}pyrrolidine-2-carbonitrile 4-methylbenzene sulfonate

### Example 29 (18)

### (2S)-1-{[(2S,4S)-4-(mesitylmethyl)pyrrolidin-2-yl]carbonyl}pyrrolidine-2-carbonitrile 4-methylbenzene sulfonate

### Example 29 (19)

### (2S)-1-{[(2S,4S)-4-(2-methylpropa-2-en-1-yl)pyrrolidin-2-yl]carbonyl}pyrrolidine-2-carbonitrile 4-methylbenzene sulfonate

### Example 30 (1) - Example 30 (3)

The following compounds were obtained, by the same procedure as Reference Example 1 →Example 14.

### Example 30 (1)

### (2S)-1-[(2S,3aS,7aS)-octahydro-1H-indol-2-ylcarhonyl]pyrrolidine-2-carbonitrile 4-methylbenzene sulfonate

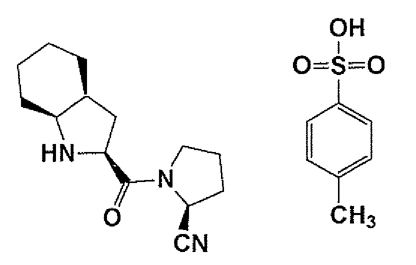

TLC: Rf 0.54 (chloroform:methanol=9:1);
NMR (DMSO-d₆): δ 1.44 (m, 7H), 1.85 (m, 2H), 2.01 (m, 2H), 2.23 (m, 3H), 2.28 (s, 3H), 2.48 (m, 1H), 3.56 (m, 3H), 4.47 (dd, J=7.69, 3.66 Hz, 1 H), 4.81 (dd, J=7.69, 4.76 Hz, 1 H), 7.10 (d, J=8.06 Hz, 2H), 7.46 (d, J=8.06 Hz, 2H), 8.09 (m, 1H), 9.59 (m, 1H).

### Example 30 (2)

### (2S)-1-(octahydrocyclopents[b]pyrrol-2-ylcarbonyl)pyrrolidine-2-carbonitrile 4-methylbenzene sulfonate

### Example 30 (3)

### (2S)-1-{[(2S,4S)-4-(1-hydroxy-1-methylethyl)pyrrolidin-2-yl]carbonyl}pyrrolidine-2-carbonitrile 4-methylbenzene sulfonate

### Example 31 (1) - Example 31 (2)

The following compounds were obtained, by the same procedure as Reference Example 29 →Example 11(1).

### Example 31 (1)

### (2S)-1-{[(2S,4S)-4-(3-phenylpropyl)pyrrolidin-2-yl]carbonyl}pyrrolidine-2-carbonitrile 4-methylbenzene sulfonate

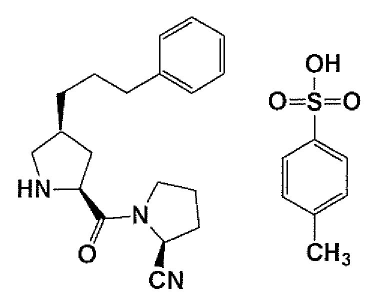

TLC: Rf 0.58 (chloroform:methanol=9:1);
NMR (DMSO-d₆): δ 1.40 (m, 3H), 1.57 (m, 2H), 2.01 (m, 2H), 2.18 (m, 3H), 2.28 (s, 3H), 2.56 (t, J=7.51 Hz, 2H), 2.67 (m, 1H), 2.83 (m, 1H), 3.37 (m, 1H), 3.56 (t, J=6.68 Hz, 2H), 4.42 (dd, J=9.79, 7.78 Hz, 1H), 4.82 (dd, J=7.87, 4.76 Hz, 1 H), 7.10 (d, J=8.24 Hz, 2H), 7.18 (m, 3H), 7.27 (m, 2H), 7.46 (d, J=8.24 Hz, 2H), 8.95 (s, 2H).

Example 31 (2)

(2S)-1-{[(2S,4S)-4-isobutylpyrrolidin-2-yl]carbonyl}pyrrolidine-2-carbonitrile 4-methylbenzene sulfonate

## Claims

1. A compound represented by formula (I): 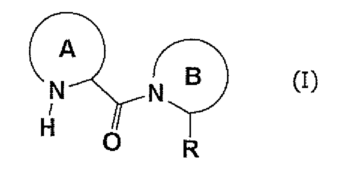 wherein ring A represents a nitrogen-containing heterocyclic ring which may have a substituent(s);
ring B represents 5-membered heterocyclic ring which may have a substituent(s) and
R represents a hydrogen atom or cyano, or a salt thereof.

2. The compound according to claim 1, which is represented by formula (IA): 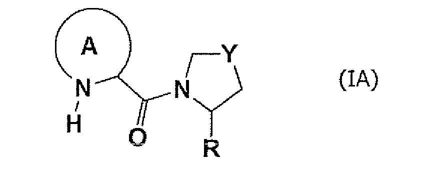 wherein Y represents -CH₂-, an oxygen atom, a nitrogen atom, or a sulfur atom which may be oxidized;
the ring represented by 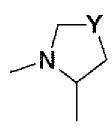 may be substituted and the other symbols have the same meanings as defined in claim 1, or a salt thereof.

3. The compound according to claim 1, which is represented by formula (IA-1): 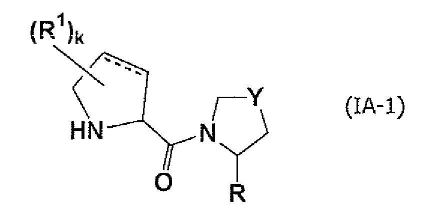 wherein k's of R¹ each independently, represents:
(1) a C1-8 carbon chain which may be substituted by 1-5 of R²,
(2) a carbocyclic ring which may be substituted by 1-5 of R ³,
(3) a heterocyclic ring which may be substituted by 1-5 of R ³, wherein a carbon atom of the heterocyclic ring binds to ring A, or
(4) two R¹'s, taken together with abutting carbon atoms or the same carbon atom of ring A, form a carbocyclic ring or a heterocyclic ring, wherein the ring may be substituted by 1-5 of R³;
R² represents halogen, nitro, cyano, oxo, OR¹⁰, NR¹¹R¹², SR¹⁰, SO₂R¹³, COOR¹⁰, CONR¹¹R¹², COR¹³, =N-OR¹⁰, SO₂NR¹¹R¹², OCOR¹³, OSO₂R¹³, NR¹⁴CONR¹¹R¹², NR¹⁴COOR¹⁰, OCOOR¹⁰, OCONR¹¹R¹², SO₂OR¹⁰, OSO₂OR¹⁰, SOR¹³, a carbocyclic ring which may be substituted by 1-5 of R ³, or a heterocyclic ring which may be substituted by 1-5 of R ³;
R³ represents a C1-8 carbon chain which may be substituted with 1 to 5 groups selected from halogen, nitro, cyano, oxo, OR¹⁰, NR¹¹R¹², SR¹⁰, SO₂R¹³, COOR¹⁰, CONR¹¹R¹², COR¹³, =N-OR¹⁰, SO₂NR¹¹R¹², OCOR¹³, OSO₂R¹³, NR¹⁴CONR¹¹R¹², NR¹⁴COOR¹⁰, OCOOR¹⁰, OCONR¹¹R¹², SO₂OR¹⁰, OSO₂OR¹⁰, SOR¹³, a carbocyclic ring which may have a substituent(s) or a heterocyclic ring which may have a substituent(s); or halogen, nitro, cyano, oxo, OR¹⁰, NR¹¹R¹², SR¹⁰, SO₂R¹³, COOR¹⁰, CONR¹¹R¹², COR¹³, =N-OR¹⁰, SO₂NR¹¹R¹², OCOR¹³, OSO₂R¹³, CONR¹¹R¹², CONR¹¹COR¹³, CONR¹¹SO₂R¹³, NR¹⁴CONR¹¹R¹², NR¹⁴COOR¹⁰, OCOOR¹⁰, OCONR¹¹R¹², SO₂OR¹⁰, OSO₂OR¹⁰, SOR¹³, a carbocyclic ring which may have a substituent(s) or a heterocyclic ring which may have a substituent(s);
R¹⁰ represents:
(1) a hydrogen atom,
(2) a C1-8 carbon chain which may have a substituent(s),
(3) a carbocyclic ring which may have a substituent(s), or
(4) a heterocyclic ring which may have a substituent(s);
R¹¹, R¹², and R¹⁴ each independently represents:
(1) a hydrogen atom,
(2) a C1-8 carbon chain which may have a substituent(s),
(3) a carbocyclic ring which may have a substituent(s),
(4) a heterocyclic ring which may have a substituent(s),
(5) COR¹³, or
(6) SO₂R¹³;
R¹³ represents:
(1) a C1-8 carbon chain which may have a substituent(s),
(2) a carbocyclic ring which may have a substituent(s), or
(3) a heterocyclic ring which may have a substituent(s);
k represents 0 or an integer of 1 to 5; 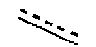 represents a single or double bond; wherein when 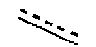 is a single bond, k represents an integer of 1 to 5;
the other symbols have the same meanings as defined in claims 1 and 2, or a salt thereof.

4. The compound according to claim 1, which is represented by formula (IA-2): 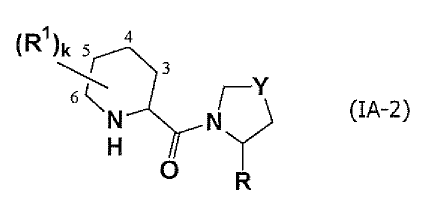 wherein all symbols have same meanings as defined in claims 1 and 2, wherein a ring represented by 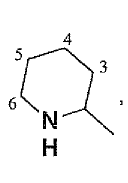 taken together with two R¹'s, does not form 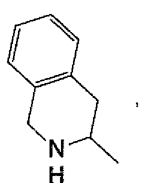 or a salt thereof.

5. The compound according to claim 1, which is represented by formula (IA-3): 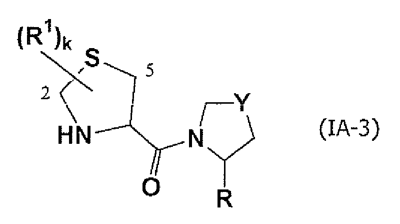 wherein all symbols have the same meanings as defined in claims 1 and 2, or a salt thereof.

6. The compound according to claim 1, which is represented by formula (IA-4): 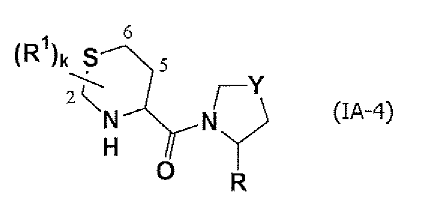 wherein all symbols have the same meanings as defined in claim 2; or a salt thereof.

7. A dipeptidyl peptidase IV inhibitor which comprises the compound represented by formula (I) described in any one of claims 1 to 5 or a salt thereof as an active ingredient.

8. The inhibitor according to claim 7, which is an agent for prevention and/or treatment of dipeptidyl peptidase IV mediated diseases.

9. The inhibitor according to claim 8, wherein the dipeptidyl peptidase IV mediated disease is diabetes mellitus, obesity, autoimmune disease, cancer metastasis, HIV infection, dermatosis or prostatic hypertrophy.

10. A dipeptidyl peptidase IV inhibitor which comprises the compound represented by formula (I), or a salt thereof, and one or at least two agents selected from a PPAR agonist, a sulfonyl urea system hypoglycaemic agent, an insulin sensitizer, an α-glucosidase inhibitor and an acute effect type insulin secernent.

11. A method of inhibiting a dipeptidyl peptidase IV which comprises administering to a mammal an effective amount of the compound represented by formula (I) or a salt thereof.

12. A use of the compound according to claim 1 or a salt thereof for the manufacture of a dipeptidyl peptidase IV inhibitor.

13. A pharmaceutical composition which comprises the compound described in claim 1 or a salt thereof.

14. A prodrug of the compound according to claim 1.
